# FASCICULE DE BREVET EUROPEEN

(11) **EP 1 745 083 B1**
(45) Date de publication et mention de la délivrance du brevet: **16.12.2009**
(21) Numéro de dépôt: 05763665.6
(22) Date de dépôt: 28.04.2005
(51) Int. Cl.: C08G 18/58, C08G 59/30, A61K 6/093

(54) **COMPOSITION DENTAIRE RETICULABLE/POLYMERISABLE PAR VOIE CATIONIQUE STABLE ET A HAUTE TENEUR EN CHARGE**
MITTELS KATIONENREAKTION VERNETZBARE/POLYMERISIERBARE HOCHPOLARE STABILE DENTALZUSAMMENSETZUNG
HIGHLY-CHARGED STABLE DENTAL COMPOSITION WHICH MAY BE CROSS-LINKED/POLYMERISED BY CATIONIC REACTION

(30) Priorité: 13.05.2004 FR 0405177; 30.06.2004 FR 0407209
(43) Date de publication de la demande: 24.01.2007
(73) Titulaire: Bluestar Silicones France, 69486 Lyon (FR)
(72) Inventeur: DERUELLE, Martial, 12 Lotissement Marie Thélisson, 69440 Mornant (FR); GIRAUD, Yves, F-69110 Sainte Foy Les Lyon (FR); FRANCES, Jean-Marc, F-69330 Meyzieu (FR)
(86) Numéro de dépôt international: PCT/FR2005/001048
(87) Numéro de publication internationale: WO 2005/121200

(56) Documents cités:
- WO-A-00/19967
- WO-A-01/08639
- WO-A-01/30304
- WO-A-02/066535
- FR-A- 2 784 024

## Description

Le domaine de l'invention est celui des compositions dentaires. Plus précisément, les compositions dentaires mises au point dans le cadre de la présente invention sont utilisables pour la réalisation de prothèses dentaires et pour la restauration dentaire.

Ces compositions dentaires sont classiquement des résines époxy, ou silicones photopolymérisables ou des résines acrylates polymérisables par voie radicalaire. Ces compositions incluent en outre des charges particulaires de renfort (e.g. en silice hydrophobisée), des photoamorceurs et éventuellement des photosensibilisateurs voire d'autres additifs fonctionnels tels que des pigments ou des stabilisants.

Une fois mélangées, ces compositions sont mises en forme puis photoréticulées en une masse de structure analogue à celle des dents.

Le fait que la charge soit constituée de particules très fines (≃ 0,01 à 5 µm) et de grande surface spécifique, est un facteur limitant de son taux d'incorporation dans la résine. Cette dernière a en effet une capacité d'absorption limitée. Il en résulte que les taux de charges de telles compositions atteignent rarement plus de 45 % en volume. Cela pénalise donc la fonction de renfort mécanique assignée à la charge particulaire

De plus, les compositions dentaires sont formulées avec des charges de renforcement telles que des verres minéraux ou des silices de combustion de très faible granulométrie dont les silanols de surface et/ou l'eau résiduelle réagissent avec les fonctions cationiques ne permettant pas le stockage des compositions. C'est également le cas lorsque ces verres ou ces silices de combustion sont prétraités par des silanes comme le glycidyloxypropyltriméthoxysilane ou glycidyloxypropyltriéthoxysilane ou encore le méthacryloxypropyltriméthoxysilane. Les charges de renforcement interagissent avec les fonctions réactives des espèces (photo)polymérisables/réticulables et sont ainsi à l'origine de problèmes d'instabilité de la composition dentaire lors de son stockage qui peut atteindre des durées de plusieurs mois. Ce phénomène d'instabilité au stockage est accentué pour les compositions fortement chargées (e.g. taux de charge global≥ 50 %).

Le brevet US-B-6,306,926 concerne des compositions dentaires à base de résines époxy (e.g. UV^{®} 6105, EPON^{®} 828, GY281^{®}), oxétane, ou vinyléther, entre autres, polymérisables/réticulables, par voie cationique et sous irradiation, et éventuellement des résines (méth)acrylate polymérisables par voie radicalaire. Outre les inducteurs de polymérisation que sont les photoamorceurs cationiques et éventuellement les initiateurs radicalaires selon le cas, ces compositions comprennent une charge minérale microparticulaire, radioopaque, qui est sélectionnée parmi les composés métalliques suivants : oxydes, halogénures, borates phosphates, silicates, carbonates, germanates, tétrafluoroborates, hexafluoro-phosphates, ayant un point isoélectrique inférieur à 7. Cette composition est telle que sa dureté Barcol est d'au moins 10, après 30 min de polymérisation cationique à 25°C.

Ces résines ont l'inconvénient de ne pas être parfaitement transparentes au rayonnement actinique d'activation de la polymérisation par rayonnement UV-visible, ce qui est dommageable à la cinétique réactionnelle et donc limite les possibilités d'obtenir des matériaux photoréticulés très épais.

La demande de brevet FR-A-2 784 025 vise à remédier ce problème en proposant des compositions dentaires à base de résines silicones polymérisables/réticulables, par voie cationique et sous irradiation suivie ou non d'une post-réticulation thermique. Ces résines silicones sont à fonctionnalités oxirane (époxyde, oxétane...) ou vinyléther. De telles compositions comprennent :
- un ou plusieurs polydiméthylsiloxanes réticulables et/ou polymérisables par voie cationique et porteurs à au moins l'une de leurs extrémités des fonctions réactives de formule : 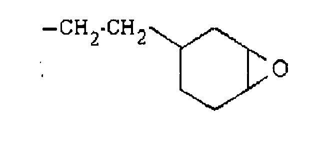
- une quantité efficace d'au moins un amorceur de type borate d'onium: 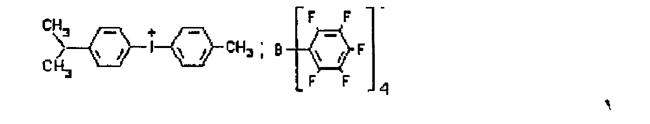
- au moins un photosensibilisateur, et
- au moins une charge dentaire ou de renforcement inerte à base de verres dentaire ou de polyméthacrylate de méthyle ou de silice de combustion éventuellement traitée hexaméthyldisilazane ou polydiméthylsiloxane de surface spécifique 200 m²/g.

Ces compositions dentaires sont destinées à la fabrication de prothèses ou d'appareils dentaires et à la restauration dentaire.

Ces silicones présentent l'avantage par rapport à des résines organiques réticulant par voie cationique d'être très transparents à la lumière UV-visible et donc de permettre l'obtention de matériaux très épais (plusieurs millimètres d'épaisseur) photoréticulés en très peu de temps (inférieur à une minute) avec une lampe UV émettant dans le domaine du visible >400 nm.

Cependant ces silicones sont formulés avec des charges de renforcement à caractère acide de Lewis ou de Bronsted, telles que des verres broyés ou des silices de combustion de très faible granulométrie, dont les silanols de surface et/ou l'eau résiduelle, réagissent avec les fonctions cationiques. De telles formulations silicones sont donc instables au stockage. De plus, lorsque ces silicones sont formulés avec des photosensibilisateurs de type thioxanthone, on observe une grande variation chromatique lors de l'exposition en vue d'une photoréticulation. Ceci se traduit par une coloration rosâtre du produit fini (après exposition) qui n'est pas souhaitable sur le plan esthétique.

Outre ces problèmes, ces compositions dentaires silicones restent perfectibles en ce qui concerne l'accroissement du taux de charge, de manière à permettre l'amélioration des propriétés mécaniques.

On connaît par ailleurs, au travers de la demande de brevet EP-A-1 050 291 des compositions dentaires fortement chargées, présentées comme étant dotées de bonnes propriétés mécaniques et comprenant de 10 à 70 % en volume de charge (e.g. silice de combustion) de granulométrie (Φm) comprise entre 0,05 et 0,5 µm (moins de 50 % en volume de particules de diamètre Φ > 0,50 µm), un monomère acrylique photopolymérisable par voie radicalaire et un dispersant de type ester d'acide phosphorique de formule :

R-[-CO-(CH₂)₅-O-]ₙ-PO₃H₂

Un tel enseignement relatif à des compositions dentaires radicalaires n'est nullement transposable aux compositions dentaires cationiques à base de silicone. En effet, les dispersants R-[-CO-(CH₂)₅-O-]ₙ-PO₃H₂ ne sont pas adaptés aux compositions cationique, notamment car ils contiennent un résidu acide RPO₃H₂ important qui réagit en présence de fonctions oxiranes et nuit à la stabilité de la composition.

Il apparaît donc que l'art antérieur n'apporte pas de solution satisfaisante au double problème de stabilisation au stockage des compositions dentaires à base de motifs polymérisables par voie cationique sous UV (par exemple des oxiranes) et de dispersion de quantités importantes de charges dans la résine. De plus, l'art antérieur n'apporte pas non plus de solution quand au problème de coloration résiduelle des compositions dentaire après réticulation.

L'un des objectifs essentiels de la présente invention est donc de remédier à cela en fournissant de nouvelles compositions dentaires à base de motifs polymérisables par voie cationique sous UV (par exemple des oxiranes), ne présentant pas les inconvénients de fart antérieur sur le plan de la stabilité au stockage et du taux de charge limité.

Un autre objectif essentiel de la présente invention est de fournir de nouvelles compositions dentaires cationiques, polymérisables et/ou réticulables en environnement oral, qui soient non seulement stables au stockage et fortement chargées (e.g. ≥ 50 %) mais qui aient également l'avantage d'être très transparentes à la lumière UV-visible et donc de permettre l'obtention de matériaux très épais (plusieurs millimètres d'épaisseur) photoréticulés en très peu de temps (inférieur à une minute) avec une lampe UV émettant dans le domaine du visible > 400 nm.
Un autre objectif essentiel de la présente invention est de fournir de nouvelles compositions dentaires cationiques, polymérisables et/ou réticulables en environnement oral, qui soient stables, fortement chargées (e.g. ≥ 50 %), faciles à préparer et économiques mais aussi qui ne présentent pas l'inconvénient de générer des colorations parasites après réticulation.

Un autre objectif essentiel de la présente invention est de fournir un nouveau procédé pour traiter une charge de renforcement, en particulier dentaire, de manière à ce qu'elle puisse répondre aux contraintes exposées ci-dessus lorsqu'elle est utilisée notamment comme charge de renfort dans une composition dentaire.

Ces objectifs, parmi d'autres, sont atteints par la présente invention qui concerne tout d'abord une Composition dentaire comprenant:
(1) au moins un composé (**A**) réactif par voie cationique;
(2) au moins une charge dentaire (**B**);
(3) éventuellement au moins un dispersant (**C**) comprenant au moins un polymère ou copolymère organique ;
(4) au moins un photoamorceur cationique (**D**) ;
(5) et éventuellement au moins un photosensibilisateur (**E**),
ladite composition étant caractérisée en ce qu'elle comprend au moins une charge dentaire (**B**) traitée par un procédé (I) comprenant les étapes suivantes :
a) on mélange en milieu solvant la charge dentaire (**B**) et au moins un agent de couplage organosilicié (**F**) de formule 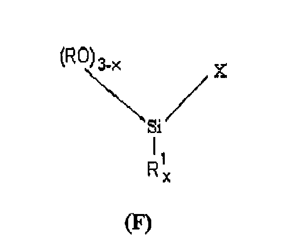 formule dans laquelle :
   - R est un hydrogène ou un radical alkyle linéaire ou ramifié en C1 - C4,
   - R¹ est un radical alkyle linéaire ou ramifié, ou un radical phényle,
      - x est égal à 0, 1 ou 2, et
      - X étant défini par les formules suivantes : 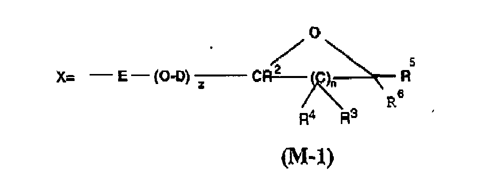 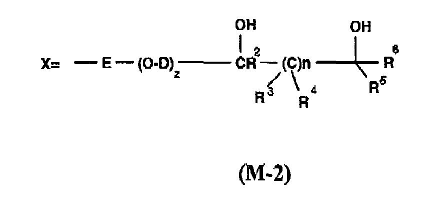 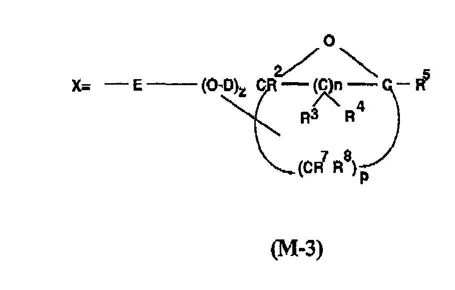 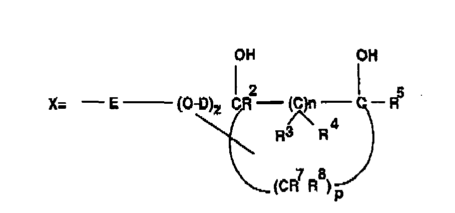 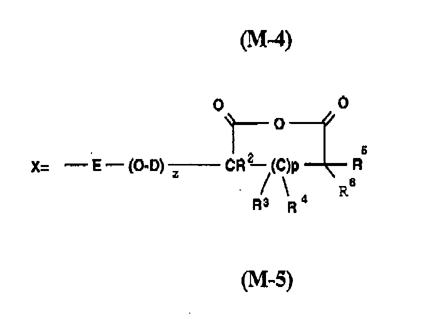 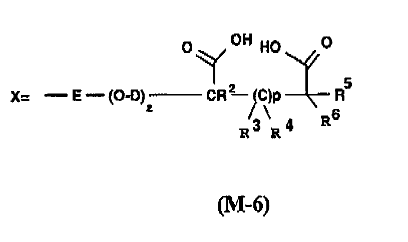 avec:
      - E et D sont des radicaux identiques ou différents choisis parmi les alkyles en C1-C12 linéaires ou ramifiés,
      - z est égal à 0 ou 1,
      - n est égal à 0 ou 1,
   - p est égal à 0, 1, 2, 3, 4, 5 ou 6.
   - R², R³, R⁴, R⁵, R⁶, R⁷ et R⁸ qui sont des radicaux identiques ou différents représentant l'hydrogène ou un alkyle linéaire ou ramifié en C1-C12.
b) on évapore le solvant pour obtenir une charge dentaire intermédiaire (**B-1**),
c) la charge dentaire intermédiaire (**B-1**) subit un traitement thermique de manière à permettre la réaction de couplage entre la charge dentaire intermédiaire (**B-1**) et l'agent de couplage (**F**) et ainsi d'obtenir une charge dentaire intermédiaire (**B-2**),
d) la charge dentaire intermédiaire (**B-2**) est ensuite mélangée en milieu solvant avec au moins un composé (**G**) qui est un monomère, oligomère ou polymère organique
   ou un organosiloxane comprenant au moins une fonction oxirane, oxétane, alcényle éther et/ou carbonate,
e) on évapore le solvant pour obtenir une charge dentaire intermédiaire (**B-3**), et
f) la charge dentaire intermédiaire (**B-3**) subit un traitement thermique de manière à permettre la réaction entre la charge dentaire intermédiaire (**B-3**) et le composé (**G**) et ainsi d'obtenir une charge dentaire traitée (**B-4**).

Il est du mérite des inventeurs d'avoir montré, de manière surprenante et inattendue, qu'il est possible de traiter la surface de la charge et d'augmenter ainsi le taux de charge et donc in fine de renforcer le matériau tout en améliorant la stabilité au stockage de la composition. Cette stabilité se traduit par une durée limite d'utilisation de plusieurs mois ou années.

Cette solution technique est d'autant plus intéressante qu'elle est économiquement viable et facile à mettre en oeuvre.

Il est également intéressant de noter que cette composition donne satisfaction en termes de limitation du retrait après polymérisation/réticulation, ce qui est tout à fait appréciable dans l'application dentaire.

Selon une autre variante préférée de l'invention, le traitement thermique des étapes c) et f) du procédé (I) s'effectue par un chauffage à une température inférieure ou égale à 200°C, de préférence inférieure ou égale à 165°C et encore plus préférentiellement comprise entre 100 et 165°C.

Selon un mode de réalisation préféré, le taux global des charges dentaires (**B**) représente jusqu'à 85 % en poids par rapport au poids total de la composition dentaire et de préférence entre 60 et 80 % en poids.

De manière avantageuse, le traitement de la charge dentaire (**B**) est mis en oeuvre avec jusqu'à 20 % en poids, de préférence entre 1 et 15 % en poids et encore plus préférentiellement entre 2 et 10% en poids du composé (**G**) par rapport au poids total de la composition dentaire.

Le traitement de la charge dentaire (**B**) par l'agent de couplage organosilicié (**F**) se fait avec des composés de formule : 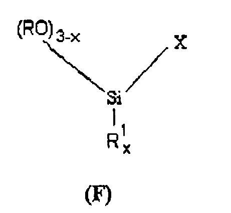 formule dans laquelle :
- R est un hydrogène ou un radical alkyle ou alcényle linéaire ou ramifié en C1 - C4,
- R¹ est un radical alkyle linéaire ou ramifié, ou un radical phényle,
- x est égal à 0, 1 ou 2, et
   X étant défini par la formule suivante : 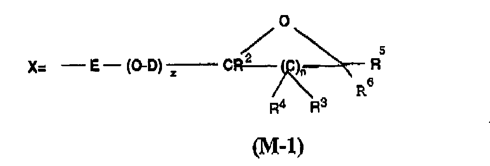 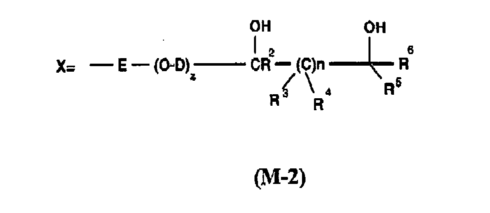 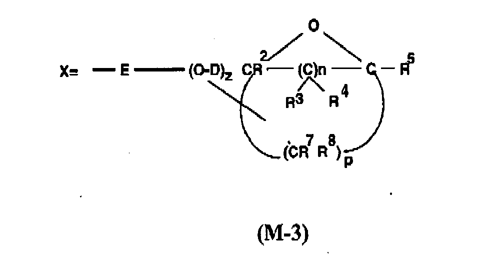 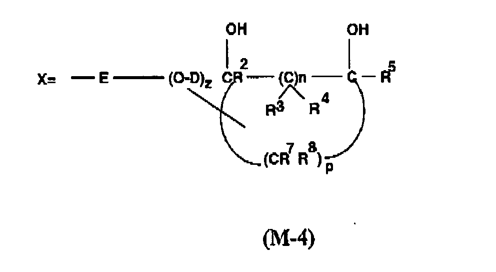 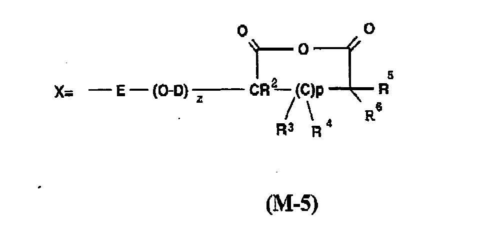 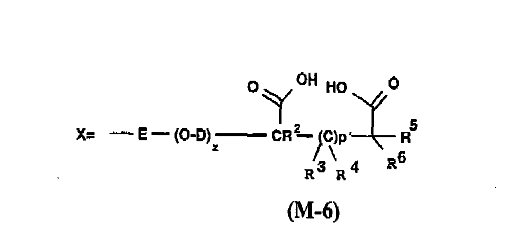 avec :
   - E et D qui sont des radicaux identiques ou différents choisis parmi les alkyles en C1-C12 linéaires ou ramifiés,
- z est égal à 0 ou 1 ;
- n est égal à 0 ou 1 ;
- p est égal à 0,1, 2, 3, 4, 5 ou 6;
- R², R³, R⁴, R⁵, R⁶, R⁷, R⁸ sont des radicaux, identiques ou différents, représentant un atome d'hydrogène ou un alkyle linéaire ou ramifié en C1-C12.

Comme agent de couplage organosilicié (**F**) préféré on peut citer les composés suivants: le glycidyloxypropyltriméthoxysilane, le produit d'hydrolyse du glycidyloxypropyltriméthoxysilane; le glycidyloxypropyltriéthoxysilane, le produit d'hydrolyse en milieu acide du glycidyloxypropyltriéthoxysilane ; le glycidyloxypropyldiméthoxymethylsilane ou le produit d'hydrolyse , le silane béta-(3,4-époxycyclohexyl)ëthyltxiéthoxysilane ou le produit d'hydrolyse, le silane béta-(3,4-époxycyclohexyl)éthyltriméthoxysilane ou le produit d'hydrolyse.

Le traitement de la charge dentaire B-2 par le composé (**G**) se fait avec un composé qui est un monomère, un oligomère, un polymère organique ou un organosiloxane comprenant au moins une fonction oxirane, oxétane, alcényle éther et/ou carbonate et encore plus préférentiellement comprenant au moins une fonction oxirane.

D'une manière préférentielle, le composé (**G**) comprend au moins une fonction choisie parmi le groupe constitué par les structures suivantes (**M-7**) à (**M-12**) : 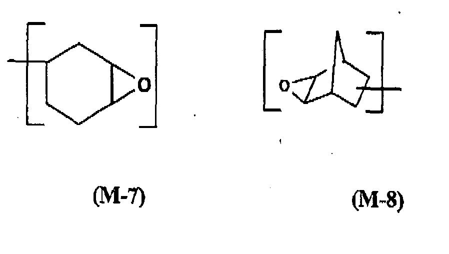 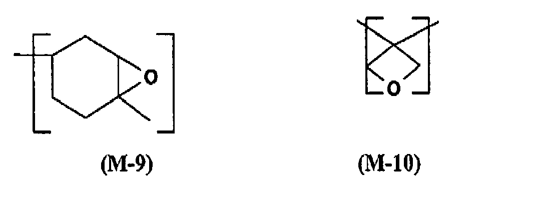 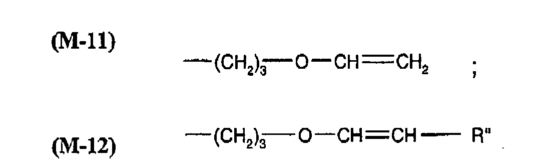 avec R" représentant un radical alkyle linéaire ou ramifié en C₁-C₆.

Selon un premier mode de réalisation préférentiel, le composé (**G**) est un oligomère silicone (**G-1**) ou un polymère silicone (**G-2**). L'oligomère silicone (**G-1**) et le polymère silicone (**G-2**) comprennent :
a) au moins un motif de formule: 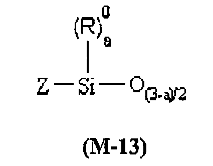 formule dans laquelle :
   - a = 0,1 1 ou 2,
   - R⁰, identique ou différent, représente un radical alkyle, cycloalkyle, aryle, vinyle, hydrogéno, alcoxy, de préférence un alkyle inférieur en C1-C6,
   - Z, identique ou différent, est un substituant organique comportant au moins une fonction oxirane, alcénylether, oxétane et/ou carbonate, et
b) au moins deux atomes de silicium.

D'une manière avantageuse, l'oligomère silicone (**G-1**) et le polymère silicone (**G-2**) sont choisis parmi le groupe constitué par les composés de formules : 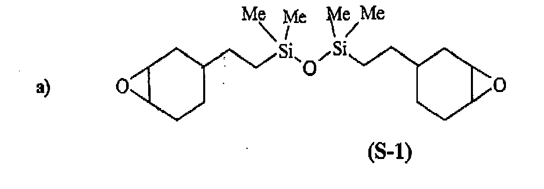 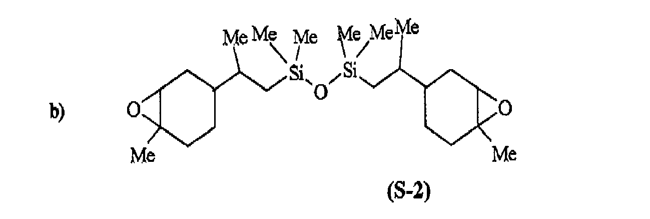 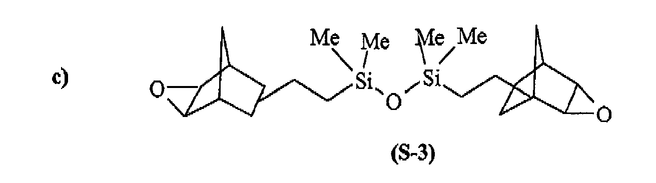 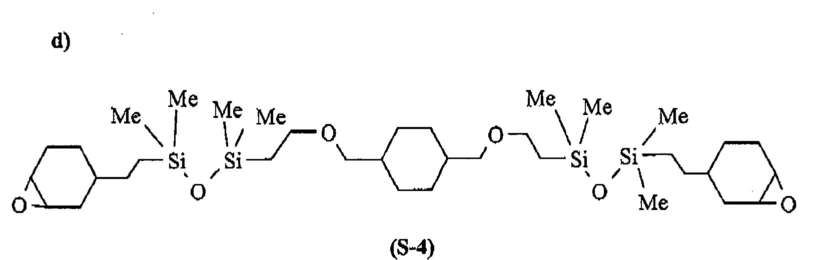 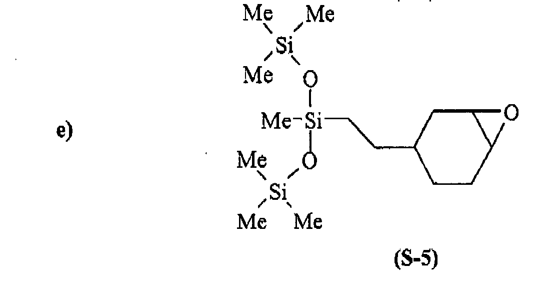 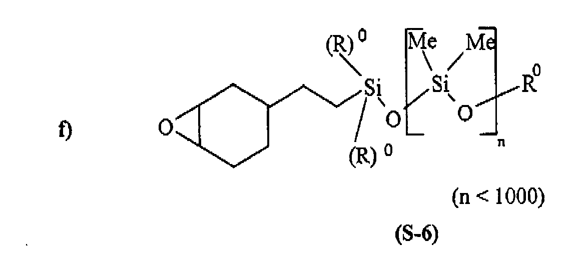 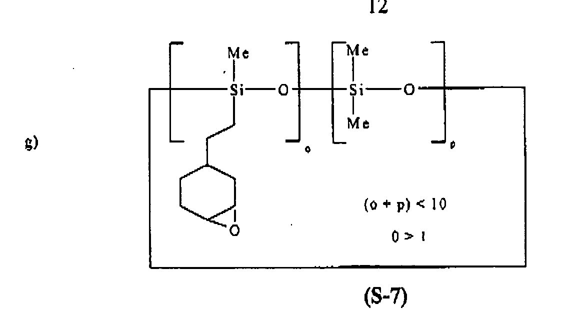 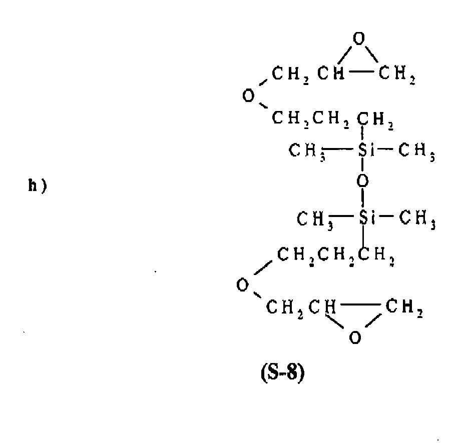 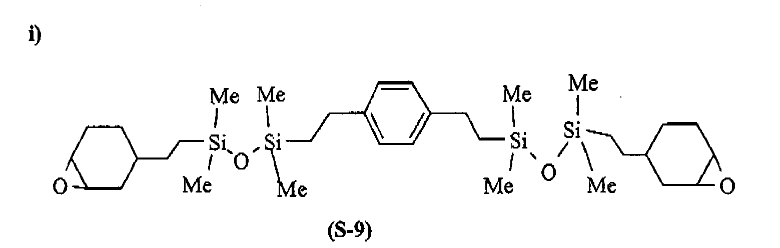 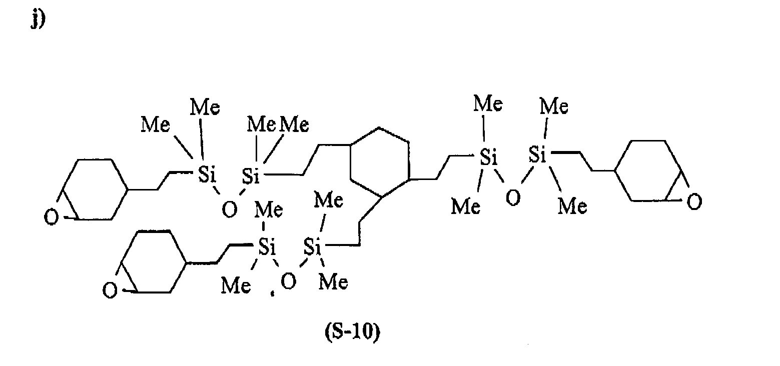 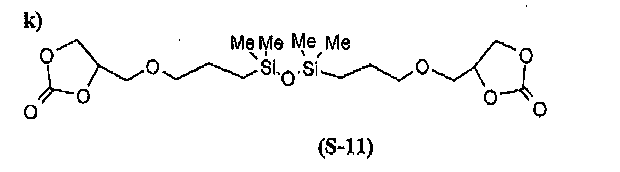 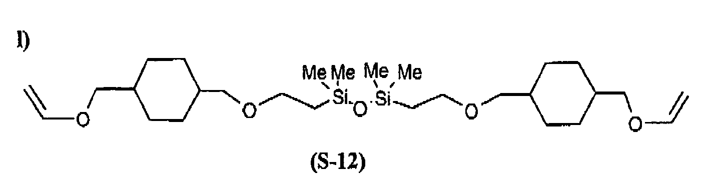 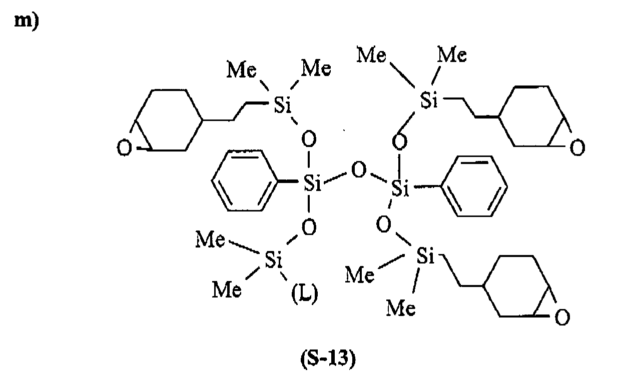 avec L= H; OH; Me; Phényle; Alkyle C1-C12 ; Cycloalkyle C1-C6 ; les groupements 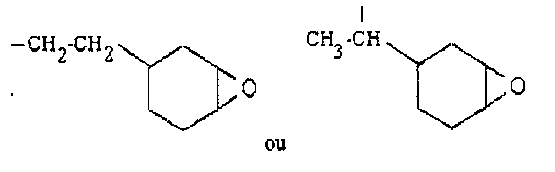 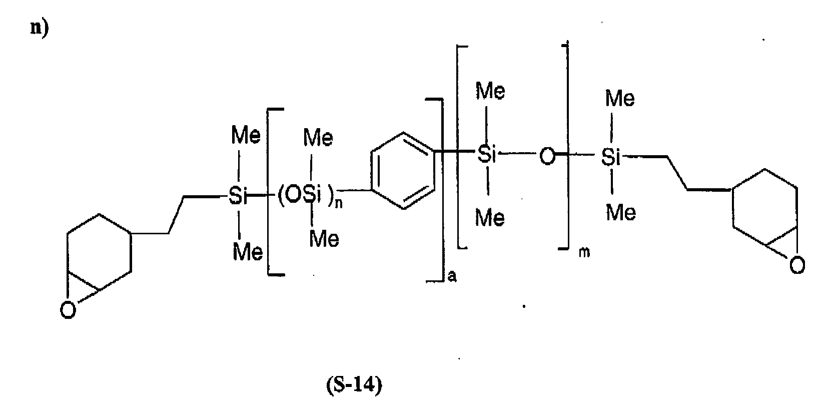 avec n<100 ; a<100 et m<100 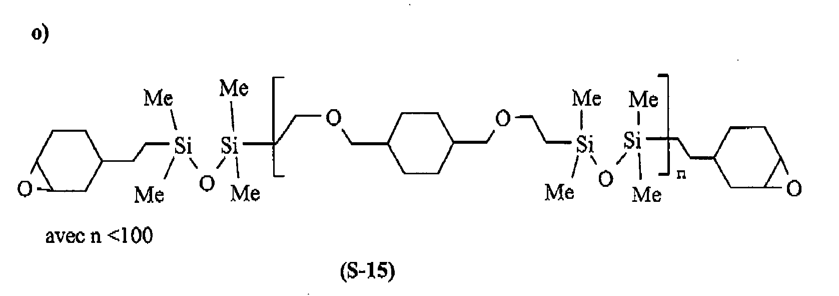 formules dans lesquelles R° ou R₀, identiques ou différents, représente un radical alkyle, cycloalkyle, aryle, de préférence un alkyle inférieure en C1-C6. 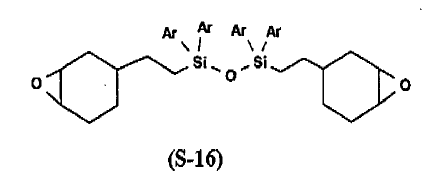 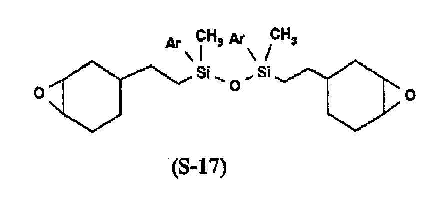 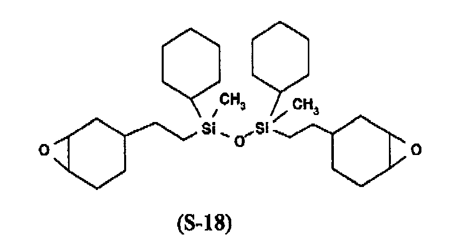 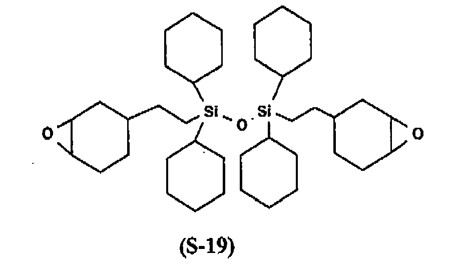 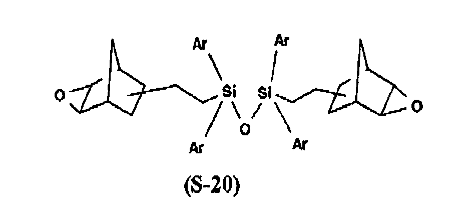 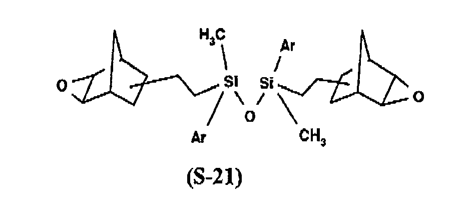 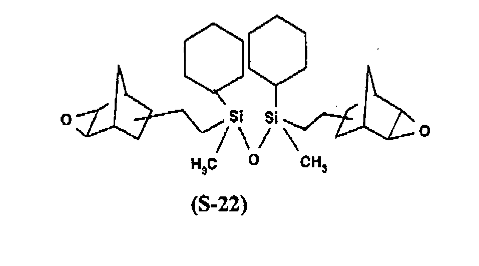 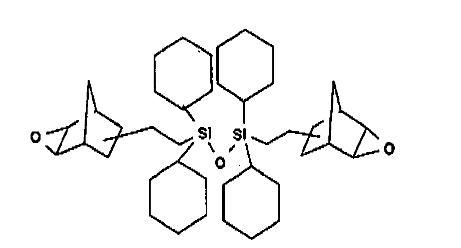 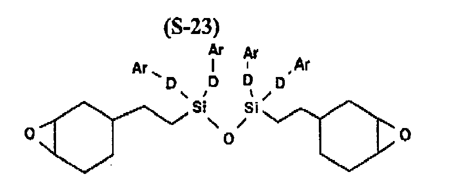 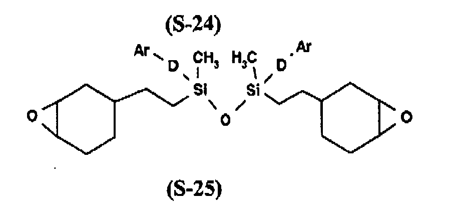 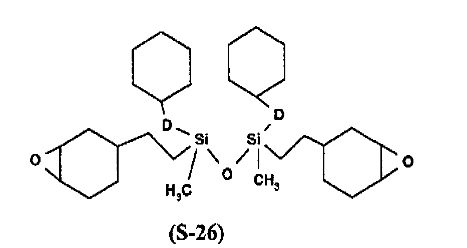 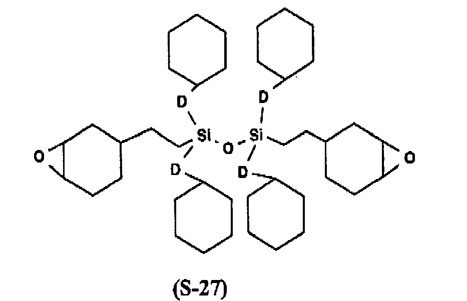 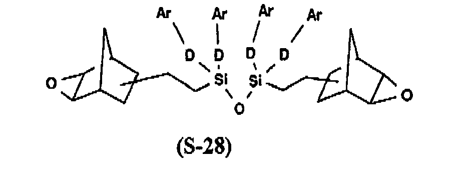 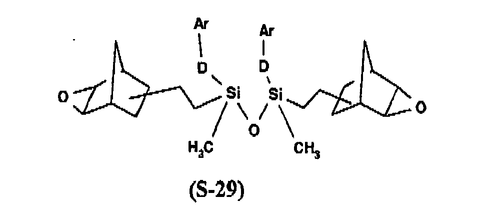 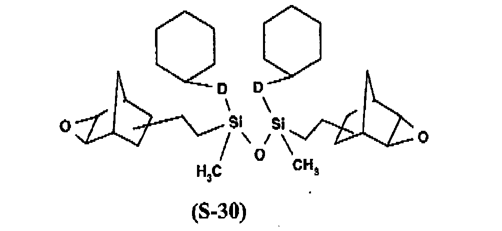 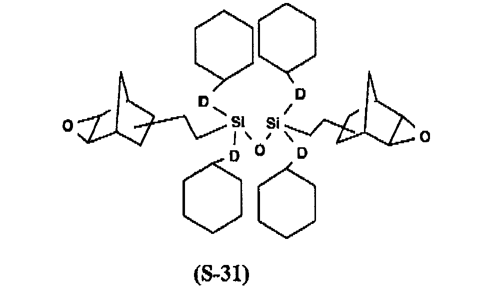 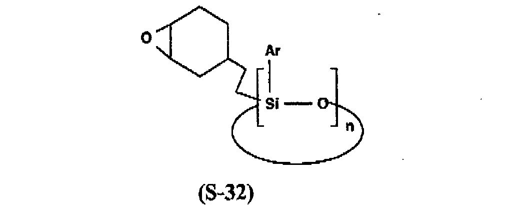 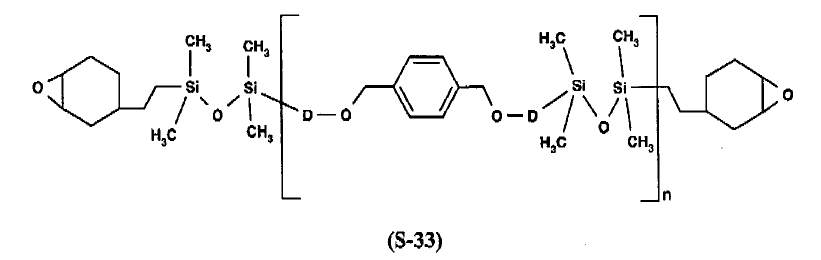 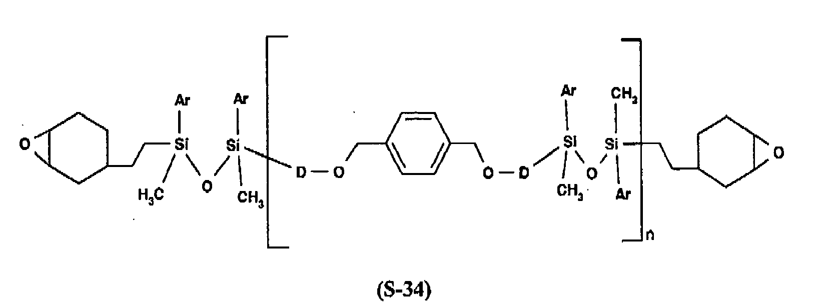 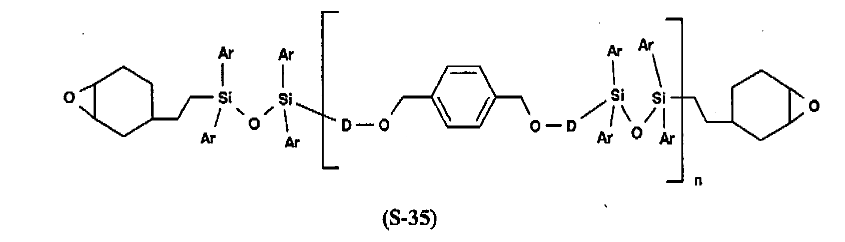 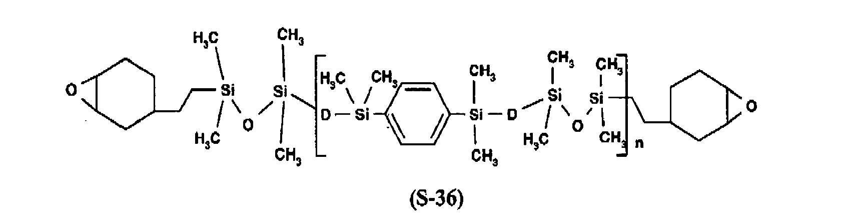 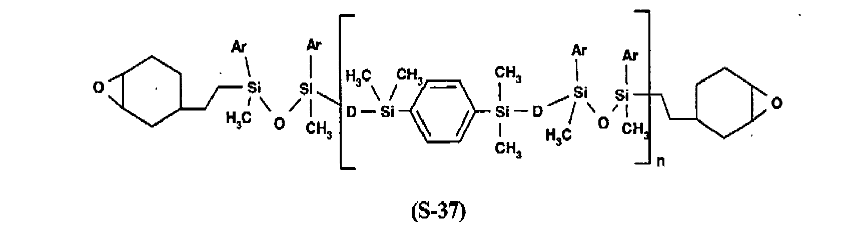 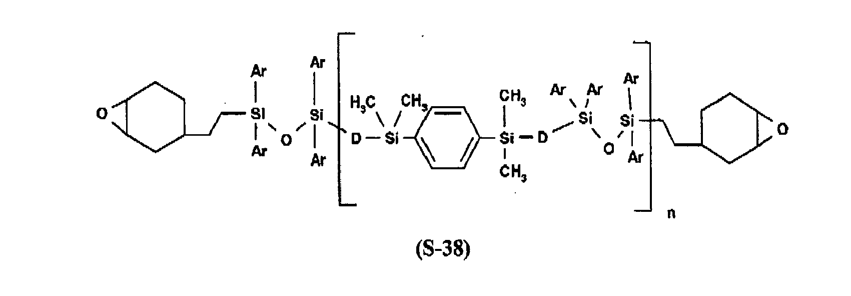 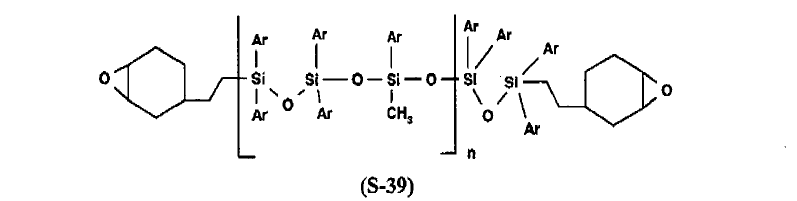 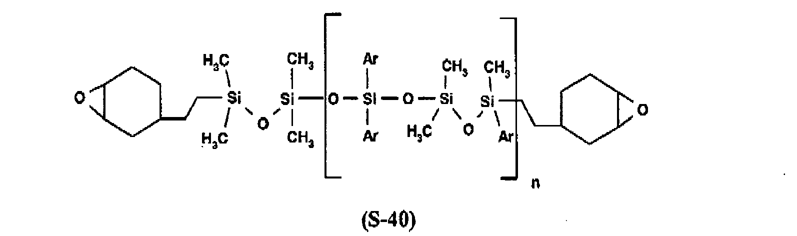 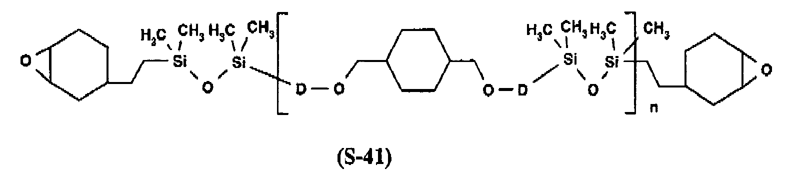 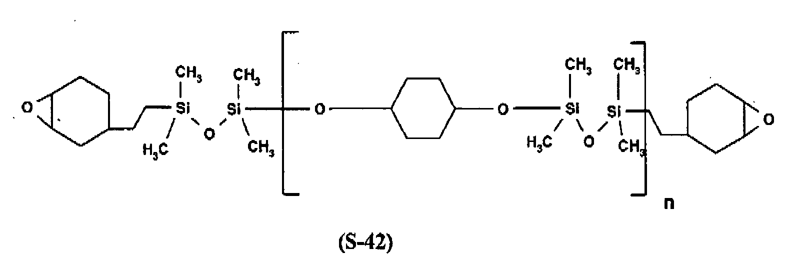 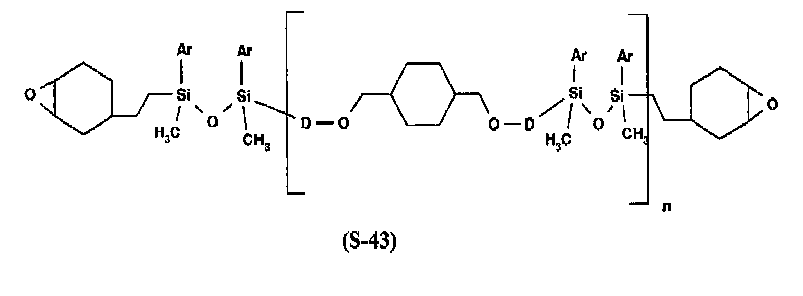 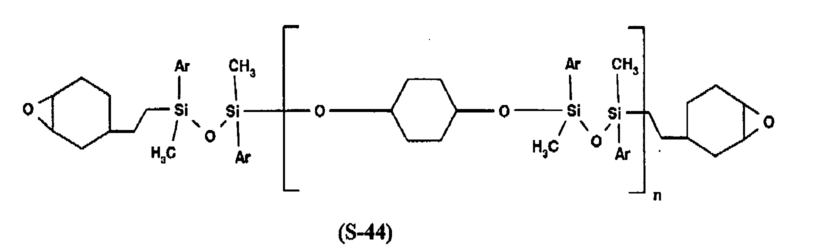 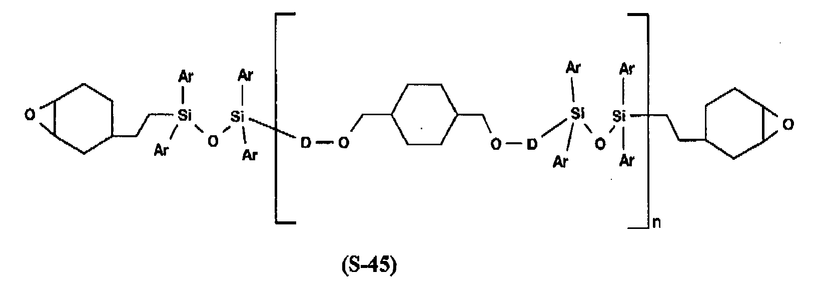 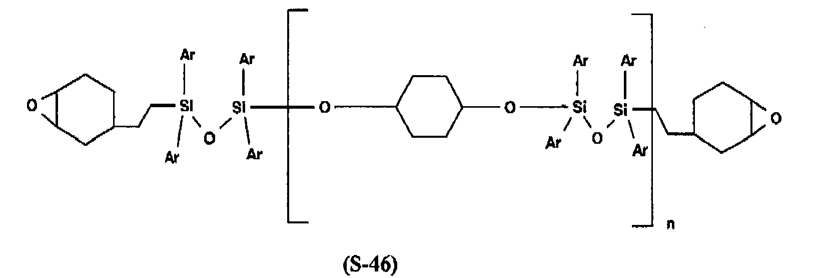 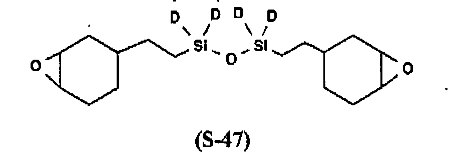 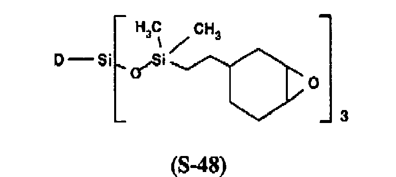 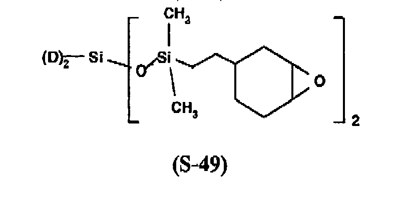 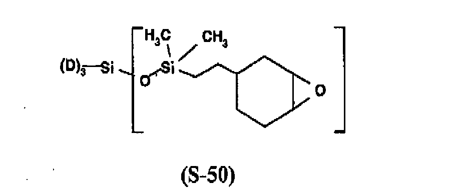 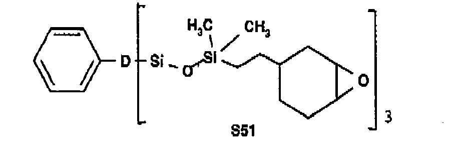 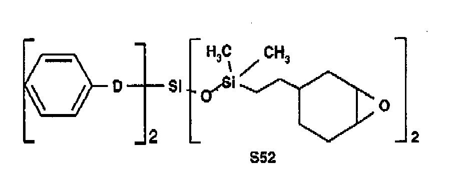 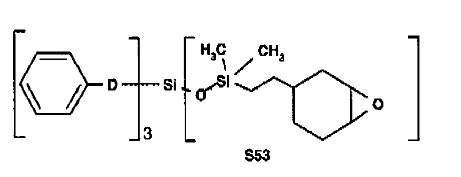 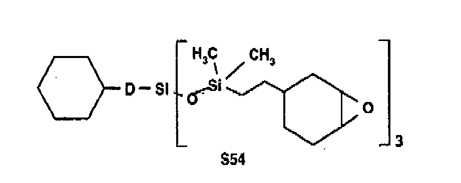 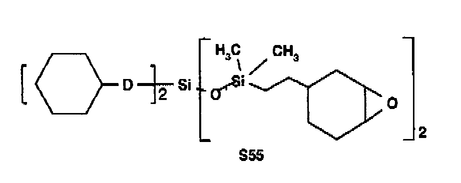 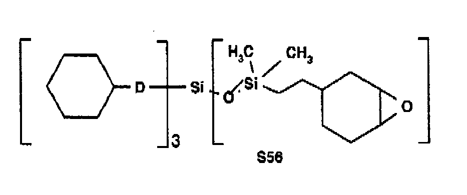 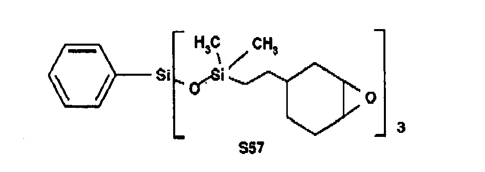 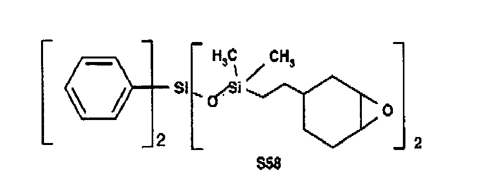 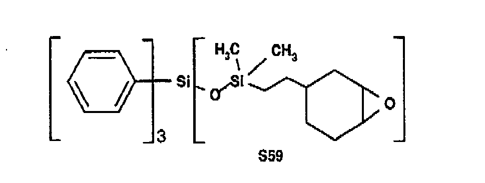 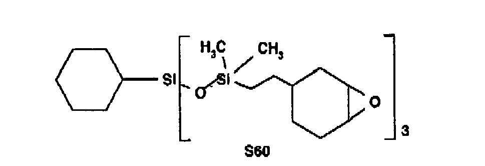 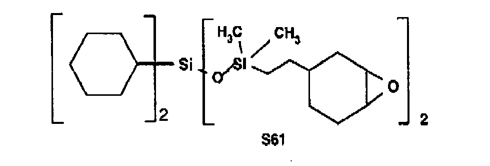 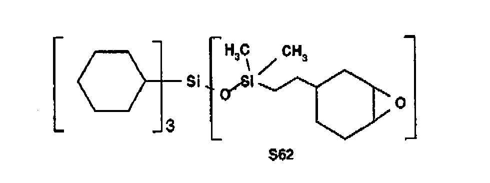 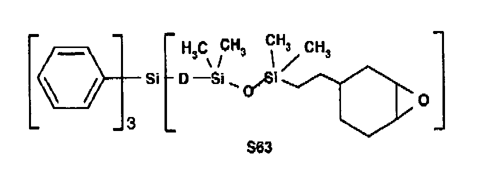 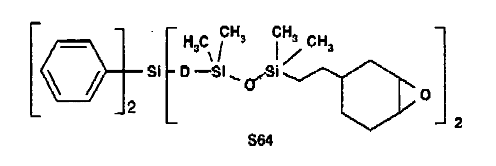 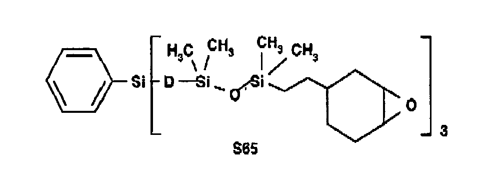 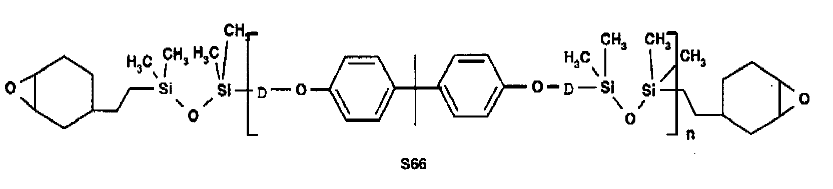 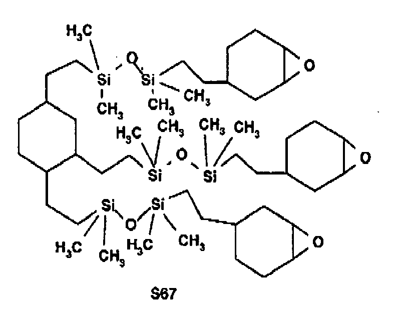 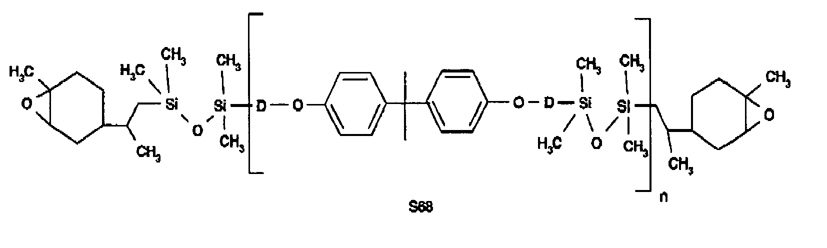 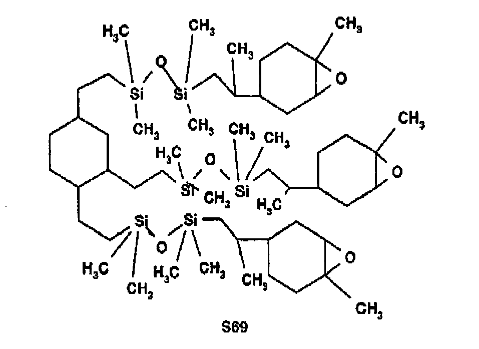 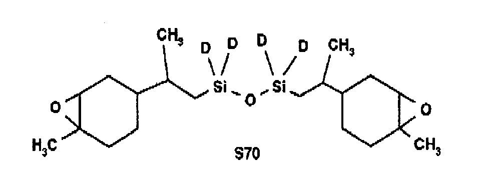 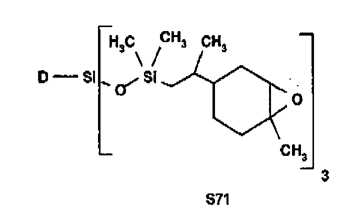 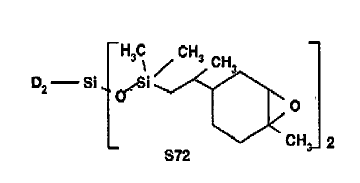 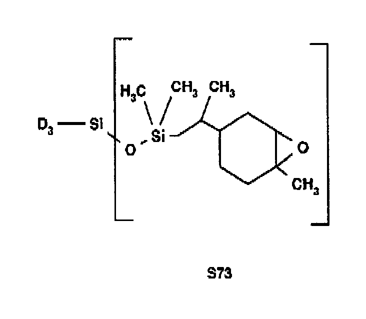 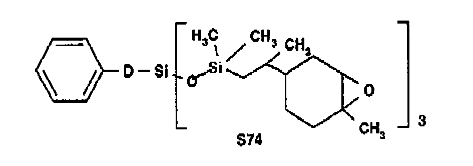 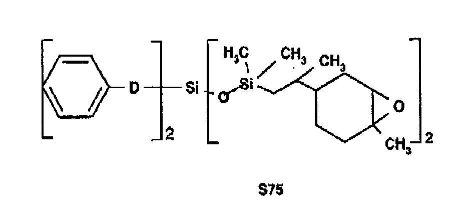 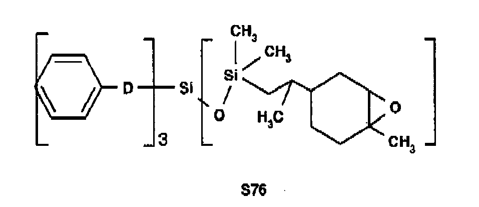 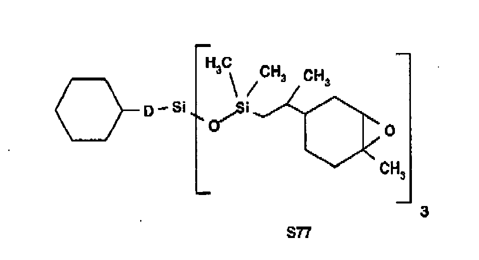 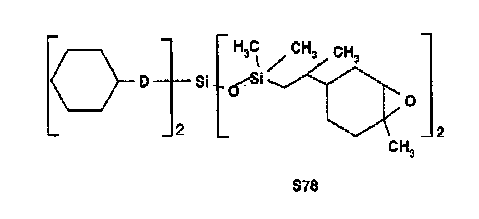 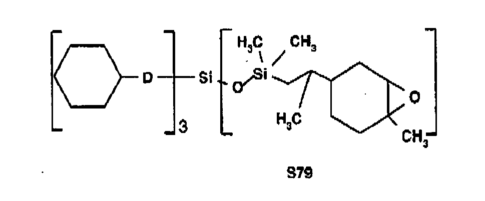 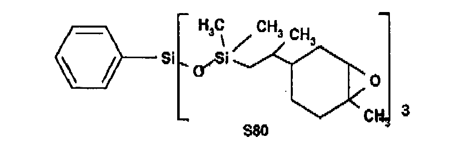 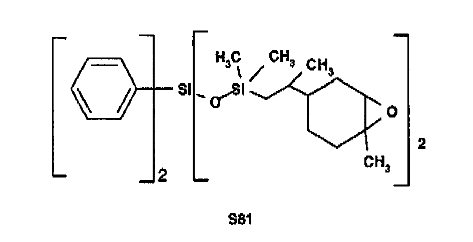 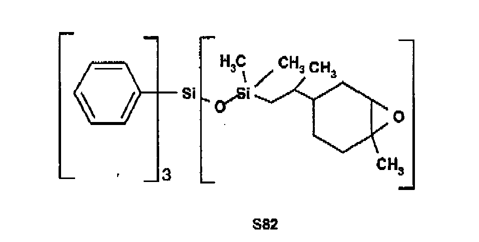 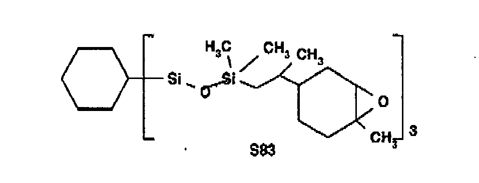 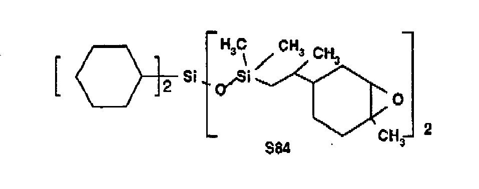 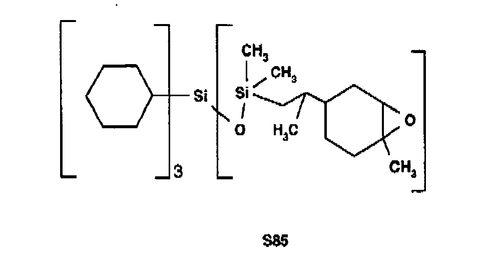 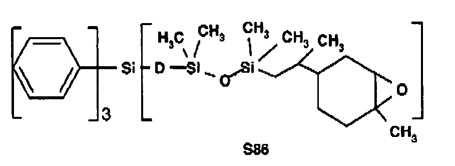 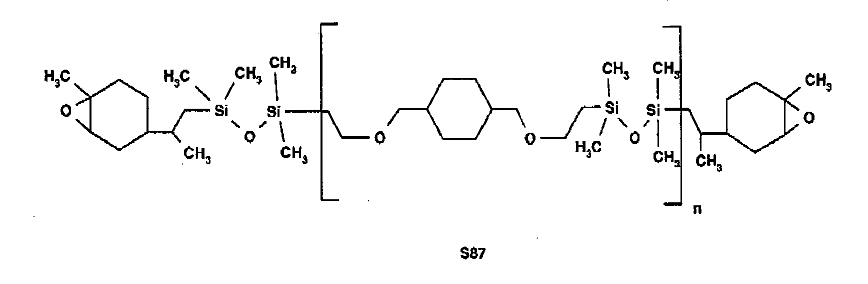 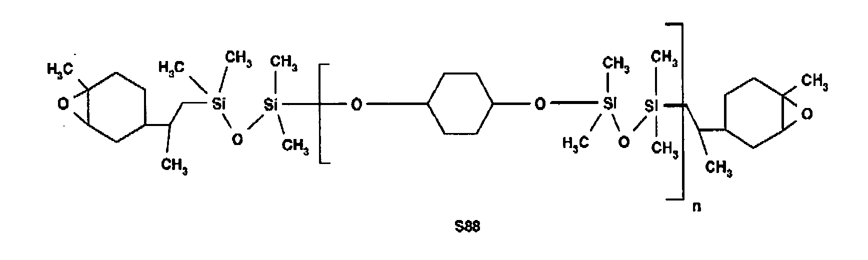 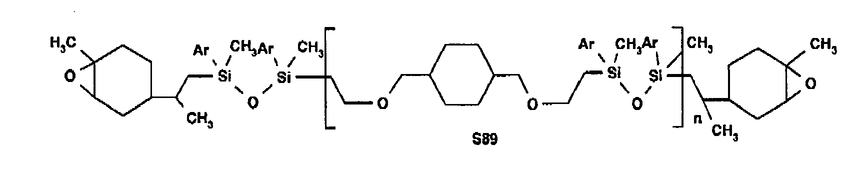 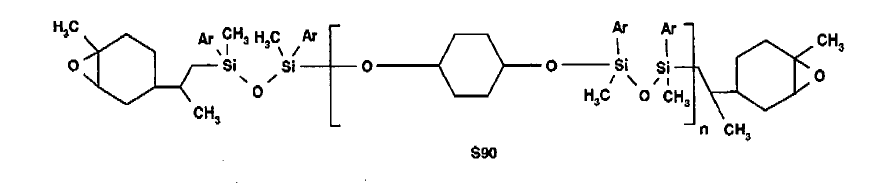 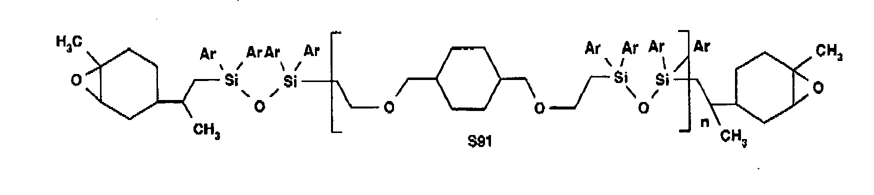 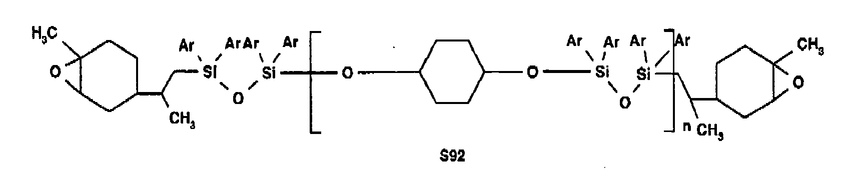 formules dans lesquelles le groupement D est un alkyle en C1-C12 linéaires ou ramifiés et n est un nombre entier compris entre 1 et 20 (bornes de l'intervalle incluses), avec Ar = groupe aryle.
- Selon un deuxième mode de réalisation préférentiel, le composé (**G**) est un silane (**G-3**) de formule : 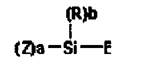 formule dans laquelle :
   E= -CH₂- ;-CH= ; 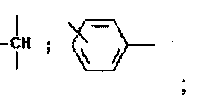
- R, identique ou différents, représente un radical alkyle, cycloalkyle, aryle, vinyle, hydrogéno, alcoxy, de préférence un alkyle inférieur en C1-C6,
- Z, identique ou différent, est un substituant organique comportant au moins une fonction oxirane, alcénylether, oxétane et/ou carbonate, et
- a+b=3.

Selon un mode de réalisation préféré, le silane (**G-3**) est choisi parmi le groupe constitué par les molécules (**S-93**) à (**S-95**) :

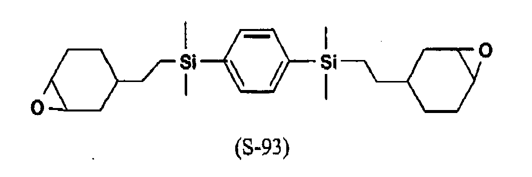

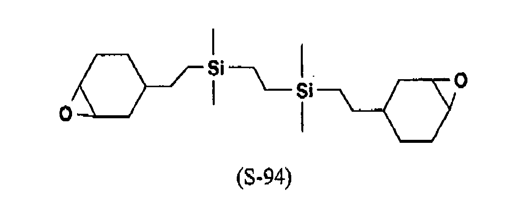

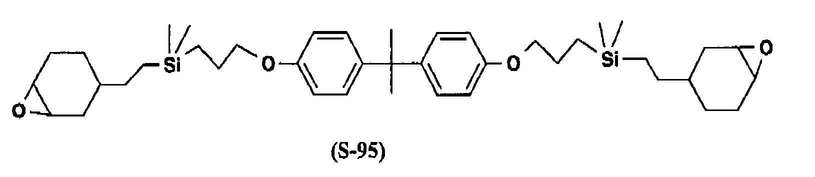

Selon un autre mode de réalisation préférentiel, le composé (**G**) est un composé organique (**G-4**) choisi parmi le groupe constitué par les molécules (**S-96**) à (**S-104**) : 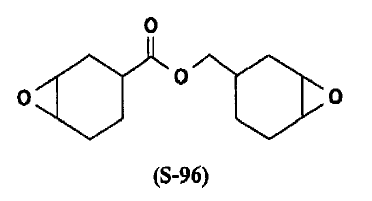 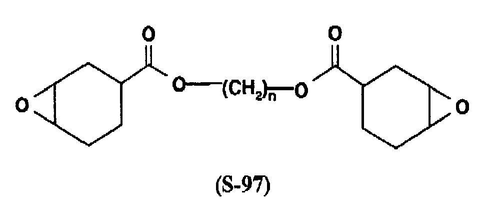 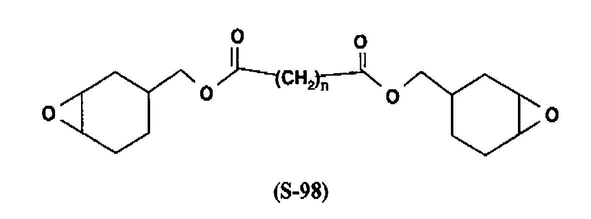 formules dans lesquelles : n est un nombre entier compris entre 1 et 10 (bornes incluses). 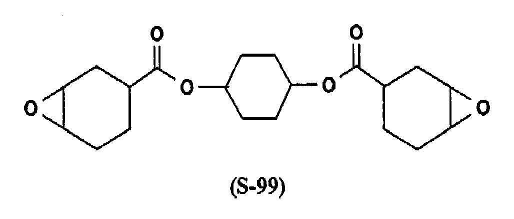 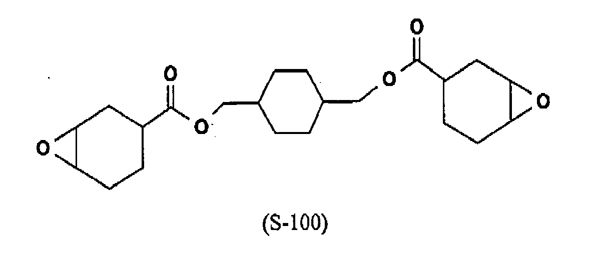 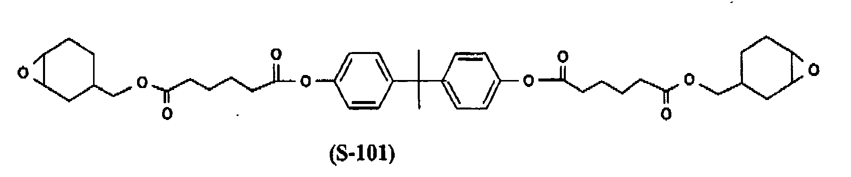 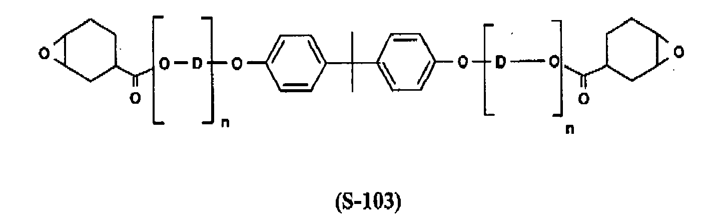 avec n<100 et D= alkyle en C1-C12 linéaires ou ramifiés.

Parmi les molécules de type (**S-103**) on peut choisir la résine UVR6150^{®} commercialisé par la société DOW-CHEMICAL. 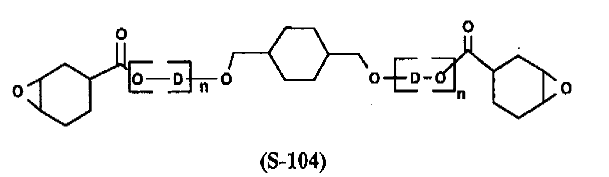 avec n<100 et le groupement D= alkyle en C1-C12 linéaires ou ramifiés.

Pour les résines de type (**S-104**), celle où n=0 est particulièrement adaptée à l'invention.

En général, l'activation photochimique est réalisée sous rayonnement U.V. Plus particulièrement, on utilise un rayonnement U.V. de longueur d'onde de l'ordre de 200 à 500 nm pour la réalisation de prothèses dentaires et un rayonnement U.V, visible de longueur d'onde supérieure à 400 nm pour la réalisation de matériaux de restauration. Une longueur d'onde supérieure à 400 nm permet la réticulation et/ou polymérisation en environnement oral.

L'activation par voie actinique (photochimique) peut être avantageusement complétée (voire remplacée) par une activation thermique.

De préférence, le composé réactif par voie cationique (A) est choisi dans le groupe de monomères et/ou (co)polymères comprenant :
les époxy, les vinyles éthers, les oxétanes, les spiroorthocarbonates, les spiro-orthoesters et leurs associations.

Plus préférentiellement encore, le composé réactif par voie cationique (**A**) est un un oligomère silicone (**G-1**), un polymère silicone (**G-2**), un silane (**G-3**) ou un composé organique (**G-4**) tel que défini ci-dessus par le motif (**M-13**), les molécules (**S-1**) à (**S-101**) ou.

Dans la formule (**M-13**), les fonctions réactives Z particulièrement intéressantes comportent au moins une fonction réactive choisies parmi les radicaux suivants : 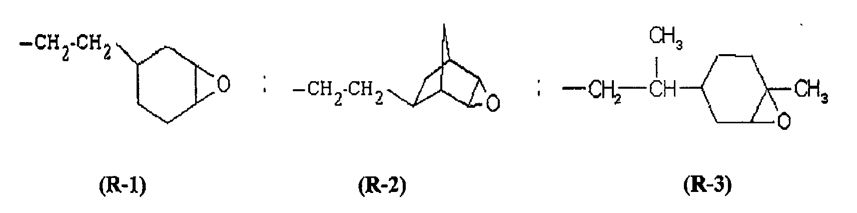 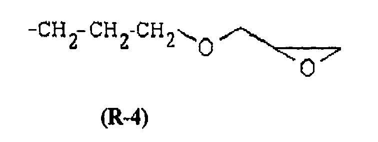 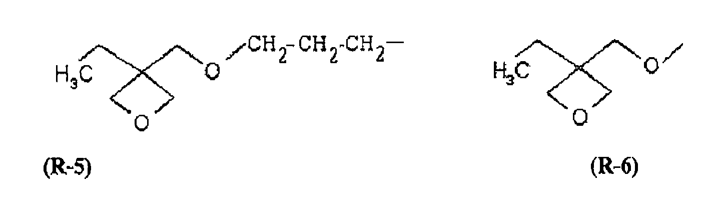 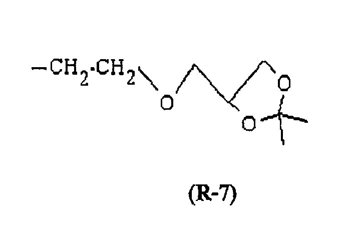 (**R-8**) : -(CH₂)₃-O-CH=CH₂

(**R-9**): -(CH₂)₃-O-CH=CH-R"

- avec R" représentant un radical alkyle linéaire ou ramifié en C₁-C₆.

Selon une variante avantageuse le composé réactif par voie cationique (**A**) est associé à une résine époxy organique ou oxétane représentant moins de 80% en masse de la fraction. Parmi les résines organiques fonctionnelles choisies on préférera celles dont le pourcentage massique de fonction réactive est inférieur à 20% et de préférence inférieur à 15%. On diminuer d'autant le retrait volumique lors de la polymérisation. On choisira de préférence les résines de formule (**S-103**) et (**S-104**) : 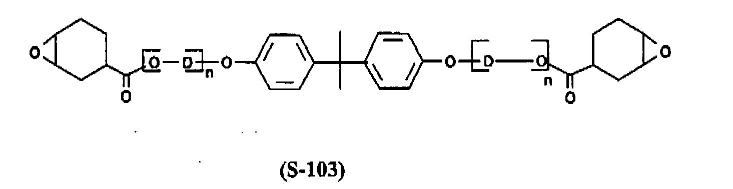 avec n<100 et D= alkyles en C1-C12 linéaires ou ramifiés.

Parmi les résines de type (**S-103**) on peut choisir la résine UVR6150^{®} commercialisée par la société DOW-CHEMICAL. 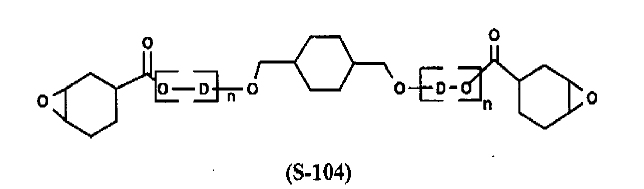 avec n<100 et le groupement D= alkyle en C1-C12 linéaires ou ramifiés.

Pour les résines de type (**S-104**), celle où n=0 est particulièrement adaptée à l'invention.

Différents types de charges dentaires (**B**) sont utilisables pour préparer les compositions selon l'invention. Les charges sont choisies en fonction de l'utilisation finale de la composition dentaire : celles-ci affectent d'importantes propriétés telles que l'apparence, la pénétration du rayonnement U.V., ainsi que les propriétés mécaniques et physiques du matériau obtenu après réticulation et/ou polymérisation de la composition dentaire.

Comme charge de renforcement, on peut utiliser des charges de silice de pyrogénation traitée ou non, des charges de silice amorphe, du quartz, des verres ou des charges non vitreuses à base d'oxydes de silicium par exemple du type de celles décrites dans US-6297181 (sans baryum), de zirconium, de baryum, de calcium, de fluor, d'aluminium, de titane, de zinc, des borosilicates, des aluminosilicates, du talc, des sphérosil, du trifluorure d'yterbium, des charges à base de polymères sous forme de poudre broyée tel que des polyméthacrylates de méthyle inertes ou fonctionnalisés, des polyépoxydes ou des polycarbonates, des whiskers de céramiques (Si-C, Si-O-C, Si-N, Si-N-C, Si-N-C-O ), des fibres de verte,

### A titre d'exemple, on citera:

- des charges inertes à base de polyméthacrylate de méthyle LUXASELF^{®} de la société UGL utilisables dans le domaine dentaire et pigmentées en rose,
- des charges de silice de combustion traitée hexaméthyldisilazane ou polydiméthylsiloxane de surface spécifique 200 m /g,
- des charges de silice de combustion non traitée (Aerosil-AE200^{®} commercialisée par la société DEGUSSA),
- des quartz ou verres à base d'oxydes de silicium.
Selon un mode de réalisation de l'invention, la charge dentaire (**B**) est un verre minéral ou une silice de combustion.
Suivant une caractéristique avantageuse de l'invention,les charges dentaires (**B**) représentent jusqu'à 85 % en poids, de préférence entre 50 et 85 % en poids, encore plus préférentiellement entre 60 et 85 % en poids, par rapport au poids total de la composition dentaire.

Conformément à l'invention, le dispersant (**C**) est sélectionné dans le groupe comprenant : les copolymères polyuréthanne/acrylate éventuellement salifiés par un alkylammonium, les copolymères acryliques éventuellement salifiés par un alkylammonium, les monodiesters d'acides carboxyliques, les polyesters, les polyéthers, les polyuréthannes, les polyuréthannes modifiés, les polyol-polyacrylates, leurs copolymères ou leurs mélanges. Les dispersants commercialisés sous la marque DISPERBYK^{®} (de la société Byk) ou SOLSPERSE^{®} (de la société Avecia) conviennent particulièrement à l'invention. On peut citer en particulier et à titre d'exemples, les produits commerciaux : Disperbyk^{®} 164, Disperbyk^{®} 161, Disperbyk^{®}166, Disperbyk^{®}2070, Disperbyk^{®} 9075, Disperbyk^{®} 9076. On peut citer aussi les dispersants cités dans les brevets suivants :
- le brevet US-B-5,882,393 décrivant des dispersants à base de polyuréthannes/imidazoles-acrylates ou époxydes ;
- le brevet US-B-5,425,900 décrivant des dispersants à base de polyuréthannes ;
- le brevet US-B-4,795,796 décrivant des dispersants à base de polyuréthannes/ polyoxyalkyléne-glycolmonoalkyléther;
- la demande de brevet WO-A-99/56864 décrivant des dispersants à base de polyuréthannes/poly(oxyalkylène-carbonyle): dérivés de ε-caprotactone et de δ-valérolactone ; et
- le brevet EP-B-0 403 197 décrivant des dispersants de type polyolpolyacrylate greffé comprenant un copolymère statistique polyuréthanne/polyvinylique/polyacrylate et un polyoxyalkylène polyéther.

Quantitativement parlant, le dispersant (**C**) est présent à raison de 50 ppm à 1 %, de préférence 100 ppm à 5000 ppm.

De préférence, l'indice d'amine du dispersant (**C**) est inférieur ou égal à 60, et plus préférablement encore compris entre 0,1 et 50 mg de potasse par gramme de dispersant (**C**).

Avantageusement, l'indice d'acide du dispersant est inférieur ou égal à 200, de préférence inférieur ou égal à 100, et plus préférablement compris entre 1 et 60 mg de potasse par gramme de dispersant.

Les photoamorceurs cationiques (**D**) sont choisis parmi les borates d'onium (pris à eux seuls ou en mélange entre eux) d'un élément des groupes 15 à 17 de la classification périodique [Chem. & Eng. News, vol.63, N° 5, 26 du 4 février 1985] ou d'un complexe organométallique d'un élément des groupes 4 à 10 de la classification périodique [même référence].

Selon un mode préférentiel le photoamorceur cationique (**D**) est de type borate et est choisi parmi ceux dont :
a) l'entité cationique du borate est sélectionnée parmi :
   (1) les sels d'onium de formule:

      [(R⁹)ₙ - A - (R¹⁰)ₘ]⁺ (**I**)

      formule dans laquelle :
      - A représente un élément des groupes 15 à 17 tel que par exemple : 1, S, Se, P ou N,
      - R⁹ représente un radical aryle carbocyclique ou hétérocyclique en C6-C20, ledit radical hétérocyclique pouvant contenir comme hétéroéléments de l'azote ou du soufre,
      - R¹⁰ représente R⁹ ou un radical alkyle ou alkényle linéaire ou ramifié en C1-C30 ; - lesdits radicaux R⁹ et R¹⁰ étant éventuellement substitués par un groupement alcoxy en C1-C25, alkyle en C1-C25, nitro, chloro, bromo, cyano, carboxy, ester ou mercapto,
      - m et n sont des nombres entiers, avec n + m = v + 1, v étant la valence de l'élément A,
   (2) les sels d'oxoisothiochromanium en l'occurrence ceux décrits dans la demande de brevet WO 90/11303, notamment le sel de sulfonium du 2-éthyl-4-oxoisothiochromanium ou de 2-dodécyl-4-oxoisothio-chromanium et les sels d'oxoisothiochromanium de formule structurale V : 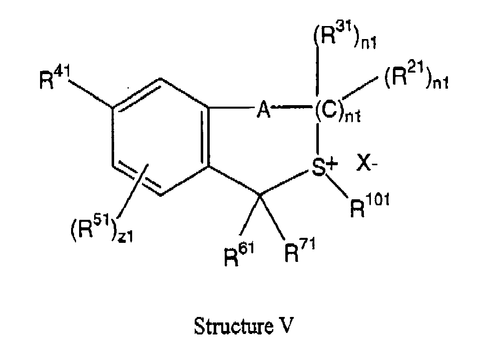
      Formule dans laquelle :
      A représente 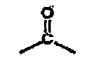
         n1 = un entier entre 1 et 3 ;
         z 1 = un entier entre 0 et 3 ;
         X représente un groupe de formule M¹Y¹ᵣ₁ (1) ou de formule Q¹(2), où dans
         M¹Y¹ᵣ₁ (1) : M¹ = Sb, As, P, B ou Cl, Y¹ représente un halogène (de préférence F ou Cl) ou 0 et où r1 est un entier entre 4 et 6, la formule Q¹(2) représente un acide sulfonique
         R⁸¹-SO₃ où R⁸¹ est un groupe alkyle ou aryle, ou un groupe alkyle ou aryle substitué par un halogène, de préférence F ou Cl,
         R¹⁰¹ représente un groupe alkyle ou cycloalkyle, de préférence en C₁-C₂₀, ou un groupe aryle,
         R²¹ représente un hydrogène, un groupe alkyle, alcényle, cycloalcényle ou cycloalkyle, de préférence C₁-C₂₀, ou un groupe aryle, tous les R²¹ étant indépendants les uns des autres,
         R³¹ représente un hydrogène, un groupe alkyle, alcényle, cycloalcényle ou cycloalkyle, de préférence C₁-C₂₀, ou un groupe aryle, tous les R³¹ étant indépendants les uns des autres,
         R⁴¹ représente un hydrogène, un halogène, un groupe alcényle, par exemple vinyle, cycloalcényle, alkyle, ou cycloalkyle, de préférence en en C₁-C₂₀, un groupe alcoxy ou thioalcoxy, de préférence en en C₁-C₂₀, un groupe poly(alkylènoxyde) avec jusqu'à 10 unités alkylènoxydes terminé par un hydroxyle ou un alkyle (C₁-C₁₂), un groupe aryle, un groupe aryloxy ou thioaryloxy,
         R⁵¹ représente un halogène, un groupe alcényle, par exemple vinyle, cycloalcényle, alkyle, ou cycloalkyle, de préférence en en C₁-C₂₀, un groupe alcoxy ou thioalcoxy, de préférence en en C₁-C₂₀, un groupe poly(alkylènoxyde) avec jusqu'à 10 unités alkylènoxydes terminé par un hydroxyle ou un alkyle (C₁-C₁₂), un groupe aryle, un groupe aryloxy ou thioaryloxy,
         R⁶¹ représente un hydrogène, un groupe alkyle, alcényle, cycloalcényle ou cycloalkyle, de préférence en C₁-C₂₀, ou un groupe aryle,
         k⁷¹ représente un hydrogène, un groupe alkyle, alcényle, cycloalcényle ou cycloalkyle, de préférence en C₁-C₂₀, ou un groupe aryle; et
   (3) les sels organométalliques de formule suivante:

      (L₁L₂L₃M^{)+q} (**II**)

      formule dans laquelle :
      - M représente un métal du groupe 4 à 10, notamment du fer, manganèse, chrome, cobalt,
      - L1 représente 1 ligand lié au métal M par des électrons π, ligand choisi parmi les ligands η³-alkyl, η⁵- cyclopendadiènyl et η⁷-cycloheptratriènyl et les composés η⁶- aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons π,
      - L2 représente un ligand lié au métal M par des électrons π, ligand choisi parmi les ligands η⁷-cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π,
      - L3 représente de 0 à 3 ligands identiques ou différents liés au métal M par des électrons σ, ligand(s) choisi(s) parmi CO et N02+ ; la charge électronique totale q du complexe à laquelle contribuent L1, L2 et L3 et la charge ionique du métal M étant positive et égale à 1 ou 2 ; et
b) ceux dont l'entité anionique borate a pour formulé

   [BXₐ R_{b}]⁻ (**III**)

   formule dans laquelle :
   - a et b sont des nombres entiers allant pour a de 0 à 3 et pour b de 1 à 4 avec a+b=4,
   - les symboles X représentent : .
      * un atome d'halogène (chlore, fluor) avec a = 0 à 3, ou
      * une fonction OH avec a = 0 à 2,
   - les symboles R sont identiques ou différents et représentent :
      - un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
      - un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10, ou
      - un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO₂, CN, quelle que soit l'entité cationique.

Sans que cela ne soit limitatif, sont données ci-après plus de précisions quant aux sous classes de borate d'onium et de borate de sels organométalliques plus particulièrement préférés dans le cadre de l'utilisation conforme à l'invention.

Selon une première variante préférée de l'invention, les espèces de l'entité anionique borate qui conviennent tout particulièrement sont les suivantes :

| | | | |
|---|---|---|---|
| 1': | [B(C₆F₅)₄]⁻ | 5': | [B(C₆H₃(CF3)₂)₄]⁻ |
| 2': | [(C₆F₅)₂BF₂]⁻ | 6': | [B(C₆H₃F₂)₄]⁻ |
| 3': | [B(C₆H₄CF₃)₄]⁻ | 7': | [C₆F₅BF₃]⁻ |
| 4': | [B(C₆F₄OCF₃)₄]⁻. | | |

Selon une deuxième variante préférée de l'invention, les sels d'onium de formule (S-26) utilisables sont décrits dans de nombreux documents notamment dans les brevets US-A-4 026 705, US-A-4 032 673, US-A-4 069 056, US-A-4 136 102, US-A-4 173 476. Parmi ceux-ci, on privilégiera tout particulièrement les cations suivants :

[(C₈H₁₇)-O- (C₆H₄)-I- C₆H₅)]⁺ ; [C₁₂H₂₅- (C₆H₄)-I-C₆H₅]⁺ ; [(C₈H₁₇-O- (C₆H₄))₂I]⁺

[(C₈H₁₇)-O- (C₆H₄)-I- C₆H₅)]⁺ ; [(C₆H₅)₂S-(C₆H₄)-O-C₈H₁₇]⁺

[CH₃- C₆H₄-I- C₆H₄-CH₂CH(CH₃)₂]⁺ ; [(C₁₂H₂₅-(C₆H₄)-I-(C₆H₄)-CH-(CH₃)₂)]⁺

[(C₁₂H₂₅- C₆H₄)₂I] ⁺ ; [(C₆H₅)₃ S]⁺ ; [CH₃-(C₆H₄)-I-(C₆H₄)-CH(CH₃)₂]⁺

(η5 - cyclopentadiènyle) (η6 - toluène) Fe⁺ ; (η5 - cyclopentadiènyle) (η6 - cumène) Fe⁺,

(η5 - cyclopentadiènyle) (η6 - methyl-1-naphtalène) Fe⁺;

[(C₆H₅)-S- C₆H₄-S-(C₆H₅)₂]⁺ ; [(CH₃-(C₆H₄)-I-(C₆H₄)-OC₂H₅]⁺ ;

[(CₙH₂ₙ₊₁- C₆H₄)₂I] ⁺ (avec pour le groupement CₙH₂ₙ₊₁, n= 1 à 18 et est linéaire ou ramifié).

Selon une troisième variante préférée, les sels organométalliques (3) de formule (S-27) utilisables sont décrits dans les documents US-A-4 973 722, US-A-4 992 572, EP-A-203 829, EP-A-323 584 et EP-A-354 181. Les sels organométalliques plus volontiers retenus selon l'invention sont notamment:
- le (η⁵ - cyclopentadiènyle) (η⁶ - toluène) Fe⁺,
- le (η5 - cyclopentadiènyle) (η⁶ - méthyl-1-naphtalène) Fe⁺,
- le (η⁵ - cyclopentadiènyle) (η⁶ - cumène) Fe⁺,
- le bis (η⁶ - mesitylène) Fe⁺,
- le bis (η⁶ - benzène) Cr⁺

En accord avec ces trois variantes préférées, on peut citer, à titre d'exemples de photoamorceurs du type borates d'onium, les produits suivants :

**(P-16)** : [(C₈H₁₇)-O- C₆H₄-I- C₆H₅)]⁺ [B(C₆F₅)₄]⁻ ;

**(P-17)** : [C₁₂H₂₅- C₆H₄-I-C_{6H5}]⁺, [B(C₆F₅)₄]⁻ ;

**(P-18)** : [(C₈H₁₇-O- C₆H₄)₂I]⁺, [B(C₆F₅)₄]⁻;

**(P-19)** : [(C₈H₁₇)-O- C₆H₄-I- C₆H₅)]⁺, [B(C₆F₅)₄]⁻;

**(P-20) :** [(C₆H₅)₂S- C₆H₄-O-C₈H17]⁺, [B(C₆H₄CF₃)₄]⁻;

**(P-21)** : [(C₁₂H₂₅- C₆H₄)₂I]⁺, [B (C₆F₅)₄]⁻;

**(P-22)** : (CH₃-C₆H₄-I- C₆H₄-CH(CH₃)₂]⁺, [B(C₆F₅)₄]⁻;

**(P-23)** : (η5-cyclopentadiènyle) (η6-toluène) Fe⁺, [B(C₆F₅)₄]⁻;

**(P-24)** : (η5-cyclopentadiènyle) (η6-methyl-1-naphtalène)Fe⁺, [B(C₆F₅)₄]⁻;

**(P-25)** : (η5-cyclopentadiènyle) (η6 - cumène) Fe⁺, [B(C₆F₅)₄]⁻ ;

**(P-26)** : [C₁₂H₂₅-C₆H₄)₂I]⁺, [B(C₆H₃(CP₃)₂]⁻;

**(P-27)** : [CH₃-C₆H₄-I-C₆H₄-CH₂CH(CH₃)₂]⁺; [B(C₆F₅)₄]⁻;

**(P-28)** : [CH₃-C₆H₄-I-C₆H₄-CH₂CH(CH₃)2]⁺, [B(C₆H₃(CF₃)₂)₄]⁻;

et

**(P-29**) : [CH₃-C₆H₄-I-C₆H₄-CH(CH₃)₂]⁺, [B(C₆H₃(CF₃)₂)₄]⁻,

Comme autre référence littéraire pour définir les borates d'onium (1) et (2) et les borates de sels organométalliques (3), on peut citer l'ensemble du contenu des demandes de brevet EP 0 562 897 et 0 562 922. Ce contenu est intégralement incorporé par référence dans le présent exposé.

Comme autre exemple de sel d'onium utilisable comme photoamorceur, on peut citer ceux divulgués dans les brevets américains US 4 138 255 et US 4 310 469.

On peut également utiliser d'autres photoamorceurs cationiques, e. g.:
- les sels d'iodonium hexafluorophosphate ou hexafluoro-antimonate, tels que :
   - [CH₃-[(C₆H₄)-I-[(C₆H₄)-CH(CH₃)₂]⁺ [PF₆]⁻ ;
   - [CH₃-(C₆H₄)-I-(C₆H₄)-CH₂CH(CH₃)₂]⁺ [PF₆]⁻ ;
   - [(C₁₂H₂₅- C₆H₄)₂I]⁺ [PF₆]⁻ ou
- les sels de ferrocénium de ces différents anions.

Le photosensibilisateur contenu au sein de la composition dentaire selon l'invention peut être de nature très variée. Dans le cadre de l'invention celui-ci répond notamment à l'une des formule (**IV**) à (**XXIV**) suivantes : 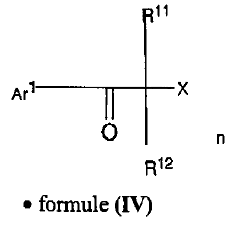 dans laquelle :
- lorsque n = 1, Ar¹ représente un radical aryle contenant de 6 à 18 atomes de carbone, un radical tétrahydronaphtyle, thiényle, pyridyle ou furyle ou un radical phényle porteur d'un ou plusieurs substituants choisis dans le groupe constitué de F, Cl, Br, CN, OH, les alkyles linéaires ou ramifiés en C₁-C₁₂, -CF³, -OR¹³, -OPhényle, -SR¹³, -SPhényle, -SO₂Phényle, -COOR¹³, -O-(CH₂-CH=CH₂), -O(CH₂H₄-O)ₘ-H, -O(C₃H₆O)ₘ-H, m étant compris entre 1 et 100,
- lorsque n = 2, **Ar₁** représente un radical arylène en C₆-C₁₂ ou un radical phénylène-T-phényléne, où T représente -O-, -S-, -SO₂- ou -CH₂-,
- **X** représente un groupe -OR¹⁴ ou -OSiR¹⁵(R¹⁶)₂ ou forme, avec R¹¹, un groupe -O-CHT(R¹⁷ )-,
- **R¹¹** représente un radical alkyle linéaire ou ramifié en C₁-C₈ non substitué ou porteur d'un groupe -OH, -OR¹³, acyloxy en C₂-C₈, -CF³, ou -CN, un radical alcényle en C₃ ou C₄, un radical aryle en C₆ à C₁₈, un radical phénylalkyle en C₇ à C₉,
- **R¹²** a l'une des significations données pour R¹¹ ou représente un radical - CH₂CH₂R¹⁸, ou encore forme avec R¹¹, un radical alkylène en C₂-C₈ ou un radical oxa-alkylène ou aza-alkylène en C₃-C₉,
- **R¹³** représente un radical alkyle inférieur contenant de 1 à 12 atomes de carbone,
- **R¹⁴** représente un atome d'hydrogène, un radical alkyle en C₁-C₁₂, un radical alkyle en C₂-C₆ porteur d'un groupe -OH, -OR¹³ ou CN, un radical alcényle en C₃-C₆, un radical cyclohexyle ou benzyle, un radical phényle éventuellement substitué par un atome de chlore ou un radical alkyle linéaire
ou ramifié en C₁-C₁₂, ou un radical tétrahydropyrannyle-2,
- **R¹⁵** et **R¹⁶** sont identiques ou différents et représentent chacun un radical alkyle en C₁-C₄ ou un radical phényle,
- **R¹⁷** représente un atome d'hydrogène, un radical alkyle en C₁-C₈ ou un radical phényle,
- **R¹⁸** représente un radical -CONH₂, -CONHR¹³, -CON(R¹³)₂, - P(O)(OR¹³)₂ ou pyridyle-2 : 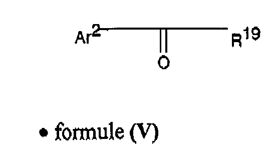
dans laquelle :
- Ar² a la même signification que Ar¹ de la formule (**IV**) dans le cas où n = 1,
- **R¹⁹** représente un radical choisi parmi le groupe constitué d'un radical **Ar²**, un radical alkyle linéaire ou ramifié en C₁-C₁₂, un radical cycloalkyle en C₆-C₁₂ et un radical cycloalkyle formant un cycle en C₆-C₁₂) avec le carbone de la cétone ou un carbone du radical **Ar²**, ces radicaux pouvant être substitués par un ou plusieurs substituants choisis dans le groupe constitué de -F, -Cl, -Br, -CN, -OH, -CF₃, -OR¹³, -SR¹³, -COOR¹³, les radicaux alkyles linéaires ou ramifiés en C₁-C₁₂ porteurs éventuellement d'un groupe -OH, -OR¹³ et/ou -CN, et les radicaux alcényles linéaires ou ramifiés en C₁-C₈ ; 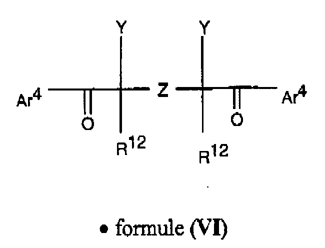
dans laquelle :
- **R¹²**, identiques ou différents, ont les mêmes significations que dans la formule (IV),
- **Y**, identiques ou différents, représentent X et/ou R⁴,
- **Z** représente :
   - une liaison directe,
   - un radical divalent alkylène en C₁-C₆, ou un radical phénylène, diphénylène ou phénylène-T-phénylène (T : alkyle linéaire ou ramifié en C₁-C₁₂), ou encore forme, avec les deux substituants R¹² et les deux atomes de carbone porteurs de ces substituants, un noyau de cyclopentane ou de cyclohexane,
      un groupe divalent -O-R¹⁹-O-, -O-SiR¹⁵R¹⁶-O-SiR¹⁵R¹⁶-O-, ou -O-SiR¹⁵R¹⁶-O-,
- **R²⁰** représente un radical alkylène en C₂-C₈, alcénylène en C₄-C₆ ou xylylène,
- ou bien encore l'entité: 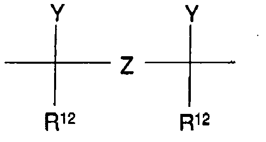 correspond à -O-O-
- et **Ar⁴** a la même signification que Ar¹ de la formulé (**IV**) dans le cas où n = 1.

• famille des thioxanthones de formule (**VII**) : 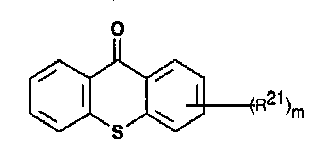
   - m = 0 à 8,
   - **R²¹**, identique(s) ou différents) substituants sur le(s) noyau(x) aromatique(s), représentent un radical alkyle linéaire ou ramifié en C1-C12, un radical cycloalkyle en C6-C12, un radical Ar¹, un atome d'halogène, un groupement -OH, -OR¹³ ,-CN, -NO₂, -COOR¹³,-OCOR¹³,-N(R¹³)₂ ,
   - O-R¹³-(NR¹³)₂ -CHO, -O-phényle, -CF₃, -SR¹³, -S-phényle , -SO₂-phényle , -O-alcényle ou -Si(R¹³)₃.
• famille des xanthènes de formule (**VIII**) : 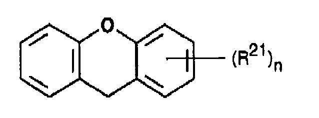 n = 0 à 8
• famille des xanthones de formule (**IX**): 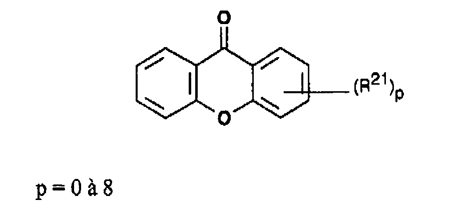
• famille du naphtalène de formule (**X**): 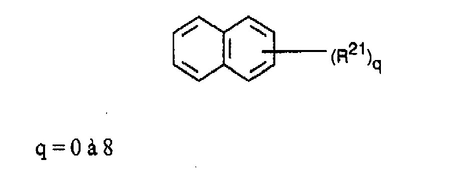
• famille de l'anthracène de formule (**XI**) : 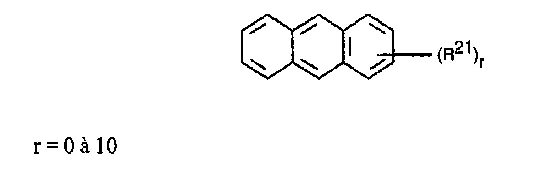
• famille du phénanthrène de formule (**XII**) : 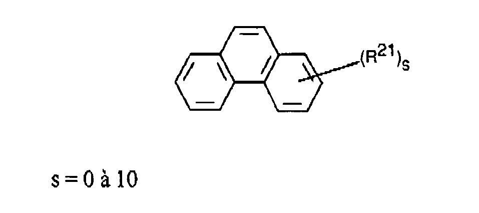
• famille du pyrène de formule (**XIII**) : 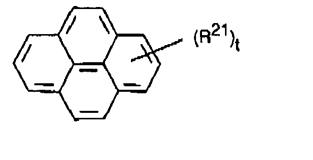 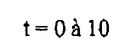
• famille du fluorène de formule (**XIV**) : 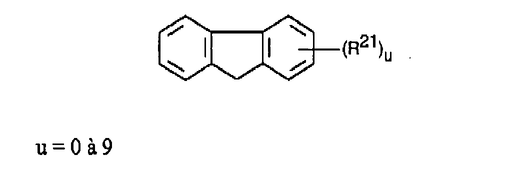
• famille du fluoranthène de formule (**XV**) : 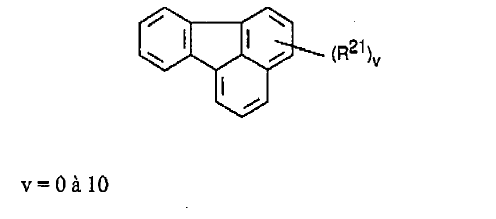
• famille du chrysène de formule (**XVI**) : 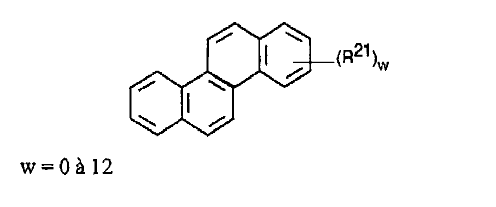
• famille de la fluorène de formule (**XVII**) : 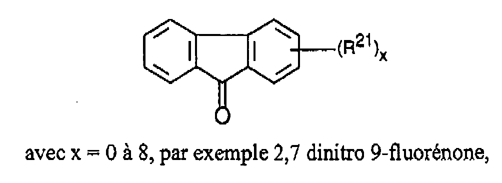
• famille de la chromone de formule (**XVIII**) : 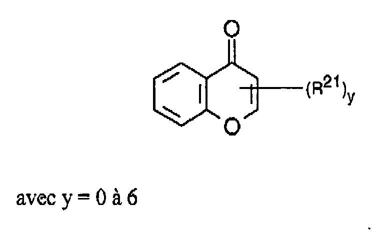
• famille de l'éosine de formule (**XIX**) : 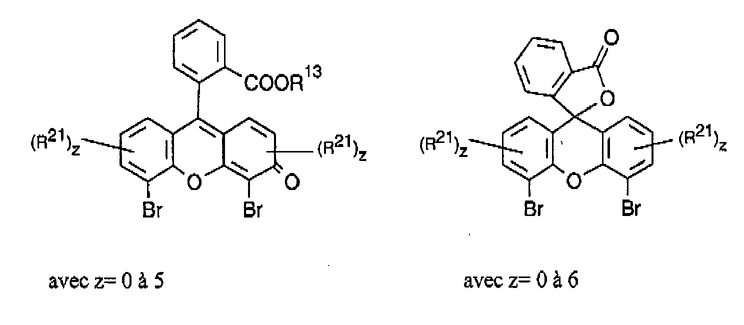
• famille de l'érythrosine de formule (**XX**) : 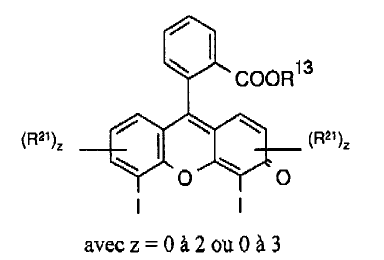
• famille de l'érythrosine de formule (**XXI**) : 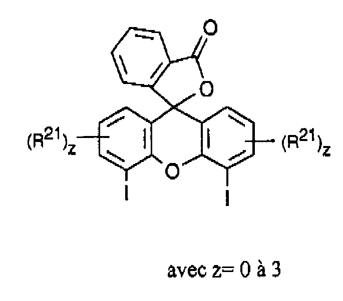
• famille des biscoumarines de formule (**XXII**) : 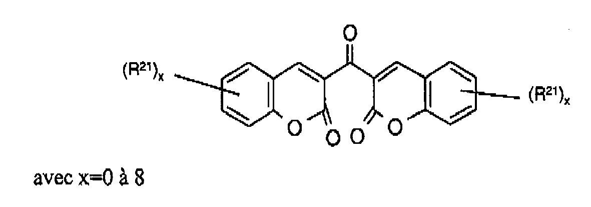
• famille des thioxanthones de formule (**XXIII**) : 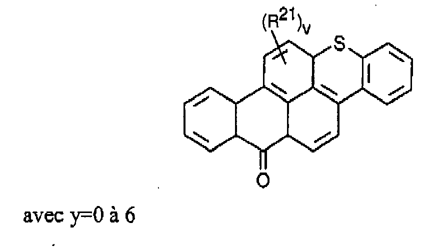
• famille des thioxanthones de formule (**XXIV**) :

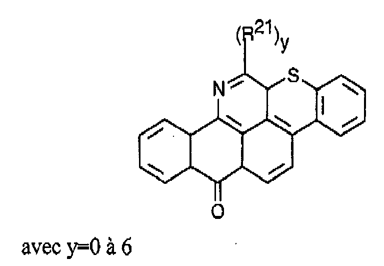

Pour les formules (**VIII**) à (**XXIII**), le groupement **R²¹** a la même définition que pour la molécule (**VII**).

D'autres sensibilisateurs sont utilisables. Notamment, on peut utiliser les photosensibilisateurs décrits dans les documents US 4,939,069; US 4,278,751; US 4,147,552 ainsi que le groupe constitué par les composés de la famille des coumarines, dicétones-conjuguées, fluorones, aminocétones, cétones para-aminostyrylique ainsi que leurs mélanges.

Selon un mode préférentiel, le photosensibilisateur (**E**) est choisi parmi le groupe constitué par les composés de la classe des anthracènes, thioxanthones, camphorquinones, et phénanthrènequinone ainsi que leurs mélanges.

Selon un autre mode préférentiel, la composition dentaire comprend comme photosensibilisateur (**E**) un sel d'une thioxanthone substituée par au moins un groupement comprenant une fonction ammonium. L'utilisation de ce type de photosensibilateur a pour avantage d'éviter les colorations parasitaires lorsque la composition dentaire est réticulée pour la réalisation de prothèse dentaire.

Selon une première variante de l'invention, l'anion asssocié du sel de la thioxanthone substituée par au moins un groupement comprenant une fonction ammonium est choisi parmi les anions suivants :
- un halogénure, BF₄⁻, PF₆⁻; SbF₆⁻; l'anion (**III**) de formule [BXₐ R_{b}]⁻ tel que défini ci-dessus, R_{f}SO₃⁻; (R_{f}SO₂)C⁻ ou (R_{f}SO₂)₂N⁻ avec R_{f} étant un radical alkyle linéaire ou ramifié, substitué par au moins un atome d'halogène, préférentiellement un atome de fluor.

Selon une deuxième variante de l'invention, le photosensibilisateur (**E**), éventuellement en association avec au moins une camphorquinone, une phénanthrènequinone et/ou un anthracène substitué, a pour formule: 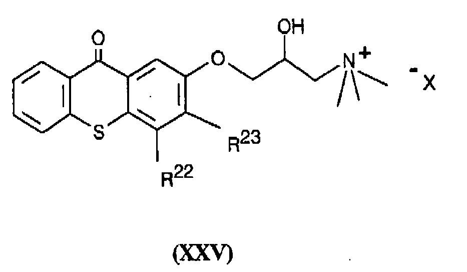 formule dans laquelle :
- **R²²** et **R²³** sont identiques ou différents et représentent un hydrogène - ou un radical alkyle en C1-C10, éventuellement substitué, de préférence R22=R23= méthyle,
- (X⁻) étant une entité anionique, de préférence un halogénure; BF₄⁻, PF₆⁻; SbF₆⁻; l'anion (**III**) de formule [BXₐ R_{b}]⁻ défini comme ci-dessus, RrSO₃⁻; (R_{f}SO₂)₃C⁻ ou (R_{f}SO₂)₂N⁻, avec R_{f} étant un radical alkyle linéaire ou ramifié, substitué par au moins un atome d'halogène, préférentiellement un atome de fluor,
et encore plus préférentiellement (X⁻) est choisi parmi les borates de formules suivantes : [B(C₆H₃(CF₃)₂)₄]⁻ et [B(C₆F₅)₄]⁻

Selon un mode préféré, le photosensibilisateur (**E**), éventuellement en association avec au moins une camphorquinone, une phénanthrènequinone et/ou un anthracène substitué a pour formule:

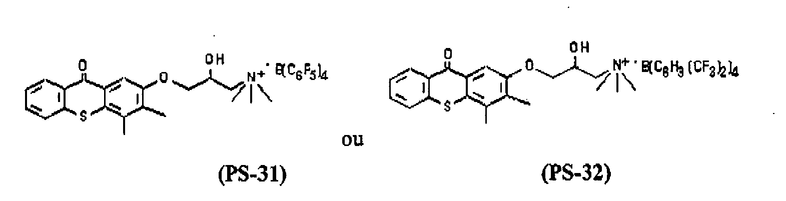

Dans le cadre de la présente invention, les photosensibilisateurs ont une absorption résiduelle de la lumière U.V. comprise entre 200 et 500 nm, de préférence 400 à 500 nm pour les préparations de prothèses dentaires. Pour la restauration dentaire, on préférera un photosensibilisateur ayant une absorption résiduelle de la lumière U.V. au-delà de 400 nm.

Selon une variante préférée, les photosensibilisateurs seront choisis parmi ceux des familles (**VII**), (**X**), (**XI**), (**XIII**), (**XXIII**), (**XXIV**) et (**XXV**). Selon un autre mode de réalisation on choisit le phosensibilisateur parmi le groupe constitué par les composés suivants :
- (**PS-30**) :chlorure de 3-(3,4diméthyl-9-oxo-9thioxanthènen-2-yloxy)-2-hydroxypropyl)-triméthylammonium ;
- (**PS-31**) : tétrakis(pentafluorophényl)borate de 3-(3,4diméthyl-9-oxo-9-thioxanthènen-2-yloxy)-2-hydroxypropyl)-triméthylammonium ;
- (**PS-32**) : tétrakis(bis(trifluoromethyl)phényl)borate de 3-(3,4diméthyl-9-oxo-9-thioxanthènen-2yloxy)2-hydroxypropyl)-triméthylammonium;
- (**PS-33**) : phénanthrènequinone ;
- (**PS-34**) : camphorquinone ;
- (**PS-35**) : Acénaphtènequinone ;
- (**PS-36**) : dibenzoylpéroxyde ;
- (**PS-37**) : 2-ethyl-9,10-diméthoxyanthracène ;
- (**PS-38**) : 9,10-diéthoxyanthracène ;
- (**PS-39**) : 9,10-dibutoxyanthracène ;
- (**PS-40**) : 9-hydroxyméthylanthracène ;
- (**PS-41**) : 2-diméthylaminothioxanthone ;
- (**PS-42**) : 3-éthylcarboxy-7-méthoxythioxanthone ;
- (**PS-43**) : 1-phényl-thio-4-propoxythioxanthone ;
- (**PS-44**) :2-méthoxythioxanthone ;
- (**PS-45**) : 2-(N,Ndiethylaminopropoxy)thioxanthone ;
- (**PS-46**) : 2-isopropylthioxanthone ;
- (**PS-47**) : 1-chloro-4-propoxythioxanthone ;
- (**PS-48**): 4-isopropylthioxanthone ; 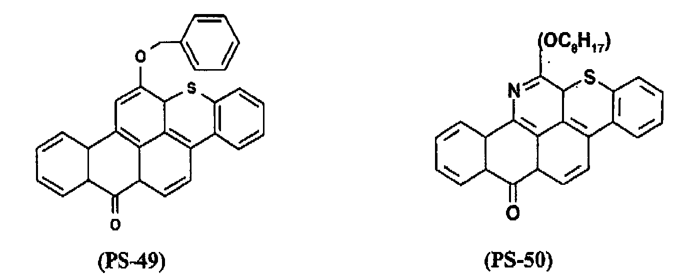
- et leurs mélanges.

Dans le cadre de l'invention, le matériau obtenu après réticulation présente une stabilité au stockage, une tenue mécanique, un module d'élasticité et une résistance à la compression nettement améliorés. Lorsque l'on met en oeuvre les thioxanthones de la famille décrite par la formule (**XXV**), on constate un avantage supplémentaire à utiliser les thioxanthones (**PS-31**) ou (PS-32), éventuellement en association avec au moins une camphorquinone, une phénanthrènequinone et/ou un anthracène substitué, qui est l'absence de coloration résiduelle.

Outre les charges de renforcement, des pigments peuvent être utilisés pour teinter la composition dentaire selon l'utilisation envisagée et les groupes ethniques.

Par exemple, on utilise des pigments rouges en présence de microfibres pour les compositions dentaires utilisées pour la préparation de prothèses dentaires afin de simuler les vaisseaux sanguins.

On emploie aussi des pigments à base d'oxydes métalliques (oxydes de fer et/ou titane et/ou aluminium et/ou zirconium, etc.) pour les compositions dentaires utilisées pour la préparation de matériau de restauration, afin d'obtenir un matériau réticulé de couleur ivoire.

D'autres additifs peuvent être incorporés au sein des compositions dentaires selon l'invention. Par exemple, des biocides, des stabilisants, des agents de flaveur, des plastifiants et des promoteurs d'adhérence.

Parmi les additifs envisageables, on utilisera avantageusement des co-réactifs réticulables et/ou polymérisables de type organique. Ces co-réactifs sont liquides à température ambiante ou thermofusibles à température inférieure à 100°C, et chaque co-réactif comprend au moins deux fonctions réactives tels que oxétane-alcoxy, oxétane-hydroxy, oxétane-alcoxysilyle, carboxy-oxétane, oxétane-oxétane, alcénylether-hydroxy, alcénylether-alcoxysilyle, époxy-alcoxy, époxy-alcoxysilyles, dioxolane-dioxolane- alcool, etc. On peut citer par exemple les résines R70 et R71.

Les compositions dentaires selon l'invention peuvent être utilisées pour de nombreuses applications dentaires, et en particulier dans le domaine des prothèses dentaires, dans le domaine de la restauration dentaire et dans le domaine des dents provisoires.

La composition dentaire selon l'invention se présente de préférence sous la forme d'un seul produit contenant les différents composants ("*monocomposant*") ce qui facilite sa mise en oeuvre, notamment dans le domaine des prothèses dentaires. Eventuellement, la stabilité de ce produit peut être assurée par des dérivés organiques à fonctions amines selon l'enseignement du document WO 98/07798.

Dans le domaine des prothèses dentaires, le produit sous la forme "monocomposant" peut être déposé à l'aide d'une seringue directement sur le modèle en plâtre ou dans une clé. Puis, il est polymérisé (polymérisation par couches successives possibles) à l'aide d'une lampe UV (spectre lumière visible 400 à 500 nm).

En général, il est possible de réaliser en 10 à 15 min une prothèse dentaire durable et esthétique.

Il est à noter que les produits obtenus à partir de la composition dentaire selon invention sont non poreux. Ainsi, après un éventuel polissage à l'aide d'une brosse feutre par exemple, la surface des prothèses dentaires obtenues est lisse et brillante et donc ne nécessite pas d'utilisation de vernis.

Les applications dans le domaine des prothèses dentaires sont essentiellement celles de la prothèse adjointe, que l'on peut diviser en deux types :
- prothèse totale en cas de patient complètement édenté ;
- prothèse partielle due à l'absence de plusieurs dents se traduisant par soit une prothèse provisoire, soit un appareil squeletté.

Dans le domaine de la restauration dentaire, la composition dentaire selon l'invention peut être utilisée en tant que matériau d'obturation des dents antérieures et postérieures en différentes teintes (par exemple, teintes 'VITA"), rapide et facile à mettre en oeuvre.

La composition dentaire étant non toxique et polymérisable en couches épaisses, il n'est pas indispensable de polymériser le matériau en couches successives. En général, une seule injection de la composition dentaire est suffisante.

Les préparations pour prothèses dentaires et pour matériaux de restauration sont effectuées selon les techniques usuelles du métier.

Dans le cas d'application de la composition dentaire à une dent, soit la dent peut être prétraitée avec agent mordançant puis par un primaire d'accrochage qui peut être lui même photoréticulable ou soit la composition dentaire peut être préparée en mélange avec un primaire d'accrochage avant son utilisation.

L'invention concerne aussi un procédé pour traiter une charge de renforcement dentaire comprenant les étapes suivantes :
a) on mélange en milieu solvant la charge de renforcement dentaire (B) et au moins un agent de couplage organosilicié (F) de formule 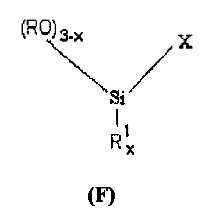 formule dans laquelle :
   - R est un hydrogène ou un radical alkyle linéaire ou ramifié en C1 - C4,
   - R¹ est un radical alkyle linéaire ou ramifié, ou un radical phényle,
      - x est égal à 0,1 1 ou 2, et
      - X étant défini par la formule suivante : 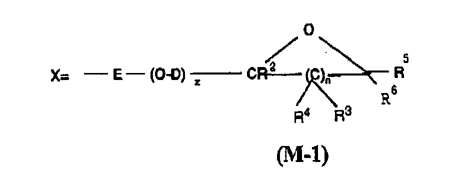 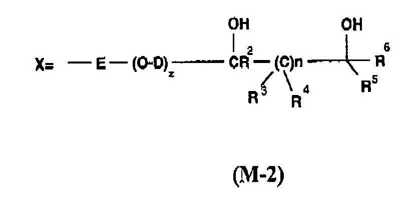 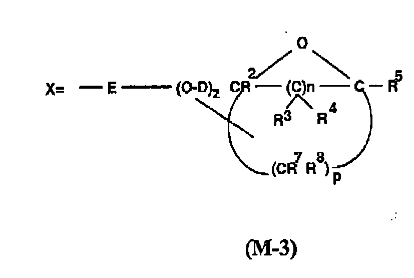 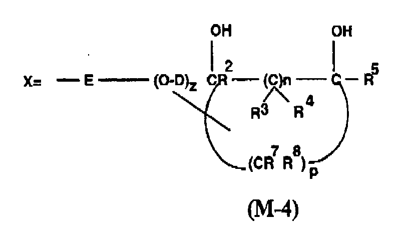 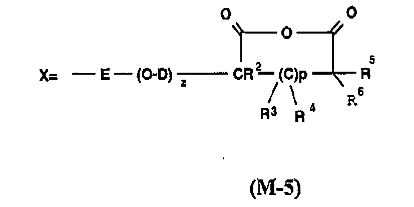 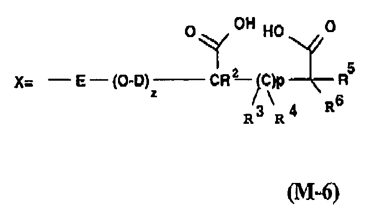 avec :
      - E et D sont des radicaux identiques ou différents choisis parmi les alkyles en C1-C12 linéaires ou ramifiés,
      - z est égal à 0 ou 1,
      - n est égal à 0 ou 1,
   - p est égal à 0,1, 2, 3, 4, 5 ou 6.
   - R², R³, R⁴, R⁵, R⁶, R⁷ et R⁸ qui sont des radicaux identiques ou différents représentant l'hydrogène ou un alkyle linéaire ou ramifié en C1-C12.
b) on évapore le solvant pour obtenir une charge dentaire intermédiaire **(B-1)**,
c) la charge dentaire intermédiaire **(B-1)** subit un traitement thermique de manière à permettre la réaction de couplage entre la charge dentaire intermédiaire **(B-1)** et l'agent de couplage **(F)** et ainsi d'obtenir une charge dentaire intermédiaire **(B-2)**,
d) la charge dentaire intermédiaire **(B-2)** est ensuite mélangée en milieu solvant avec au moins un composé **(G)** qui est un monomère, oligomère ou polymère organique ou un organosiloxane comprenant au moins une fonction oxirane, oxétane, alcényle éther et/ou carbonate,
e) on évapore le solvant pour obtenir une charge dentaire intermédiaire **(B-3)**, et
f) la charge dentaire intermédiaire **(B-3)** subit un traitement thermique de manière à permettre la réaction entre la charge dentaire intermédiaire **(B-3)** et le composé **(G)** et ainsi d'obtenir une charge dentaire traitée **(B-4)**.

Selon un mode préférentiel, le traitement thermique des étapes c) et f) du procédé (I) s'effectue par un chauffage à une température inférieure ou égale à 200°C, de préférence inférieure ou égale à 160°C et encore plus préférentiellement comprise entre 100 et 165°C.

L'agent de couplage organosilicié **(F)**, le composé **(G)** et la charge de renforcement dentaire (B) sont tels que définis dans la composition dentaire selon l'invention.

L'invention concerne aussi une charge de renforcement obtenu par le procédé selon l'invention.

Les Exemples et Tests suivants sont donnés à titre illustratif. Ils permettent notamment de mieux comprendre l'invention et de faire ressortir certains de ses avantages et d'illustrer quelques unes de ses variantes de réalisation.

### Exemples et Tests

### Structures

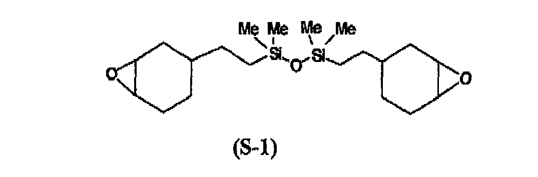

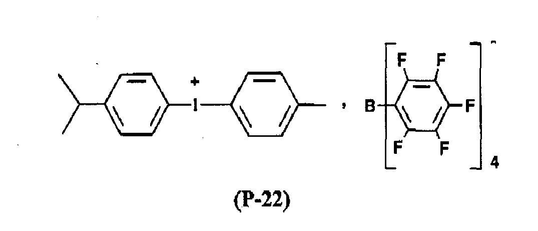

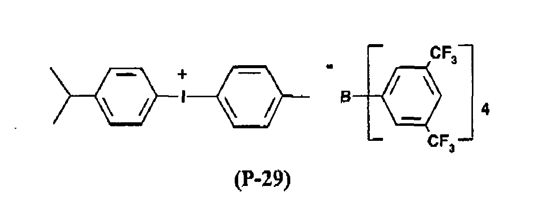

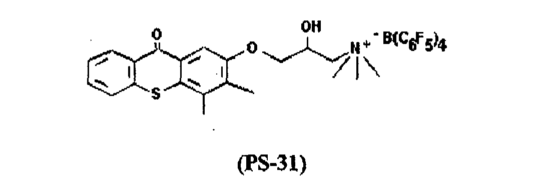

- résine commercialisée par la société Dow-Chemical sous la référence ERLX-4360 (résine de la famille décrite sous la structure **S-104** citée supra),
- Charge **(B-1)** : verre de type quartz (granulométrie = 1.5 µm, SiO₂ 56% ;SrO 14% ;B₂O₃ 14% ; Al₂O₃ 14% ; F 2% ) commercialisé par la société Schott sous la référence G018-163
- Charge **(B-2)** : verre de type quartz (granulométrie =1.5 µm, SiO₂ 55% ;BaO 25% ; B₂O₃ 10% ; Al₂O₃ 10%) commercialisé par la société Schott sous la référence GM27-884.

### Préparation d'une thioxanthone à fonctionnalité ammonium borate (S-61) :

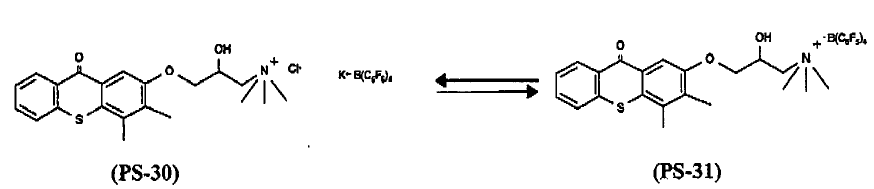

On introduit à l'abri de la lumière dans un flacon opaque 1,02 g de chlorure de 3-(3,4 dimethyl-9-oxo-9H thioxanthen-2-yloxy)-2hydroxypropyl)tritnethylammonium (vendu par la société Aldrich) ; 2,688g de sel de *« Kisbore »* KB(C₆F₅)₄ (préparé par la société Rhodia), 50ml d'isopropanol et on laisse sous agitation magnétique 48 heures à température ambiante. On coule ensuite le mélange dans de l'eau déminéralisée (200ml). Un précipité jaune se forme. La suspension est filtrée sur Büchner et le solide est séché 24h à l'étuve à 100°C. On obtient le sel noté **(PS-31)** (point de fusion 235°C ; maximum d'absorption λmax=397,3nm).

### Exemple 1 (comparatif) Traitement de la charge (B-1)

### a) Traitement de la charge par une agent de couplage à fonctionnalité diol (F-2)

Le silane glycidyloxypropyltriméthoxysilane ou GLYMO de formule **(F-1)** et commercialisé par la société Degussa est hydrolysé en milieu acide suivant la réaction suivante : 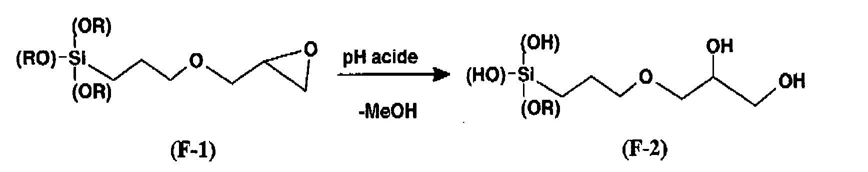 avec R= méthyle

Le composé **(F-2)** est sous une forme plus ou moins polycondensé et est soluble en phase aqueuse.

La solution utilisée est une solution à 40% de silane **(F-2)** avec un pH=3-4

On verse 12.5g de cette solution à 40% de silane **(F-2)** dans un bécher et on complète avec 200g d'eau déminéralisée. On verse 200 g de charge **(B-1)** dans cette solution et on mélange pendant une heure sous agitation à hélice pendant 1heure à température ambiante. On verse l'ensemble dans un cristallisoir et on sèche à l'étuve la charge pendant 16h à 150°C. La charge est ensuite tamisée sur une toile de 250 microns.

### b) Traitement par un agent de couplage à fonctionnalité oxirane

A titre comparatif nous avons effectué l'hydrolyse du silane glycidyloxypropyltnméthoxysilane en milieu neutre et nous avons éliminé une grosse partie du méthanol issu de la réaction d'hydrolyse par distillation. L'analyse par RMN¹H révèle que la fonction époxyde n'est pas hydrolysée lorsqu'on stocke à froid cette solution pendant quelques semaines.

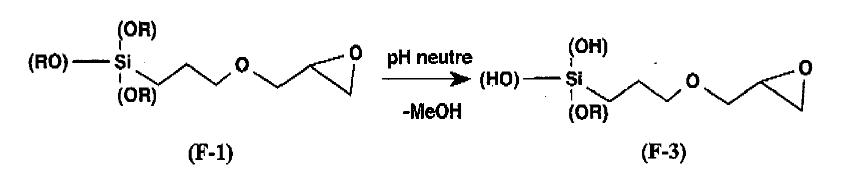

On utilise une solution à 40% de silane **(F-3)** à pH=7.

On verse 12.5g de cette solution à 40% dans un bécher et on complète avec 200g d'eau déminéralisée. On verse 200 g de charge **(B-1)** non traitée dans cette solution et on mélange pendant une heure sous agitation à hélice à température ambiante. On verse l'ensemble dans un cristallisoir et on sèche à l'étuve la charge pendant 16h à 150°C. La charge est ensuite tamisée sur une toile de 250 microns.

### c) Propriétés des compositions dentaires

On charge dans un mélangeur centrifuge (marque Hauschild) 11.625 g de résine siloxane à fonction oxirane **(S-1)**.

On rajoute 1.125g d'une solution à 4% de dispersant (C-1) (commercialisé par la société Byk sous la référence Disperbyk-164^{®}). On agite 16s avec le mélangeur centrifuge à 3000t/min et on rajoute 3 g de trifluorure d'ytterbium. On agite de nouveau 16s avec le mélangeur centrifuge à 3000t/min puis on ajoute 1.25g de système photoamorceur **(P)** renfermant en poids 30 % de photoamorceur **(P-22)** et 0.89 % du photosensibilisateur de formule (**PS-31)** en solution dans **(S-1)**.

On agite 16s avec le mélangeur centrifuge à 3000t/min à température ambiante puis on ajoute 25 g de la charge **(B-1)** :
- non traité **(Formulation 1t)**,
- ou traitée selon a) **(Formulation 1a)** ;
- ou traitée selon b) **(Formulation 1b)**.

On agite 16s avec le mélangeur centrifuge. On rajoute 3 grammes d'une charge de type silice de combustion (B-3) (SiO₂>99% et commercialisée par la société Degussa sous la référence OX50^{®}) puis on agite 16s. On ajoute enfin 5 g d'une charge de type silice de combustion (B-4) commercialisée par la société Degussa sous la référence R202^{®} puis on agite 16s.

On suit l'évolution de la viscosité au cours du temps pour les trois formulations. On note le moment où la formulation de composite est gélifiée et n'est plus manipulable. Les résultats sont présentés dans le Tableau 1.

**Tableau 1.**

| Comparatifs | Stabilité à 25°C |
|---|---|
| **Formulation 1t** | <24h |
| **Formulation 1a** | <24h |
| **Formulation 1b** | 48h |

### Exemple 2 (Invention)

### a) Traitement par la résine à fonction oxirane (S-1) des charges obtenues dans l'exemples 1a)

Une solution à 2,5% en poids de résine à fonction oxirane (S-1) est préparée dans l'acétone.

100g de charge **(B-1)** traitée suivant l'exemple 1a) est versée sur 100g de cette solution à 2.5% et agitée mécaniquement à température ambiante pendant environ une minute. On verse ensuite le mélange dans un cristallisoir et on évapore l'acétone à température ambiante.

On chauffe le résidu pendant 16heures à 150°C afin de polymériser la résine à fonction oxirane (S-1) à la surface de la charge. La charge est ensuite tamisée pour obtenir une poudre.

### b) Traitement par la résine à fonction oxirane (S-1) des charges obtenues dans l'exemples 1b)

Une solution à 2,5% en poids de résine à fonction oxirane (S-1) est préparée dans l'acétone.

100g de charge (B-1) traitée suivant l'exemple 1b) est versé sur 100g de cette solution à 2.5% et agitée mécaniquement à température ambiante pendant environ une minute. On verse ensuite le mélange dans un cristallisoir et on évapore l'acétone à température ambiante. On chauffe le résidu pendant 16heures à 150°C afin de polymériser la résine à fonction oxirane **(S-1)** à la surface de la charge. La charge est ensuite tamisée pour obtenir une poudre.

### c) Propriétés des compositions dentaires

Les deux charges des exemples 2-a) et 2-b) sont formulées de la même façon que dans l'exemple 1-c), le taux de charge global est de 72% en poids par rapport au poids de la composition, pour obtenir deux compositions dentaires (formulations (2a) et (3b) respectivement) dont on suit la stabilité et l'évolution dans le temps.

**Tableau 2.**

| **Invention** | **Traitement** | **Stabilité à 25°C** |
|---|---|---|
| Formulation (2a) | a) Solution à 2.5 % en poids de résine (S-1 ) | 1 mois |
| Formulation (2b) | b) Solution à 2.5 % en poids de résine (S-1) | 1 semaine |

La comparaison des résultats entre :
- les formulation (1a) et (2a), et
- les formulations (1b) et (2b) montrent une nette amélioration de la stabilité.

### Exemple 3 (Invention, double traitement de la charge)

### Variation du taux de résine à fonction oxirane (S-1)

On répète l'exemple 2a) en faisant varier lors du traitement de la charge **(B-1)** le taux de résine à fonction oxirane **(S-1)**.

Puis on formule une composition dentaire comme dans l'exemple 2c).

La composition exacte correspond à la formulation suivante (le taux de charge global est de 66% en poids par rapport au poids de la composition):
- Résine à fonction oxirane **(S-1)** ------------> 29.25 g
- Solution Dispersant à 4%**(C-1)** ------------> 2.25 g
- Système photoamorceur **(P)** (voir exemple 1) : **(P-22)** + **(PS-31)** -> 2.5 g
- Trifluorure d'Ytterbium ------------> 6 g
- Charge **(B-1)** doublement traitée ------------> 50 g
- Charge non traitée **(B-3)** silice de combustion OX5d^{®} ------------> 5 g
- Charge non traitée **(B-4)** silice de combustion R202^{®} ------------> 5 g

On suit l'évolution du taux d'époxy par dosage potentiométrique en solution du composite dans la solution titrante d'acide perchlorique.

**Tableau 3.**

| Compositions dentaires | % en poids de la résine (S-1) dans l'acétone pour le traitement de la charge (B-1): | Taux de conversion des Epoxy % après 26 jours | Stabilité à 25°C (mois) |
|---|---|---|---|
| Formulation témoin (1t) | 0 | Non mesurable | 0 |
| Formulation (3a) | 1 | 23 | 1 |
| Formulation (3b) | 2.5 | 19 | 3 |
| Formulation (3c) | 5 | 16 | >12 mois |

### Exemple 4 (Invention double traitement de la charge): Préparation d'une composition dentaire dont le taux de charge global est de 70 % en poids par rapport au poids de la composition.

On charge dans un mélangeur centrifuge de la marque Hauschild 11.5 g de résine siloxane à fonction oxirane **(S-1)**. On rajoute 2.25g d' une solution à 4% de dispersant **(C-1)** commercialisé par la société Byk sous la référence Disperbyk-164^{®}. On agite 16s avec le mélangeur centrifuge à 3000t/min et on rajoute 3 g de trifluorure d'ytterbium. On agite de nouveau 16s avec le mélangeur centrifuge à 3000t/min puis on ajoute 1.25g d'un système photoamorceur (P) renfermant 30% en poids de photoamorceur **(P-22)** et 0,89% en poids de photosensibilisateur à base de **(PS-31)** dans la résine **(S-1)**. On agite 16s avec le mélangeur centrifuge à 3000t/min à température ambiante puis on ajoute 27 g de la charge **(B-1)** traitée selon l'exemple 2a) puis selon l'exemple 3 (traitement de la charge **(B-1)** avec une solution à 5 % en poids de la résine **(S-1)** dans l'acétone). On agite 16s avec le mélangeur centrifuge. On rajoute 2.5 g de silice de combustion **(B-3)** (commercialisée par la société Degussa sous la référence 0X50^{®} , SiO₂>99%) traitée comme la charge **(B-1)** puis on agite 16s. On ajoute enfin 2.5 g de silice de combustion **(B-4)** (commercialisée par la société Degussa sous la référence R202^{®}) et on agite 16s.

On suit l'évolution de la viscosité au cours du temps pour la formulation avec des charges traitées. Cette dernière est stable pendant plus de 1 an à 25°C.

Des éprouvettes telles que décrites dans la norme ISO 4049 sont réticulées avec une lampe halogène de type Demetron Optilux 500 (temps d'irradiation : 2 fois 40s).

La résistance à la flexion mesurée selon la norme ISO-4049 est de 90 Mpa +/- 10 avec un module de flexion de 9000 MPa.

La profondeur de réticulation est de 4mm après irradiation 40s.

Le retrait volumique calculé à partir des densités mesurées avant et après polymérisation est de : -1,6% +/- 0,2.

La composition exacte correspond à la formulation suivante (le taux de charge global est de 70 % en poids par rapport au poids de la composition):
- Résine à fonction oxirane **(S-1)** ------------> 11,5 g
- Solution Dispersant **(C-1)** ------------> 2.25 g
- Système photoamorceur (P): **(P22)** + **(PS-31**) ------------> 1,25 g
- Trifluorure d'Ytterbium non traité ------------> 3 g
- Charge **(B-1)** doublement traitée ------------> 27 g
- Charge **(B-3)** traitée, silice de combustion OX50^{®} ------------> 2,5 g
- Charge non traitée **(B-4)**, silice de combustion R202^{®} ------------> 2,5 g

### Exemple 5 (Invention, double traitement de la charge)

Nous avons comparé l'impact de la nature des photosensibilisateurs sur la coloration de la composition après irradiation avec une lampe de dentiste. Nous avons reproduit l'exemple 4 en modifiant la nature et la quantité du photosensibilisateur introduit dans la composition.

Les compositions dentaires décrites dans les exemples 2 à 5 sont réticulées sur une épaisseur de 2 à 3 mm avec un temps d'irradiation compris entre 30s et 1minute à l'aide d'une lampe Optilux Demetron. On mesure à l'aide d'un chromamètre ou colorimètre Minolta CR200 les valeurs L*,a*,b* après ¼ heure de réticulation sur un fond blanc et après 5 jours après réticulation .

On en déduit l'écart chromatique obtenu Δc défini tel que ΔC= [(Δa)²+(Δb)²]^{½}.

On constate que la réticulation avec une lampe de dentiste Demetron Optilux 500 des compositions dentaires formulées avec le photosensibilisateur **(PS-31**) seul ou en combinaison avec d'autres photosensibilisateurs par exemple **(PS-39) ; (PS-34)** et **(PS-33)** n'engendre pas de défaut de coloration (pas de phénomène de rosissement lors de l'irradiation avec une valeur chromatique a> 1 immédiatement après irradiation).

**Tableau 4**

| Exemple | Photosensibilisateur (s) | Concentration(s) ppm | T= 1/4 heure; La, b | | | T= 5 jours; La, b | | | Δc |
|---|---|---|---|---|---|---|---|---|---|
| | | | L* | a* | b* | L* | a* | b* | |
| 5-a | PS-47 | 67 | 69,15 | 2,87 | 7,68 | 73,4 | -0,5 | 9,55 | 3,85 |
| 5-b | PS-30 | 220 | 71,1 | 1,70 | 16,2 | 75,85 | -1,51 | 14.75 | 3,5 |
| 5-c | PS-31 | 220 | 76,35 | 0,29 | 12,4 | 78,06 | -0,63 | 11.9 | 1,08 |
| 5d | PS.31 | 170 | 72,8 | 1,01 | 10,02 | 75,24 | -0,4 | 8,73 | 1,51 |
| 5-e | PS-31 ; PS-39 | 170 ; 130 | 73,4 | 0,46 | 14,16 | 76,47 | -1,0 | 13,04 | 1.84 |
| 5-f | PS-31 ; PS-34 ; PS.39 | 170 ; 100 ;120 | 70,1 | -0,21 | 15,69 | 73,69 | -1,33 | 13,94 | 2,07 |
| 5-g | PS-31; PS-33 ; PS-39 | 170 ; 50 ; 110 | 69,08 | 1,41 | 15,58 | 73,42 | -0,79 | 15,07 | 2,26 |
| 5-h | PS-34; PS-39 | 100 ; 160 | Cinétique insuffisante pour la réticulation en 1 minute sur 3 mm | | | | | | |

L'utilisation de l'association de la thioxanthone **(PS-31)** permet de s'affranchir des problèmes de coloration mais aussi des problèmes de cinétique que l'on rencontre en utilisant uniquement la camphorquinone **(PS-34)** ou la camphorquinone en association avec le dérivé anthracénique **(PS-39)** à faible concentration (inférieures à 0,1% cas de l'exemple 5-h).

### Exemple 6 : (Invention, double traitement de la charge)

On répète l'exemple 5 en remplaçant :
- le photoamorceur **(P-22)** par le photoamorceur **(P-29)** ; et
- le système photoamorceur **(P)** par le système photoamorceur **(P')** renfermant 30% en poids de photoamorceur **(P-29)** et 0,89% en poids de photosensibilisateur à base de **(PS-31)** dans la résine **(S-1)**.

Ecart chromatique :
- après 1/4 heure de réticulation sur un fond blanc, L*,a*,b* = 72,1 ; +0,10 ; 9,53 respectivement ; et
- après 5 jours, L*,a*,b* = 75 ; -0,25 ; 8,1 respectivement.

   => Δc= [(Δa)²+(Δb)²]^{½}=1,47.

### Exemple 7 : (invention, double traitement de la charge)

### a) Traitement de la charge (B-2) par le glycidyloxypropyltriméthoxysilane.

300g d'une solution d'acétone à 2,5% de glycidyloxypropyltriméthoxysilane est introduite sous azote sur 300g de verre B-2. Le mélange est ensuite agité vigoureusement à l'aide d'une hélice recouverte de téflon pendant 8h à température ambiante. On filtre ensuite le "slurry" pour récupérer le verre et le gâteau de filtration est tamisé sur toile 200 microns. Le verre tamisé est séché à l'air à 110°C pendant 8 heures.

### b) Traitement de la charge (B-2) issu de l'Exemple 7-a) par le mélange de résines (S-1) et (S-104) à fonctionnalité oxirane .

On introduit 300g de solution dans l'acétone à 3,8 % de (S-1) et 3,8 % de **(S-104)** sur 300g de verre traité selon l'exemple 7-a) puis l'ensemble est agité sous azote à l'aide d'une hélice recouverte de téflon pendant 8 h à température ambiante.

- Le verre est filtré sur filtre polyamide puis tamisé sur toile de 200 microns et enfin séché à 110°C pendant 12 heures.

Le verre traité obtenu (charge doublement traitée) est ensuite utilisé pour réaliser la composition dentaire suivante (le taux global de charge est de 75 % par rapport au poids total de la composition):
- résine à fonction oxirane **(S-1)** 4,5 g
- résine à fonctionnalité oxirane **(S-104)** 4,5g
- Solution Dispersant **(C-1)** 2,25 g
- Système photoamorceur (P) : **(P22)** + (**PS-31)** 1,25 g
- Charge **[B-2]** doublement traitée 32,5 g
- Charge **(B-3)** doublement traitée, silice de combustion 0X50 ^{®} 2,5 g
- Charge non traitée **(B-4)**, silice de combustion R202^{®} 2,5 g

On suit l'évolution du taux d'époxy au cours du temps de la composition. On observe que la composition est stable pendant au moins 9 mois à 25°C avec un taux de conversion des époxy inférieur à 20%.

Des éprouvettes telles que décrites dans la norme ISO 4049 sont réticulées avec une lampe halogène de type Demetron Optilux 500 (temps d'irradiation : 2 fois 40s).

La résistance à la flexion mesurée selon la norme ISO-4049 est de 90 Mpa +/- 10 avec un module de flexion de 8000 MPa +/- 1000.

La profondeur de réticulation est de 4 mm après irradiation de 40s.

Le retrait volumique calculé à partir des densités mesurées avant et après polymérisation est de -1,4% +/- 0,2.

### Exemple 8 : (Invention, double traitement de la charge)

Le verre traité obtenu (charge doublement traitée) selon l'exemple 7b) est ensuite utilisé pour réaliser la composition dentaire suivante (le taux global de charge est de 75 % par rapport au poids total de la composition):
- résine à fonction oxirane **(S-1)** 4,5 g
- résine à fonctionnalité oxirane **(S-104)** 4,5g
- Solution Dispersant **(C-1)** 2,25 g
- Système photoamorceur (P): **(P22)** + **(PS-31)** 1,25 g
- Charge **[B-2]** doublement traitée 32,5 g
- Charge **(B-3)** doublement traitée, silice de combustion 0X50^{®} 2,5 g
- Charge non traitée **(B-5)**, silice colloïdale en solution à environ 40% en poids dans une résine **(S-96)** fournie par la société
   Hanse Chemie 2,5 g

On suit l'évolution du taux d'époxy au cours du temps de la composition. On observe que la composition est stable pendant au moins 9 mois à 25°C avec un taux de conversion des époxy inférieur à 20%.

Des éprouvettes telles que décrites dans la norme ISO 4049 sont réticulées avec une lampe halogène de type Demetron Optilux 500 (temps d'irradiation : 2 fois 40s).

La résistance à la flexion mesurée selon la norme ISO-4049 est de 100 Ma +/- 10 avec un module de flexion de 8000 MPa +/-1000.

La profondeur de réticulation est de 4 mm après irradiation de 40s.

Le retrait volumique calculé à partir des densités mesurées avant et après polymérisation est de -1,4% +/- 0,2.

## Revendications

1. Composition dentaire comprenant :
(1) au moins un composé **(A)** réactif par voie cationique;
(2) au moins une charge dentaire **(B)**;
(3) éventuellement au moins un dispersant **(C)** comprenant au moins un polymère ou copolymère organique ;
(4) au moins un photoamorceur cationique **(D)** ;
(5) et éventuellement au moins un photosensibilisateur **(E)**,
ladite composition étant **caractérisée en ce qu'**elle comprend au moins une charge dentaire **(B)** traitée par un procédé (I) comprenant les étapes suivantes :
a) on mélange en milieu solvant la charge dentaire **(B)** et au moins un agent de couplage organosilicié **(F)** de formule 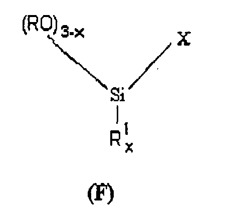 formule dans laquelle :
- R est un hydrogène ou un radical alkyle linéaire ou ramifié en C1 - C4,
- R¹ est un radical alkyle linéaire ou ramifié, ou un radical phényle,
- x est égal à 0, 1 ou 2, et
- X étant défini par les formules suivantes : 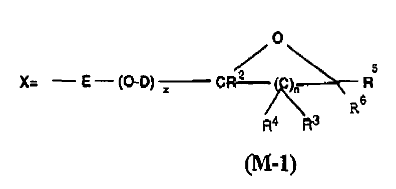 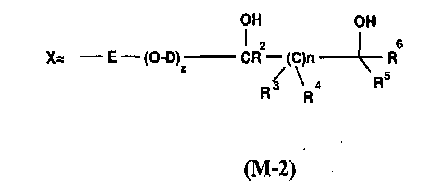 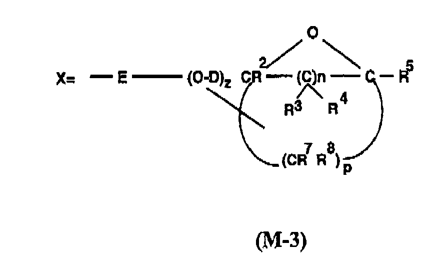 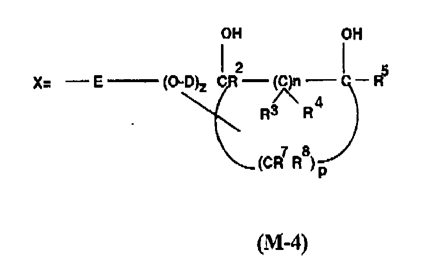 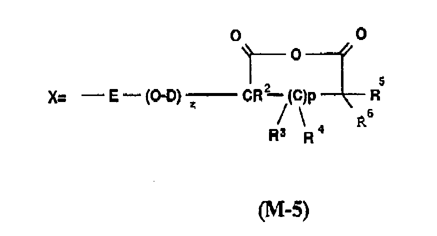 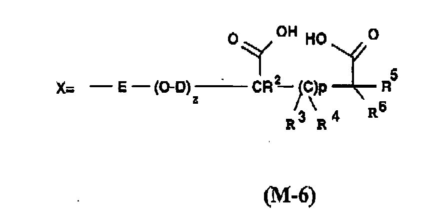 avec :
- E et D sont des radicaux identiques ou différents choisis parmi les alkyles en C1-C12 linéaires ou ramifiés,
- z est égal à 0 ou 1,
- n est égal à 0 ou 1,
- p est égal à 0, 1, 2, 3, 4, 5 ou 6.
- R², R³, R⁴, R⁵, R⁶, R⁷ et R⁸ qui sont des radicaux identiques ou différents représentant l'hydrogène ou un alkyle linéaire ou ramifié en C1-C12.
b) on évapore le solvant pour obtenir une charge dentaire intermédiaire **(B-1)**,
c) la charge dentaire intermédiaire **(B-1)** subit un traitement thermique de manière à permettre la réaction de couplage entre la charge dentaire intermédiaire **(B-1)** et l'agent de couplage **(F)** et ainsi d'obtenir une charge dentaire intermédiaire **(B-2)**,
d) la charge dentaire intermédiaire **(B-2)** est ensuite mélangée en milieu solvant avec au moins un composé **(G)** qui est un monomère, oligomère ou polymère organique ou un organosiloxane comprenant au moins une fonction oxirane, oxétane, alcényle éther et/ou carbonate,
e) on évapore le solvant pour obtenir une charge dentaire intermédiaire **(B-3)**, et
f) la charge dentaire intermédiaire **(B-3)** subit un traitement thermique de manière à permettre la réaction entre la charge dentaire intermédiaire **(B-3)** et le composé **(G)** et ainsi d'obtenir une charge dentaire traitée **(B-4)**.

2. Composition dentaire selon la revendication 1 dans laquelle le traitement thermique des étapes c) et f) du procédé (I) s'effectue par un chauffage à une température inférieure ou égale à 200°C, de préférence inférieure ou égale à 165°C et encore plus préférentiellement comprise entre 100 et 165°C.

3. Composition dentaire selon l'une quelconque des revendications précédentes dans laquelle le taux global des charges dentaires **(B)** représente jusqu'à 85 % en poids par rapport au poids total de la composition dentaire et de préférence entre 60 et 80 % en poids.

4. Composition dentaire selon l'une quelconque des revendications précédentes dans laquelle le traitement de la charge dentaire **(B)** est mis en oeuvre avec jusqu'à 20 % en poids, et de préférence entre 1 et 15 % en poids, du composé **(G)** et/ou jusqu'à 20 % en poids, de préférence entre 1 et 15 % en poids du composé **(F)** par rapport au poids total de la composition dentaire.

5. Composition dentaire selon l'une quelconque des revendications précédentes dans laquelle l'agent de couplage organosilicié **(F)** est choisi parmi le groupe constitué par les composés suivants: glycidyloxypropyltriméthoxysilane, le produit d'hydrolyse du glycidyloxypropyltriméthoxysilane ; glycidyloxypropyltriéthoxysilane, le produit d'hydrolyse en milieu acide du glycidyloxypropyltriéthoxysilane ; le glycidyloxypropyldiméthoxymethylsilane ou le produit d'hydrolyse, béta-(3,4-époxycyclohexyl)éthyltriethoxysilane ou le produit d'hydrolyse, le béta-(3,4-époxycyclohexyl)éthyltriméthoxysilane ou le produit d'hydrolyse.

6. Composition dentaire selon l'une quelconque des revendications précédentes dans laquelle le composé (G) comprend au moins une fonction choisie parmi le groupe constitué par les structures suivantes (M-7) à (M-12) : 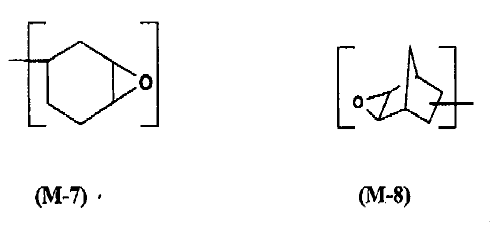 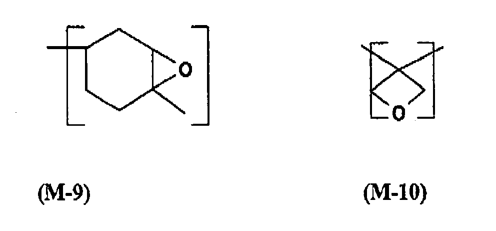
**(M-11)** -(CH₂)₃ 0-CH=CH₂
**(M-12)** -(CH₂)₃O-CH=CH- R"
avec R" représentant un radical alkyle linéaire ou ramifié en C₁-C₆.

7. Composition dentaire selon l'une quelconque des revendications précédentes dans laquelle le composé (G) est un oligomère silicone (G-1) ou un polymère silicone (G-2).

8. Composition dentaire selon la revendication 7 dans laquelle l'oligomère silicone (G-1) et le polymère silicone (G-2) comprennent :
a) au moins un motif de formule: 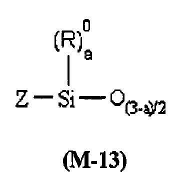 formule dans laquelle :
- a=0, 1 ou 2,
- R⁰, identique ou différent, représente un radical alkyle, cycloalkyle, aryle, vinyle, hydrogéno, alcoxy, de préférence un alkyle inférieur en C1-C6,
- Z, identique ou différent, est un substituant organique comportant au moins une fonction oxirane, alcénylether, oxétane et/ou carbonate, et
b) au moins deux atomes de silicium.

9. Composition dentaire selon la revendication 8 dans laquelle l'oligomère silicone (G-1) et le polymère silicone (G-2) sont choisis parmi le groupe constitué par les composés de formules : 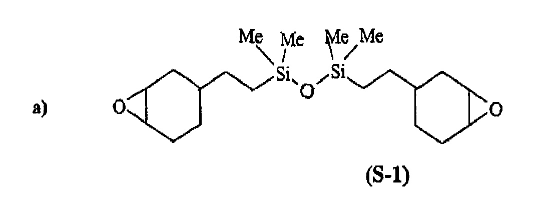 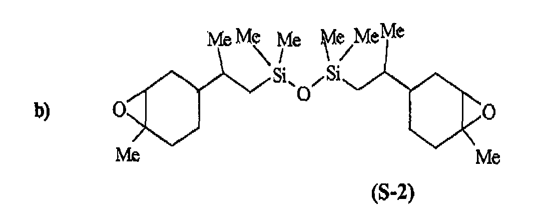 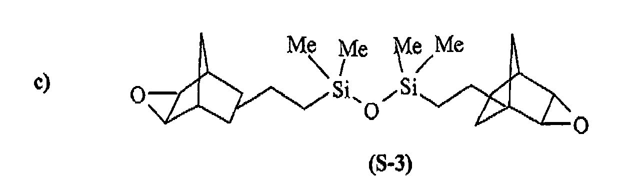 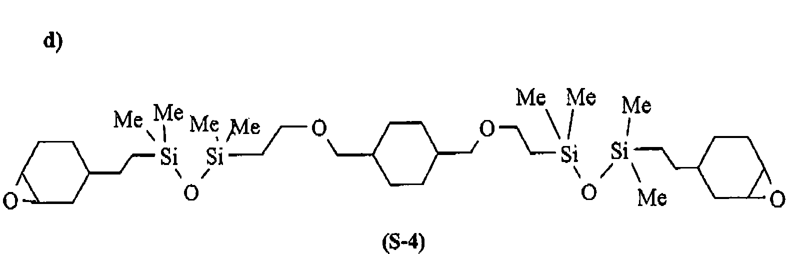 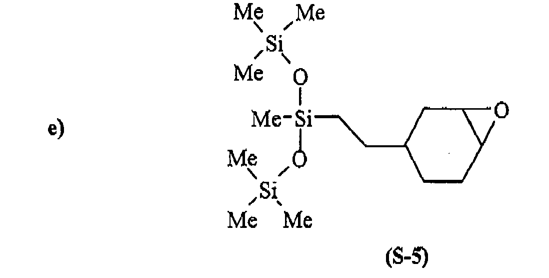 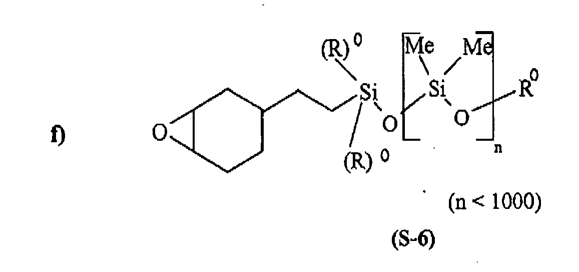 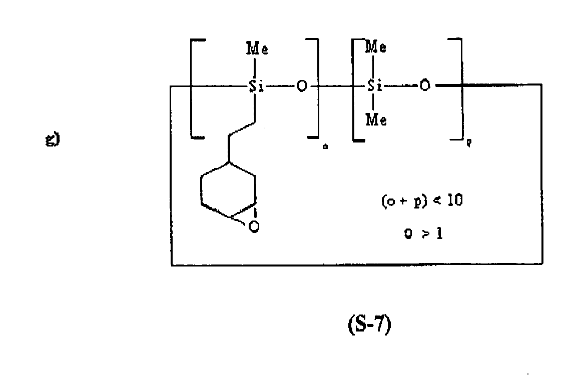 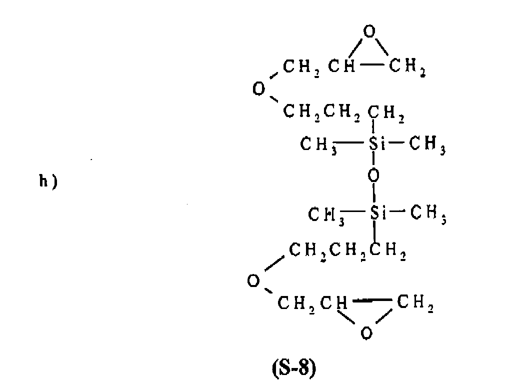 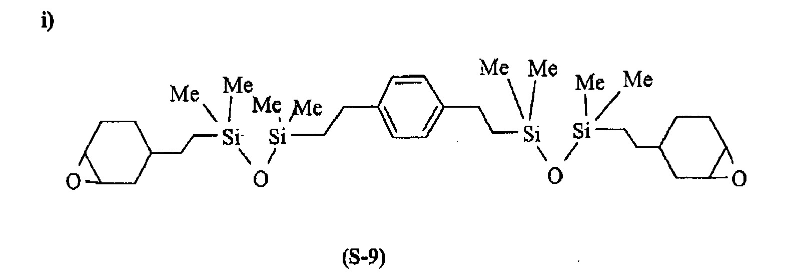 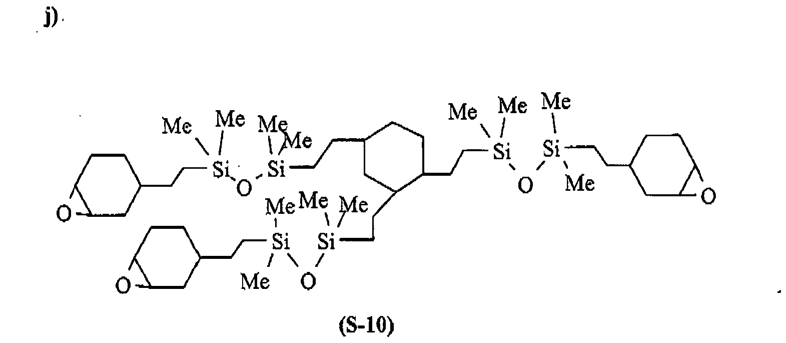 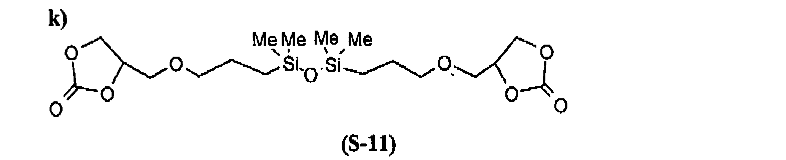 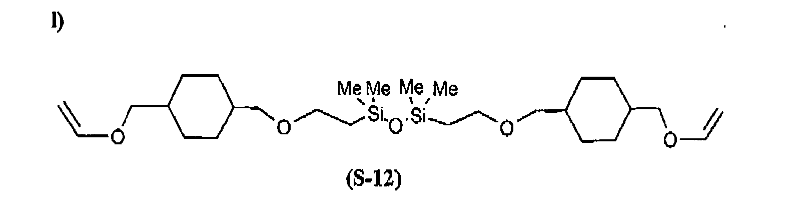 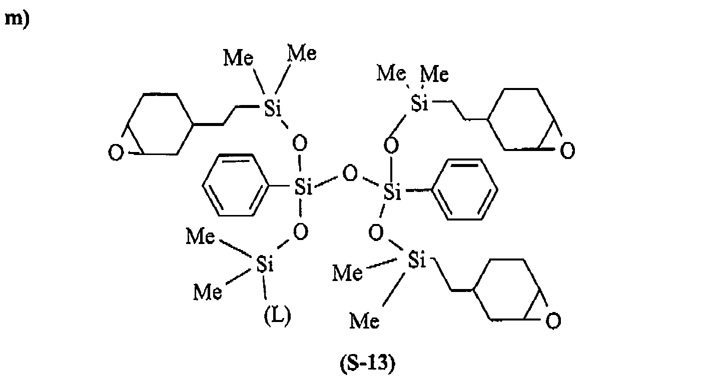 L= H; OH; Me; Phényle; Alkyle C1-C12 ; Cycloalkyle C1-C6 ou les groupements suivants : 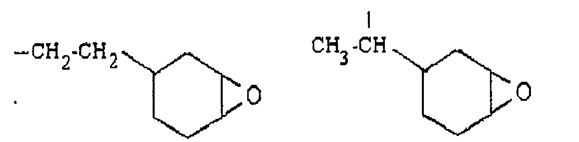 ou 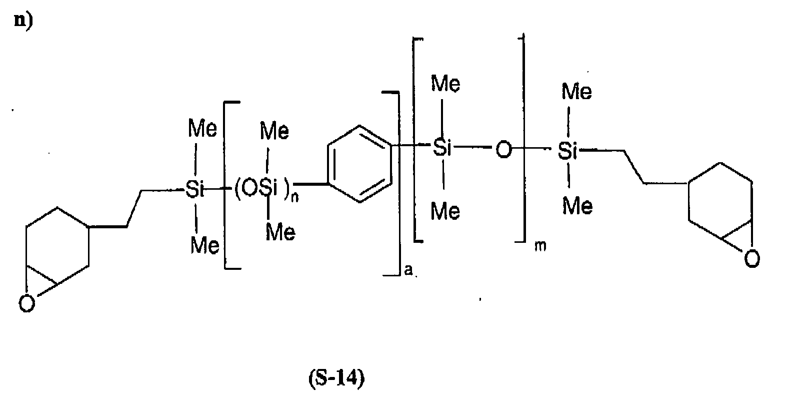 avec n<100 ; a< 100 et m< 100 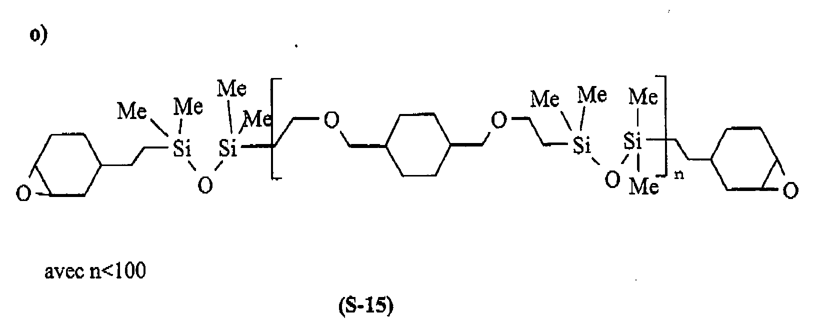 formules dans lesquelles R^{o} ou R₀, identiques ou différents, représente un radical alkyle, cycloalkyle, aryle, de préférence un alkyle inférieure en C1-C6.

10. omposition dentaire selon l'une quelconque des revendications précédentes **caractérisée en ce que** le composé **(A)** réactif par voie cationique est choisi dans le groupe des monomères et/ou (co)polymèreres comprenant :
les époxy, les vinyles éthers, les oxétanes, les spiroorthocarbonates, les spiro-orthoesters et leurs associations.

11. Composition dentaire selon la revendication 10 dans laquelle le composé **(A)** réactif par voie cationique est constitué par au moins un oligomère **(G-1)** ou polymère silicone **(G-2)** réticulable et/ou polymérisable, liquide à température ambiante ou thermofusible à température inférieure à 100 °C, et comprenant :
a) au moins un motif de formule suivante: 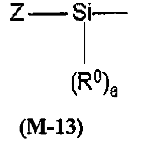 formule dans laquelle :
- a=0, 1 ou2,
- R⁰, identique ou différent, représente un radical alkyle, cycloalkyle, aryle, vinyle, hydrogéno, alcoxy, de préférence un alkyle inférieur en C1-C6,
- Z, identique ou différent, est un substituant organique comportant au moins une fonction réactive oxirane, alcénylether, oxétane, dioxolane et/ou carbonate, et
b) au moins deux atomes de silicium.

12. Composition dentaire selon la revendication 11, **caractérisée en ce que** le motif **(M-13)** comprend des groupes Z choisis parmi le groupe constitué par les radicaux **(R-1)** à **(R-9)** suivants : 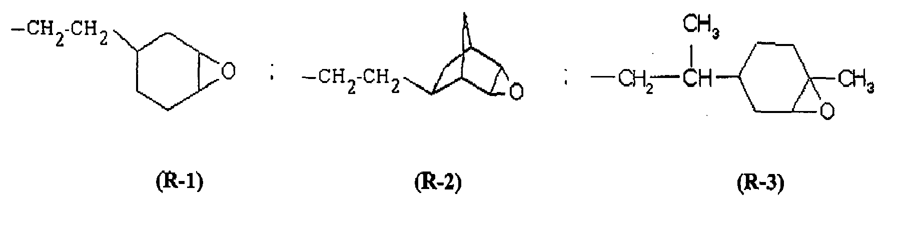 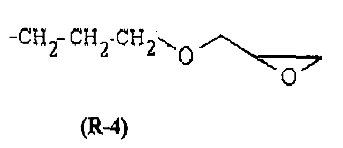 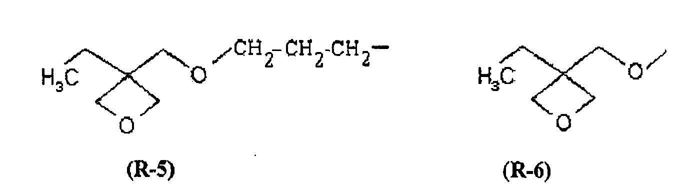 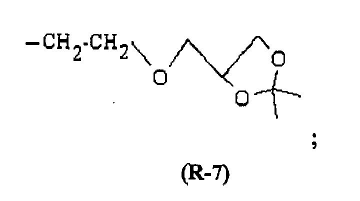
**(R-8)** -(CH₂)₃-O-CH=CH₂
**(R-8)** -(CH₂)-O-CH=CH R"
- avec R" représentant un radical alkyle linéaire ou ramifié en C₁-C₆.

13. Composition dentaire selon l'une quelconque des revendications précédentes, **caractérisée en ce que** le composé **(A)** réactif par voie cationique est choisi parmi le groupe constitué par les composés **(S-1)** à **(S-15)** tels que définis selon la revendication 9.

14. Composition dentaire selon l'une quelconque des revendications précédentes, **caractérisée en ce que** le photoamorceurs cationique (D) est de type borate et est choisi parmi ceux de formule dont:
a) l'entité cationique du borate est sélectionnée parmi :
(1) les sels d'onium de formule:
L(R⁹)ₙ - A - (R¹⁰)m]⁺ **(I)**
formule dans laquelle :
- A représente un élément des groupes 15 à 17 tel que par exemple : I, S, Se, P ou N,
- R⁹ représente un radical aryle carbocyclique ou hétérocyclique en C6-C20, ledit radical hétérocyclique pouvant contenir comme hétéroéléments de l'azote ou du soufre,
- R¹⁰ représente R⁹ ou un radical alkyle ou alkényle linéaire ou ramifié en C1-C30 ; - lesdits radicaux R⁹ et R¹⁰ étant éventuellement substitués par un groupement alcoxy en C1-C25, alkyle en C1-C25, nitro, chloro, bromo, cyano, carboxy, ester ou mercapto,
- m et n sont des nombres entiers, avec n + m = v + 1, v étant la valence de l'élément A,
(2) les sels d'oxoisothiochromanium ; et
(3) les sels organométalliques de formule suivante:
(L₁L₂L₃M^{)+q} **(II)**
formule dans laquelle :
- M représente un métal du groupe 4 à 10, notamment du fer, manganèse, chrome, cobalt,
- Il représente 1 ligand lié au métal M par des électrons π, ligand choisi parmi les ligands η³-alkyl, η⁵- cyclopendadiènyl et η⁷-cycloheptratriènyl et les composés η⁶ - aromatiques choisis parmi les ligands η⁶ -benzène éventuellement substitués et les composés ayant de 2 à 4 cycles condensés, chaque cycle étant capable de contribuer à la couche de valence du métal M par 3 à 8 électrons π,
- L2 représente un ligand lié au métal M par des électrons π, ligand choisi parmi les ligands η⁷-cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π,
- L3 représente de 0 à 3 ligands identiques ou différents liés au métal M par des électrons σ, ligand(s) choisi(s) parmi CO et NO₂⁺ ; la charge électronique totale q du complexe à laquelle contribuent L1, L2 et L3 et la charge ionique du métal M étant positive et égale à 1 ou 2 ; et
b) l'entité anionique borate a pour formule :
[BXₐ R_{b}]⁻ **(III)**
formule dans laquelle :
- a et b sont des nombres entiers allant pour a de 0 à 3 et pour b de 1 à 4 avec a+b=4,
- les symboles X représentent :
* un atome d'halogène (chlore, fluor) avec a = 0 à 3, ou
* une fonction OH avec a = 0 à 2,
- les symboles R sont identiques ou différents et représentent:
- un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
- un radical phényle substitué par au moins un élément ou un groupement électroattracteur notamment atome halogène (fluor tout particulièrement), CF3, OCF₃, NO₂, CN, et ce lorsque l'entité cationique est un complexe organométallique d'un élément des groupes 4 à 10, ou
- un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO₂ CN, quelle que soit l'entité cationique.

15. Composition selon la revendication 14, **caractérisée en ce que** le photoamorceur cationique (D) est choisi parmi le groupe constitué par les composés suivants :
**(P-16)** : [(C₈H₁₇)-Q- C₆H₄-I- C₆H₅)]⁺, [B(C₆F₅)₄]⁻;
**(P-17) :** [C₁₂H₂₅- C₆H₄-I-C₆H₅]⁺, [B(C₆F₅)₄]⁻ ;
**(P-18)** : [(C₈H₁₇-O- C₆H₄)₂I] ⁺, [B(C₆F₅)₄]⁻;
**(P-19)** : [(C₈H₁₇)-O- C₆H₄-I- C₆H₅)]⁺ , [B(C₆F₅)₄]⁻;
**(P-20) :** [(C₆H₅)₂S- C₆H₄-O-C₈H₁₇] ⁺, [B(C₆H₄CF₃)₄]⁻;
**(P-21) :** [(C₁₂H₂₅- C₆H₄)₂I]⁺ , [B (C₆F₅)₄]⁻;
**(P-22) :** [CH₃- C₆H₄-I- C₆H₄-CH(CH₃)₂]⁺ , [B(C₆F₅)₄]⁻ ;
**(P-23) :** (η5 - cyclopentadiènyle) (η6 - toluène) Fe⁺, [B(C₆F₅)₄];
**(P-24) :** (η5 - cyclopentadiènyle) (η6 -methyl-1-naphtalène) Fe⁺, [B(C₆F₅)₄]⁻;
**(P-25) :** (η5 - cyclopentadiènyle) (η6 - cumène) Fe⁺, [B(C₆F₅)₄]⁻;
**(P-26) :** [(C₁₂H₂₅- C₆H₄)2I]⁺ , [B(C₆H₃(CF₃)₂];
**(P-27) :** [CH₃- C₆H₄-I- C₆H₄-CH₂CH(CH₃) 2]⁺, [B(C₆F₅)₄];
**(P-28) :** [CH₃- C₆H₄-I- C₆H₄-CH₂CH(CH₃)₂]⁺, [B(C₆H₃(CF₃)₂)₄] et
**(P-29) :** [CH₃- C₆H₄-I- C₆H₄-CH(CH₃)₂]⁺ , [B(C₆H₃(CF₃)₂)₄]⁻.

16. Composition dentaire selon la revendication 1, **caractérisée en ce que** le photoamorceur cationique (D) est un sel d'iodonium.

17. Composition dentaire selon l'une quelconque des revendications précédentes dans laquelle le photosensibilisateur **(E)** comprend dans sa structure un ou plusieurs noyaux aromatiques substitués ou non, ayant une absorption résiduelle de la lumière comprise entre 200 et 500 nm.

18. Composition dentaire selon l'une quelconque des revendications précédentes dans laquelle le photosensibilisateur **(E)** est choisi parmi le groupe constitué par les composés de la classe des anthracènes, thioxanthones , camphorquinones, et phénanthrènequinone ainsi que leurs mélange.

19. Composition dentaire selon la revendication 18 dans laquelle le photosensibilisateur **(E)** est un sel d'une thioxanthone substituée par au moins un groupement comprenant une fonction ammonium.

20. Composition dentaire selon la revendication 19 **caractérisée en ce que** l'anion asssocié du sel de la thioxanthone substituée par au moins un groupement comprenant une fonction ammonium est choisi parmi les anions suivants : un halogénure, BF₄⁻, PF₆⁻ ; SbF₆⁻ ; l'anion **(III)** de formule [BXₐ R_{b}]⁻ défini selon la revendication 18, R_{f}SO₃⁻; (R_{f}SO₂)₃C⁻ ou (R_{f}SO₂)₂N⁻ avec R_{f} étant un radical alkyle linéaire ou ramifié, substitué par au moins un atome d'halogène, préférentiellement un atome de fluor.

21. Composition dentaire selon l'une quelconque des revendications précédentes dans laquelle le photosensibilisateur **(E)**, éventuellement en association avec au moins une camphorquinone, une phénanthrènequinone et/ou un anthracène substitué, a pour formule: 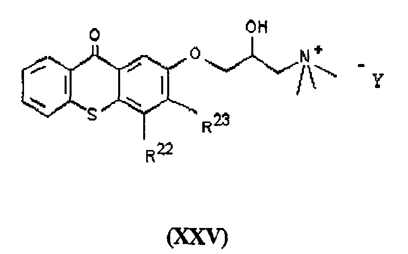 formule dans laquelle :
- R22 et R23 sont identiques ou différents et représentent un hydrogène ou un radical alkyle en C1-C10, éventuellement substitué, de préférence R22=R23= méthyle,
- (X") étant une entité anionique, de préférence un halogénure; BF₄⁻, PF₆ ; SbF₆⁻ ; l'anion **(III)** de formule [BXₐ R_{b}]⁻ défini selon la revendication 16, R_{f}SO₃⁻ ; (R_{f}SO₂)₃C⁻ ou (R_{f}SO₂)₂N⁻, avec R_{f} étant un radical alkyle linéaire ou ramifié, substitué par au moins un atome d'halogène, préférentiellement un atome de fluor,
et encore plus préférentiellement (X⁻) est choisi parmi les borates de formules suivantes : [B(C₆H₃(CF₃)₂)₄]⁻ et [B(C₆F₅)₄]⁻.

22. Composition dentaire selon l'une quelconque des revendications précédentes dans laquelle le photosensibilisateur (E), éventuellement en association avec au moins une camphorquinone, une phénanthrènequinone et/ou un anthracène substitué a pour formule:

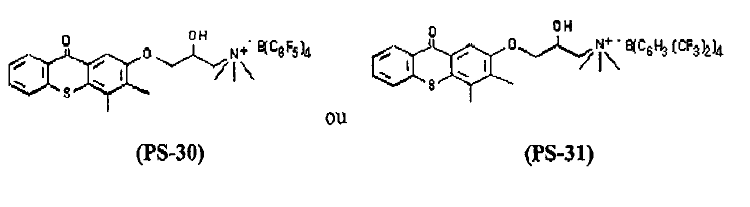

23. Composition dentaire selon l'une quelconque des revendications précédentes dans laquelle le photosensibilisateur (E) est choisi parmi le groupe constitué des composés suivantes:
**(PS-30)** :chlorure de 3-(3,4diméthyl-9-oxo-9thioxanthènen-2-yloxy)-2-hydroxypropyl)-triméthylammonium ;
- **(PS-31)** : tétrakis(pentafluorophényl)borate de 3-(3,4diméthyl-9-oxo-9-thioxanthènen-2-yloxy)-2-hydroxypxopyl)-triméthylammonium ;
- **(PS-32)** : tétrakis(bis(trifluoromethyl)phényl)borate de 3-(3,4diméthyl-9-oxo-9-thioxatathènen-2yloxy)2-hydroxypropyl)-triméthylammonium ;
- **(PS-33)** : phénanthrènequinone ;
- **(PS-34)** : camphorquinone ;
- **(PS-35)** Acénaphtènequinone ;
- **(PS-36)** : dibenzoylpéroxyde ;
- **(PS-37)** 2-ethyl-9,10-diméthoxyanthracene ;
- **(PS-38)** : 9,10-diéthoxyanthracène ;
- **(PS-39)** : 9,10-dibutoxyanthracène ;
- **(PS-40)** 9-hydroxyméthylanthracène;
- **(PS-41)** : 2-diméthylaminothioxanthone ;
- **(PS-42)** : 3-éthylcarboxy-7-méthoxythioxanthone ;
- **(PS-43)** : 1-phényl-thio-4-propoxythioxanthone ;
- **(PS-44)** :2-méthoxythioxanthone ;
- **(PS-45)** : 2-(N,Ndiethylaminopropoxy)thioxanthone ;
- **(PS-46)** : 2-isopropylthioxanthone ;
- **(PS-47)** 1-chloro-4-propoxythioxanthone ;
- **(PS-48)** : 4-isopropylthioxanthone ; 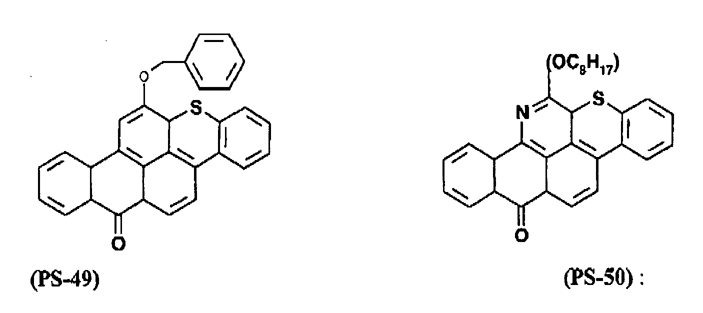
- et leurs mélanges.

24. Composition dentaire selon l'une quelconque des revendications précédentes dans laquelle le photosensibilisateur **(E)** est choisi parmi le groupe constitué par les composés de la famille des coumarines, dicétones, fluorones, aminocétones, cétones para-aminostyrylique ainsi que leurs mélanges.

25. Composition dentaire selon l'une quelconque des revendications précédentes dans laquelle le polymère silicone **(G2)** ou l'oligomère silicone **(G-1)** est associé à une résine époxy organique ou oxétane de formule **(S-103)** ou **(S-1.04)** : 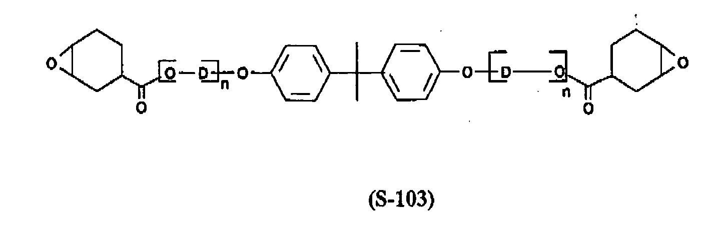 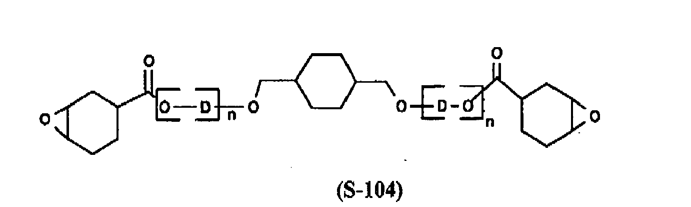 Avec :
- 0≤n≤100 et
- D = radical choisi parmi les alkyles en C1-C12 linéaires ou ramifiés.

26. Composition dentaire selon l'une des revendications précédentes dans laquelle la charge dentaire **(B)** est un verre minéral ou une silice de combustion.

27. Composition dentaire selon l'une quelconque des revendications précédentes, dans laquelle le composé **(G)** est un silane **(G-3)** de formule : 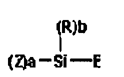 formule dans laqueuse : E= -CH₂- ; -CH = 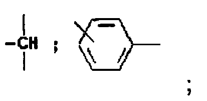
- R, identique ou différent, représente un radical alkyle, cycloalkyle, aryle, vinyle, hydrogéno, alcoxy, de préférence un alkyle inférieur en C1-C6,
- Z, identique ou différent, est un substituant organique comportant au moins une fonction oxirane, alcénylether, oxétane et/ou carbonate, et
- a+b=3.

28. Composition dentaire selon la revendication 27 dans laquelle le silane (G-3) est choisi parmi le groupe constitué par les molécules suivantes :

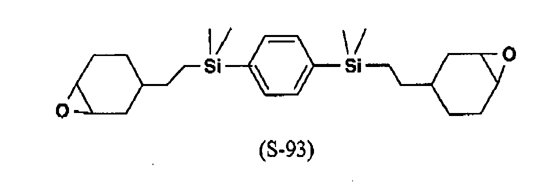

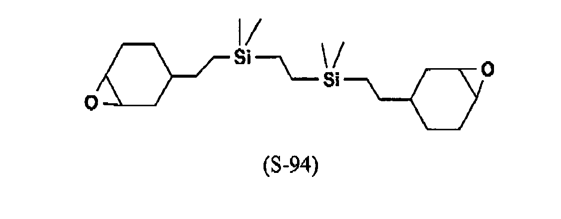

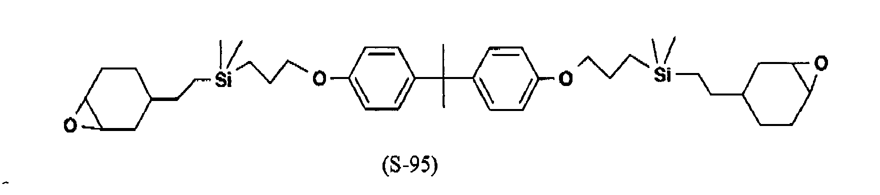

29. Composition dentaire selon l'une des revendications précédentes dans laquelle le composé **(G)** est un composé organique **(G-4)** choisi parmi le groupe constitué par les molécules **(S-96)** à **(S-104)** : 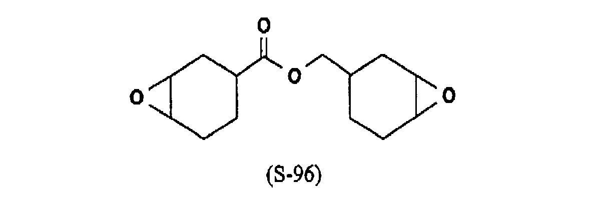 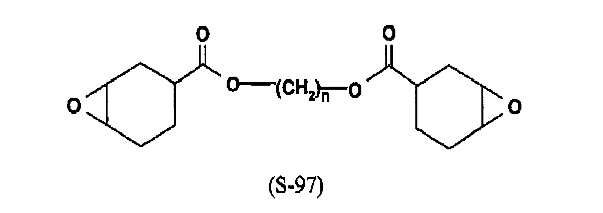 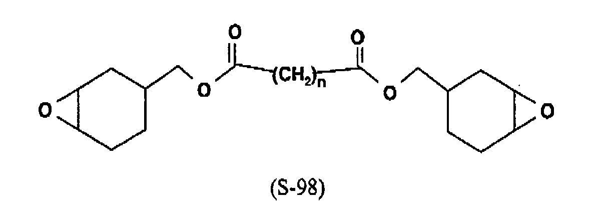 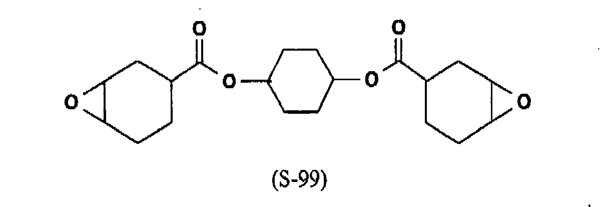 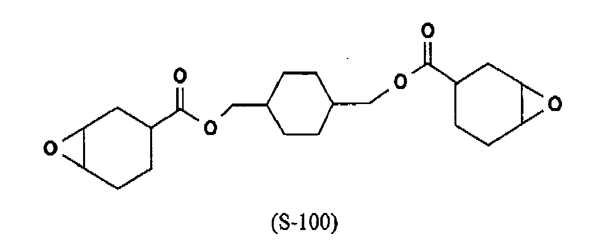 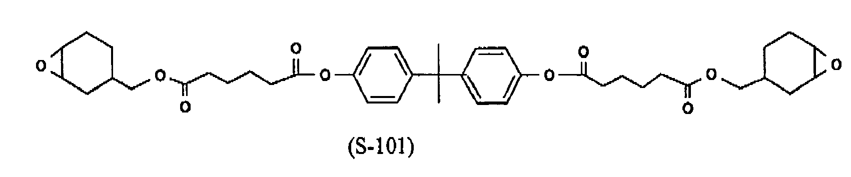 formules dans lesquelles : n est un nombre entier compris entre 1 et 10 (boranes incluses) ; 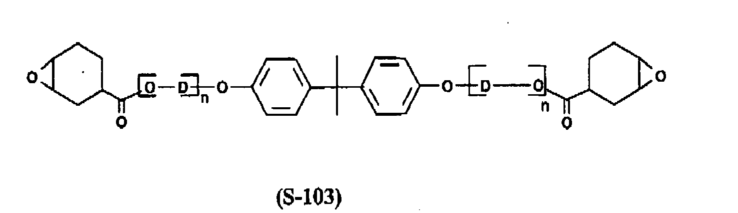 avec n<100 et D= alkyle en C1-C12 linéaires ou ramifiés. 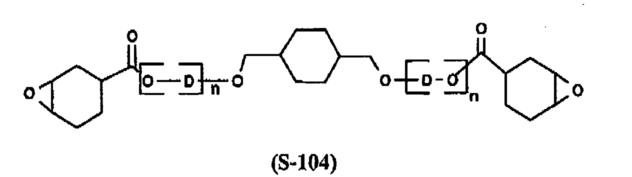 avec n<100 et le groupement D= alkyle en C1-C12 linéaires ou ramifiés.

30. Procédé pour traiter une charge de renforcement dentaire comprenant les étapes suivantes :
a) on mélange en milieu solvant la charge de renforcement dentaire **(B)** et au moins un agent de couplage organosilicié **(F)** de formule 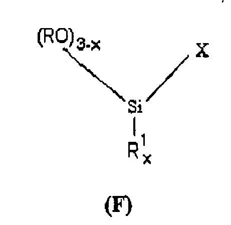 formule dans laquelle :
- R est un hydrogène ou un radical alkyle linéaire ou ramifié en C1-C4,
- R¹ est un radical alkyle linéaire ou ramifié, ou un radical phényle,
- x est égal à 0, 1 ou 2, et
- X étant défini par la formule suivante : 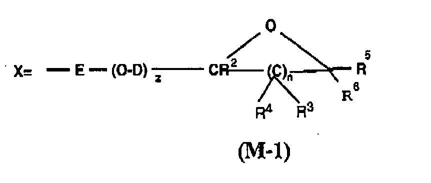 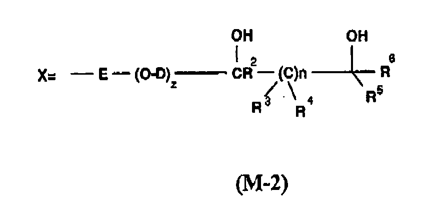 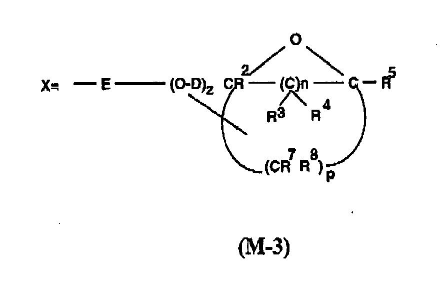 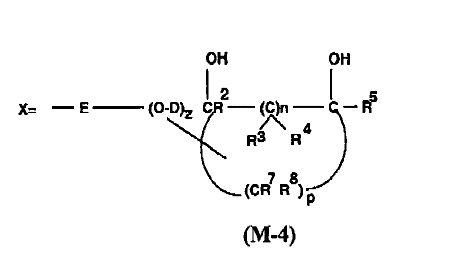 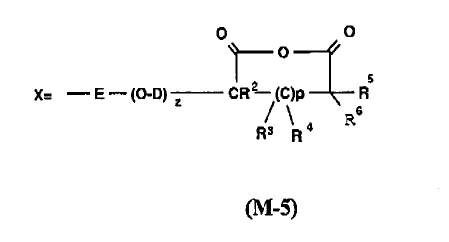 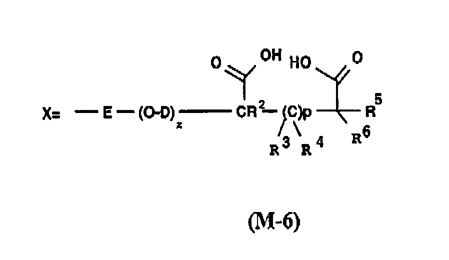 avec:
- E et D sont des radicaux identiques ou différents choisis parmi les alkyles en C1-C11 linéaires ou ramifiés,
- z est égal à 0 ou 1,
- n est égal à 0 ou 1,
- p est égal à 0, 1, 2, 3, 4, 5 ou 6.
- R², R³, R⁴, R⁵, R⁶, R⁷ et R⁸ qui sont des radicaux identiques ou différents représentant l'hydrogène ou un alkyle linéaire ou ramifié en C1-C12.
b) on évapore le solvant pour obtenir une charge dentaire intermédiaires **(B-1)**,
c) la charge dentaire intermédiaire **(B-1)** subit un traitement thermique de manière à permettre la réaction de couplage entre la charge dentaire intermédiaire **(B-1)** et l'agent de couplage **(F)** et ainsi d'obtenir une charge dentaire intermédiaire **(B-2)**,
d) la charge dentaire intermédiaire **(B-2)** est ensuite mélangée en milieu solvant avec au moins un composé **(G)** qui est un monomère, oligomère ou polymère organique ou un organosiloxane comprenant au moins une fonction oxirane, oxétane, alcényle éther et/ou carbonate,
e) on évapore le solvant pour obtenir une charge dentaire intermédiaire **(B-3)**, et
f) la charge dentaire intermédiaire **(B-3)** subit un traitement thermique de manière à permettre la réaction entre la charge dentaire intermédiaire **(B-3)** et le composé **(G)** et ainsi d'obtenir une charge dentaire traitée **(S-4)**.

31. Procédé pour traiter une charge de renforcement dentaire selon la revendication précédente dans lequel le traitement thermique des étapes c) et f) du procédé (I) s'effectue par un chauffage à une température inférieure ou égale à 200°C, de préférence inférieure ou égale à 160°C et encore plus préférentiellement comprise entre 100 et 165°C.

32. Procédé pour traiter une charge de renforcement dentaire selon l'une quelconque des revendications 30 à 31 dans lequel l'agent de couplage organosilicié **(F)** est défini selon la revendication 5.

33. Procédé pour traiter une charge de renforcement dentaire selon l'une quelconque des revendications 30 à 31 dans lequel le composé **(G)** est défini selon l'une quelconque des revendications 6 à 8.

34. Procédé pour traiter une charge de renforcement dentaire selon l'une quelconque des revendications 30 à 31 dans lequel la charge de renforcement est un verre minéral ou une silice de combustion.

35. Charge de renforcement obtenue par le procédé selon l'une quelconque des revendications 30 à 34.

36. Prothèse dentaire susceptible d'être obtenue par réticulation d'une composition selon l'une quelconque des revendications 1 à 29.

37. Matériau de restauration dentaire susceptible d'être obtenu par réticulation d'une composition selon l'une quelconque des revendications 1 à 29.

## Claims

1. Dental composition comprising:
(1) at least one cationically reactive compound (**A**);
(2) at least one dental filler (**B**);
(3) optionally at least one dispersant (**C**) comprising at least one organic polymer or copolymer;
(4) at least one cationic photoinitiator (**D**);
(5) and optionally at least one photosensitizer (**E**),
said composition being **characterized in that** it comprises at least one dental filler (**B**) treated by a process (I) comprising the following steps:
a) in a solvent medium the dental filler (**B**) is mixed with at least one organosilicon coupling agent (**F**) of formula 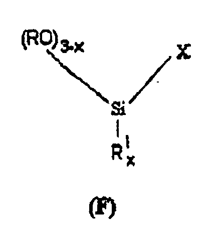 in which formula:
- R is a hydrogen or a linear or branched C1 - C4 alkyl radical,
- R¹ is a linear or branched alkyl radical or a phenyl radical,
- x is 0, 1 or 2, and
- X is defined by the following formulae: 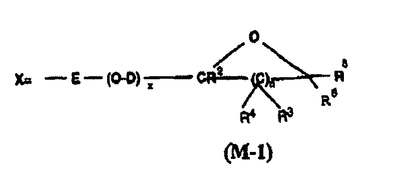 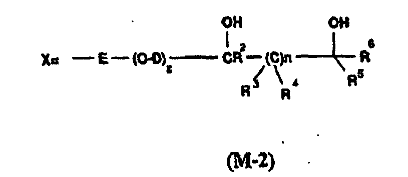 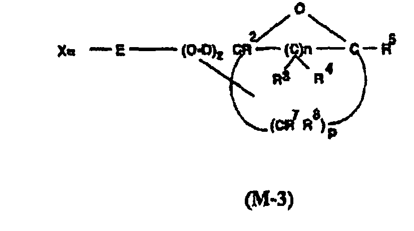 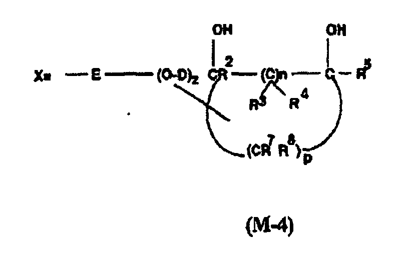 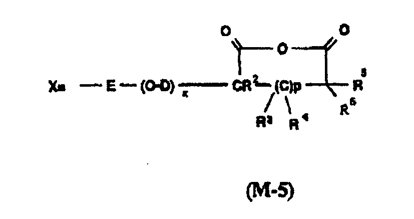 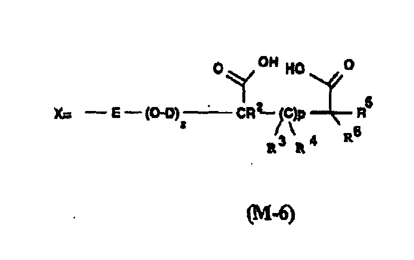
where:
- E and D are identical or different radicals selected from linear or branched C1-C12 alkyls,
- z is 0 or 1,
- n is 0 or 1,
- p is 0, 1, 2, 3, 4, 5 or 6, and
- R², R³, R⁴, R⁵, R⁶, R⁷ and R⁸, which are identical or different radicals, represent hydrogen or a linear or branched C1-C12 alkyl,
b) the solvent is evaporated to give an intermediate dental filler (**B-1**),
c) the intermediate dental filler (**B-1**) undergoes a heat treatment to allow the coupling reaction between the intermediate dental filler (**B-1**) and the coupling agent (**F**) and so to obtain an intermediate dental filler (**B-2**),
d) the intermediate dental filler (**B-2**) is then mixed in a solvent medium with at least one compound (G) which is an organic monomer, oligomer or polymer or an organosiloxane comprising at least one oxirane, oxetane, alkenyl ether and/or carbonate function,
e) the solvent is evaporated to give an intermediate dental filler (**B-3**), and
f) the intermediate dental filler (**B-3**) undergoes a heat treatment to allow the reaction between the intermediate dental filler (**B-3**) and the compound (**G**) and so to obtain a treated dental filler (**B-4**).

2. Dental composition according to Claim 1, wherein the heat treatment of steps c) and f) of process (I) takes place by heating to a temperature less than or equal to 200°C, preferably less than or equal to 165°C and more preferably between 100 and 165°C.

3. Dental composition according to any one of the preceding claims, wherein the overall proportion of dental fillers (**B**) represents up to 85% by weight, relative to the total weight of the dental composition, and preferably between 60% and 80% by weight.

4. Dental composition according to any one of the preceding claims, wherein the treatment of the dental filler (**B**) is carried out with up to 20% by weight, and preferably between 1% and 15% by weight, of the compound (**G**) and/or up to 20% by weight, preferably between 1% and 15% by weight, of the compound (**F**), relative to the total weight of the dental composition.

5. Dental composition according to any one of the preceding claims, wherein the organosilicon coupling agent (**F**) is selected from the group consisting of the following compounds: glycidyloxypropyltrimethoxysilane, the product of hydrolysis of glycidyloxypropyltrimethoxysilane; glycidyloxypropyltriethoxysilane, the product of hydrolysis in acidic medium of glycidyloxypropyltriethoxysilane; glycidyloxypropyldimethoxymethylsilane or the product of hydrolysis, beta-(3,4-epoxycyclohexyl)ethyltriethoxysilane or the product of hydrolysis, and beta-(3,4-epoxycyclohexyl)ethyltrimethoxysilane or the product of hydrolysis.

6. Dental composition according to any one of the preceding claims, wherein the compound (G) comprises at least one function selected from the group consisting of the structures (**M-7**) to (**M-12**) below: 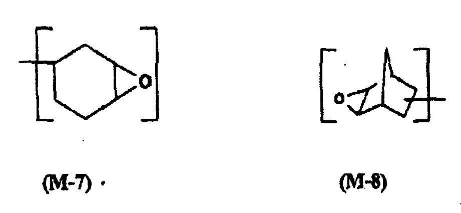 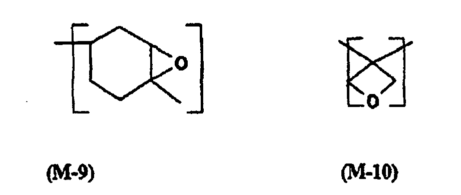
**(M-11)** -(CH₂)₃-O-CH=CH₂;
**(M-12)** -(CH₂)₃-O-CH=CH-R"
where R" represents a linear or branched C₁-C₆ alkyl radical.

7. Dental composition according to any one of the preceding claims, wherein the compound (**G**) is a silicone oligomer (**G-1**) or a silicone polymer (**G-2**).

8. Dental composition according to Claim 7, wherein the silicone oligomer (**G-1**) and the silicone polymer (**G-2**) comprise:
a) at least one unit of formula: 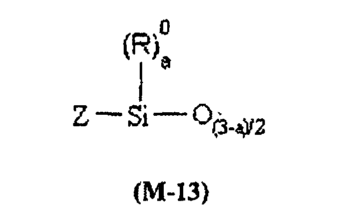 in which formula:
- a = 0, 1 or 2,
- R⁰, identical or different at each occurrence, represents an alkyl, cycloalkyl, aryl, vinyl, hydrogeno or alkoxy radical, preferably a C1-C6 lower alkyl,
- Z, identical or different at each occurrence, is an organic substituent containing at least one oxirane, alkenyl ether, oxetane and/or carbonate function, and
b) at least two silicon atoms.

9. Dental composition according to Claim 8, wherein the silicone oligomer (**G-1**) and the silicone polymer (**G-2**) are selected from the group consisting of the compounds of formulae: 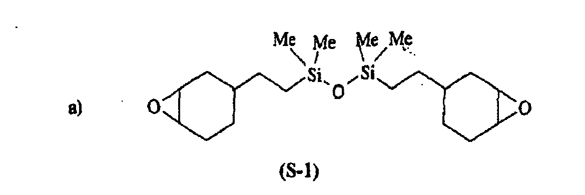 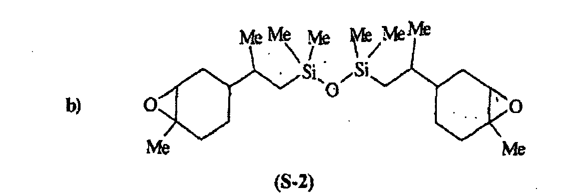 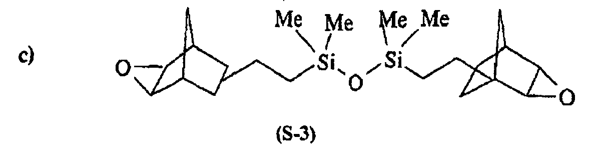 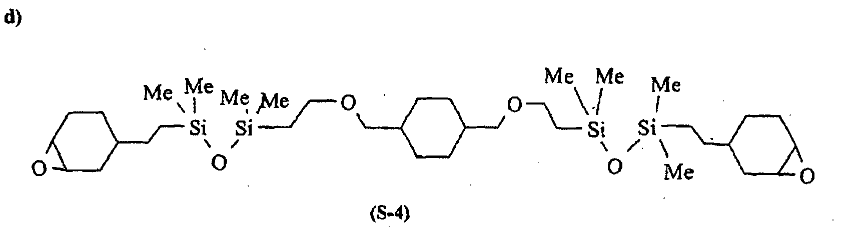 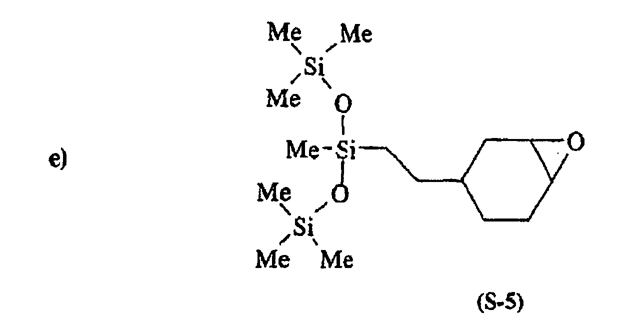 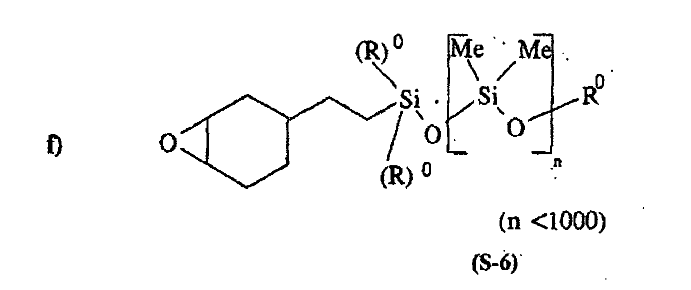 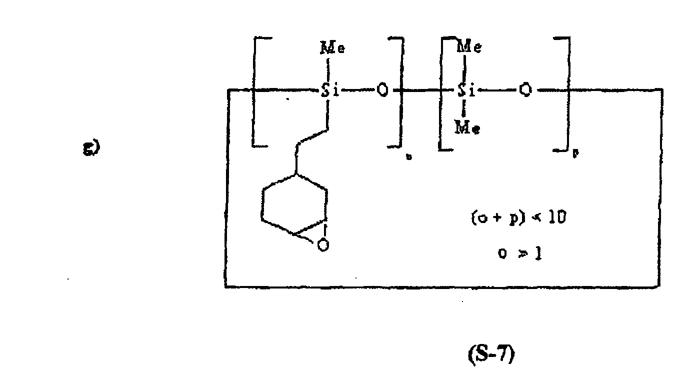 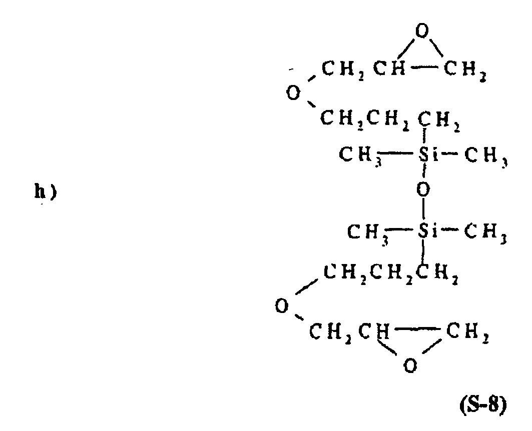 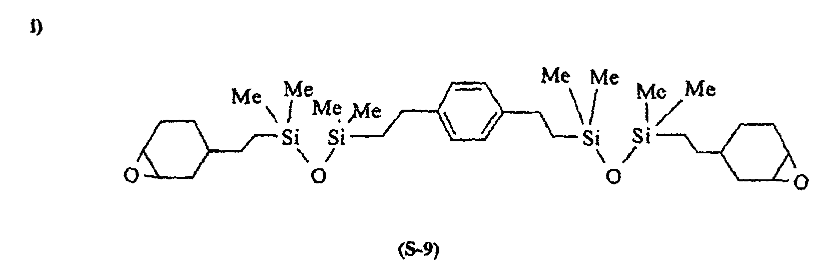 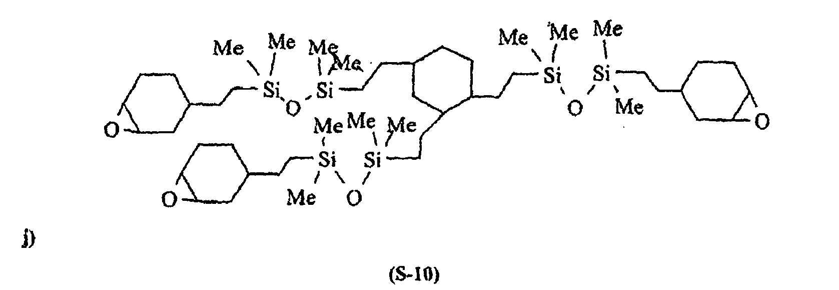 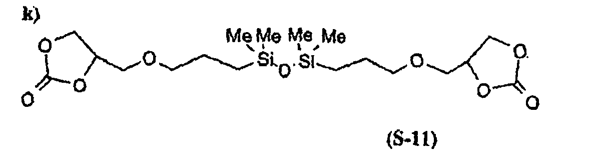 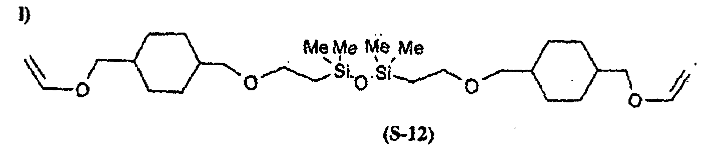 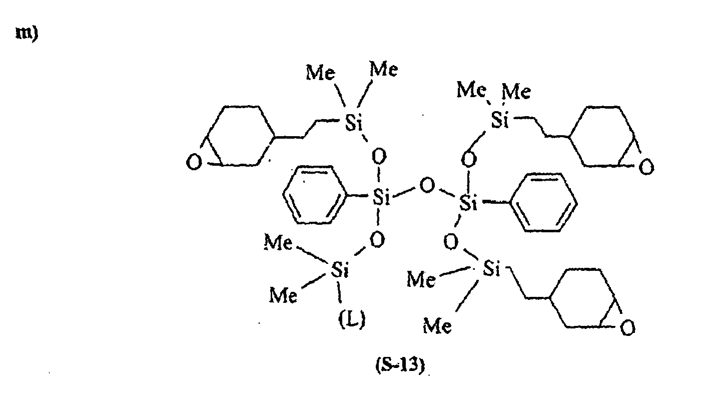 L = H; OH; Me; phenyl; C1-C12 alkyl; C1-C6 cycloalkyl or the following groups: 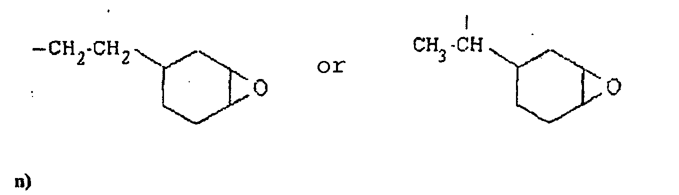 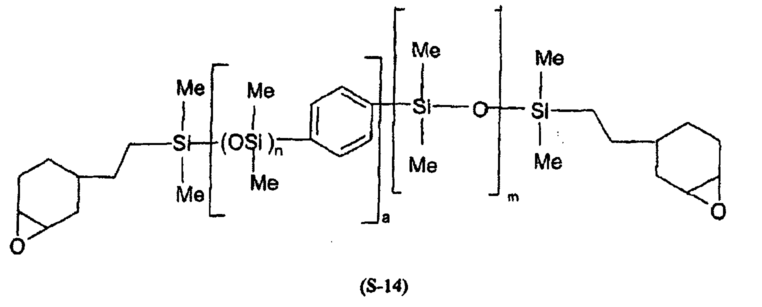 with n < 100; a < 100 and m < 100 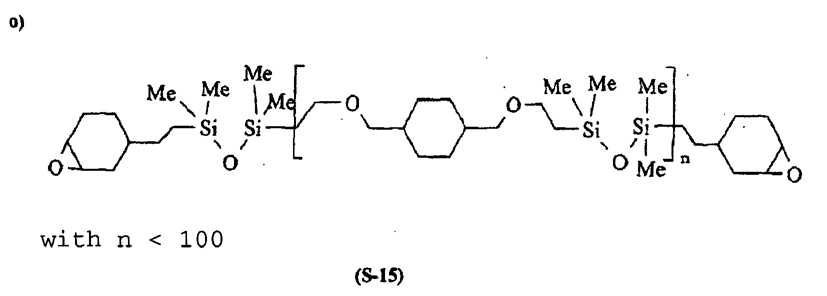 in which formulae R° or R₀, which are identical or different, represent an alkyl, cycloalkyl or aryl radical, preferably a C1-C6 lower alkyl.

10. Dental composition according to any one of the preceding claims, **characterized in that** the cationically reactive compound (**A**) is selected from the group consisting of monomers and/or (co)polymers comprising:
epoxies, vinyl ethers, oxetanes, spiroorthocarbonates, spiro-ortho esters and combinations thereof.

11. Dental composition according to Claim 10, wherein the cationically reactive compound (**A**) is composed of at least one crosslinkable and/or polymerizable silicone oligomer (**G-1**) or silicone polymer (**G-2**) which is liquid at ambient temperature or thermofusible at a temperature of less than 100ºC and comprises:
a) at least one unit of the following formula: 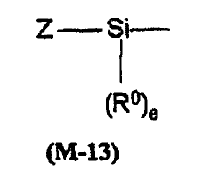 in which formula:
- a = 0, 1 or 2,
- R⁰, identical or different at each occurrence, represents an alkyl cycloalkyl, aryl, vinyl, hydrogeno or alkoxy radical, preferably a C1-C6 lower alkyl,
- Z, identical or different at each occurrence, is an organic substituent comprising at least one reactive oxirane, alkenyl ether, oxetane, dioxolane and/or carbonate function, and
b) at least two silicon atoms.

12. Dental composition according to Claim 11,
**characterized in that** the unit (**M-13**) comprises groups Z selected from the group consisting of the following radicals (**R-1**) to (**R-9**): 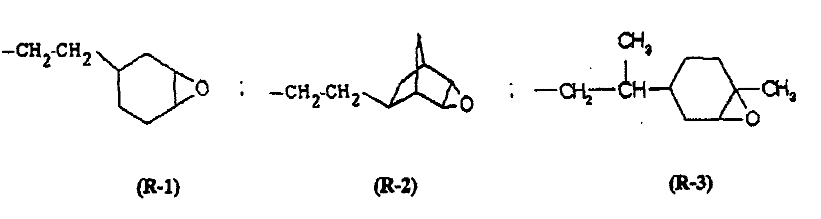 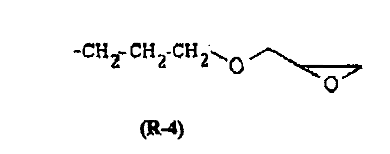 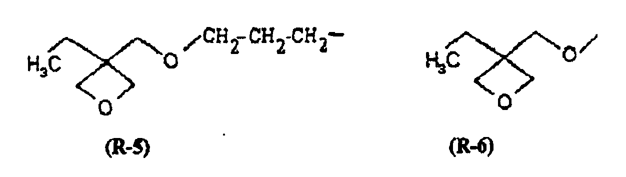 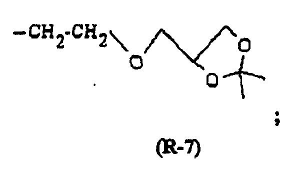
**(R-8)** -(CH₂)₃-O-CH=CH₂;
**(R-9)** -(CH₂)₃-O-CH=CH-R"
- where R" represents a linear or branched C₁-C₆ alkyl radical.

13. Dental composition according to any one of the preceding claims, **characterized in that** the cationically reactive compound (**A**) is selected from the group consisting of the compounds (**S-1**) to (**S-15**) as defined according to Claim 9.

14. Dental composition according to any one of the preceding claims, **characterized in that** the cationic photoinitiator (**D**) is of borate type and is selected from those of a formula in which:
a) the cationic entity of the borate is selected from:
(1) onium salts of formula:
**(I)** **[(R⁹)ₙ-A-(R¹⁰)ₘ]⁺**
in which formula:
- A represents an element from groups 15 to 17 such as, for example: I, S, Se, P or N,
- R⁹ represents a C6-C20 heterocyclic or carbocyclic aryl radical, it being possible for said heterocyclic radical to contain nitrogen or sulphur as heteroelements,
- R¹⁰ represents R⁹ or a C1-C30 linear or branched alkyl or alkenyl radical, said radicals R⁹ and R¹⁰ being optionally substituted by a C1-C25 alkoxy, C1-C25 alkyl, nitro, chloro, bromo, cyano, carboxyl, ester or mercapto group,
- m and n are integers, with n+m = v+1, v being the valency of the element A,
(2) oxoisothiochromanium salts; and
(3) organometallic salts of the following formula:
(L₁L₂L₃M)^{+q} **(II)**
in which formula:
- M represents a metal from group 4 to 10, especially iron, manganese, chromium or cobalt,
- L1 represents one ligand attached to the metal M by n electrons, the ligand being selected from η³-alkyl, η⁵-cyclopentadienyl and η⁷-cycloheptatrienyl ligands and η⁶-aromatic compounds selected from optionally substituted η⁶-benzene ligands and compounds having 2 to 4 fused rings, each ring being capable of contributing to the valency shell of the metal M by 3 to 8 n electrons,
- L2 represents one ligand attached to the metal M by n electrons, the ligand being selected from η⁷-cycloheptatrienyl ligands and η⁶-aromatic compounds selected from optionally substituted η⁶-benzene ligands and compounds having 2 to 4 fused rings, each ring being capable of contributing to the valency shell of the metal M by 6 or 7 n electrons,
- L3 represents from 0 to 3 identical or different ligands attached to the metal M by σ electrons and selected from CO and NO₂⁺, the total electronic charge q of the complex to which L1, L2 and L3 contribute and the ionic charge of the metal M being positive and equal to 1 or 2; and
b) the anionic borate entity has the formula:
(**III**) **[BXₐ R_{b}]⁻**
in which formula:
- a and b are integers ranging from 0 to 3 for a and from 1 to 4 for b, with a+b = 4,
- the symbols X represent:
* a halogen atom (chlorine, fluorine) with a = 0 to 3, or
* an OH function with a = 0 to 2,
- the symbols R are identical or different and represent:
- a phenyl radical substituted by at least one electron-withdrawing group such as, for example, OCF₃, CF₃, NO₂ or CN and/or by at least two halogen atoms (especially fluorine), when the cationic entity is an onium of an element from groups 15 to 17,
- a phenyl radical substituted by at least one electron-withdrawing element or group, particularly a halogen atom (especially fluorine), CF₃, OCF₃, NO₂ or CN, when the cationic entity is an organometallic complex of an element from groups 4 to 10, or
- an aryl radical containing at least two aromatic nuclei such as, for example, biphenyl, naphthyl, optionally substituted by at least one electron-withdrawing element or group, particularly a halogen atom, especially fluorine, or by OCF₃, CF₃, NO₂ or CN, irrespective of the cationic entity.

15. Composition according to Claim 14, **characterized in that** the cationic photoinitiator (D) is selected from the group consisting of the following compounds:
**(P-16)**: [(C₈H₁₇)-O-C₆H₄-I-C₆H₅)]⁺, [B(C₆F₅)₄]⁻;
**(P-17)**: [C₁₂H₂₅-C₆H₄-I-C₆H₅]⁺, [B(C₆F₅)₄]⁻;
**(P-18)**: [(C₈H₁₇-O-C₆H₄)₂I]⁺, [B(C₆F₅)₄]⁻;
**(P-19)**: [(C₈H₁₇)-O-C₆H₄-I-C₆H₅)]⁺, [B(C₆F₅)₄]⁻;
**(P-20)**: [(C₆H₅)₂S-C₆H₄-O-C₈H₁₇]⁺, [B(C₆H₄CF₃)₄]⁻;
**(P-21)**: [(C₁₂H₂₅-C₆H₄)₂I]⁺, [B(C₆F₅)₄]⁻;
**(P-22)**: [CH₃-C₆H₄-I-C₆H₄-CH(CH₃)₂]⁺, [B(C₆F₅)₄]⁻;
**(P-23)**: (η5-cyclopentadienyl) (η6-toluene)Fe⁺, [B(C₆F₅)₄]⁻;
**(P-24)**: (η5-cyclopentadienyl)(η6-1-methyl-naphthalene)Fe⁺, [B(C₆F₅)₄]⁻;
**(P-25)**: (η5-cyclopentadienyl)(η6-cumene)Fe⁺, [B(C₆F₅)₄]⁻;
**(P-26)**: [(C₁₂H₂₅-C₆H₄)₂I]⁺, [B(C₆H₃(CF₃)₂]⁻;
**(P-27)**: [CH₃-C₆H₄-I-C₆H₄-CH₂CH(CH₃)₂]⁺, [B(C₆F₅)4]⁻;
**(P-28)**: [CH₃-C₆H₄-I-C₆H₄-CH₂CH(CH₃)₂]⁺, [B(C₆H₃(CF₃)₂)₄]⁻;
and
**(P-29)**: [CH₃-C₆H₄-I-C₆H₄-CH(CH₃)₂]⁺, [B(C₆H₃(CF₃)₂)₄]⁻.

16. Dental composition according to Claim 1, **characterized in that** the cationic photoinitiator (**D**) is an iodonium salt.

17. Dental composition according to any one of the preceding claims, wherein the photosensitizer (**E**) comprises in its structure one or more substituted or unsubstituted aromatic nuclei, having a residual absorption for light of between 200 and 500 nm.

18. Dental composition according to any one of the preceding claims, wherein the photosensitizer (**E**) is selected from the group consisting of the compounds from the class of the anthracenes, thioxanthones, camphorquinones and phenanthrenequinone and also mixtures thereof.

19. Dental composition according to Claim 18, wherein the photosensitizer (**E**) is a salt of a thioxanthone substituted by at least one group comprising an ammonium function.

20. Dental composition according to Claim 19,
**characterized in that** the anion associated with the salt of the thioxanthone substituted by at least one group comprising an ammonium function is selected from the following anions: a halide, BF₄⁻, PF₆⁻; SbF₆⁻; the anion (**III**) of formula [BXₐR_{b}]⁻ defined according to Claim 18, R_{f}SO₃⁻; (R_{f}SO₂)₃C⁻ or (R_{f}SO₂)₂N⁻, with R_{f} being a linear or branched alkyl radical substituted by at least one halogen atom, preferably a fluorine atom.

21. Dental composition according to any one of the preceding claims, wherein the photosensitizer (**E**), optionally in combination with at least one camphorquinone, phenanthrenequinone and/or substituted anthracene, has the following formula: 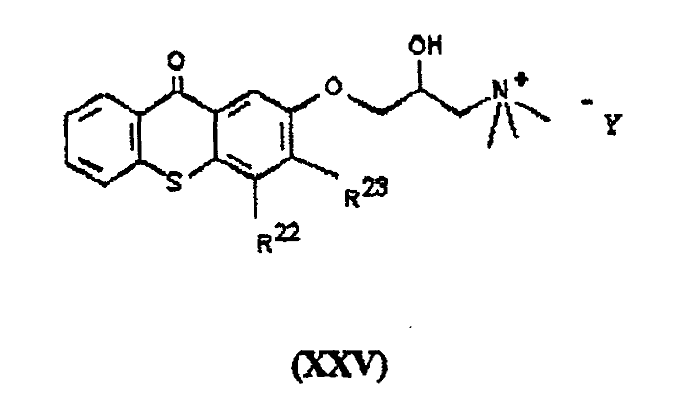 in which formula:
- R²² and R²³ are identical or different and represent a hydrogen or an optionally substituted C1-C10 alkyl radical, preferably R²²=R²³=methyl,
- (X⁻) is an anionic entity, preferably a halide; BF₄⁻, PF₆⁻; SbF₆⁻; the anion (**III**) of formula [BXₐR_{b}]⁻ defined according to Claim 16, R_{f}SO₃⁻; (R_{f}SO₂)₃C⁻ or (R_{f}SO₂)₂N⁻, with R_{f} being a linear or branched alkyl radical substituted by at least one halogen atom, preferably a fluorine atom,
and more preferably (X⁻) is selected from the borates of following formulae: [B(C₆H₃(CF₃)₂)₄]⁻ and [B(C₆F₅)₄]⁻.

22. Dental composition according to any one of the preceding claims, wherein the photosensitizer (**E**), optionally in combination with at least one camphorquinone, phenanthrenequinone and/or substituted anthracene, has the formula:

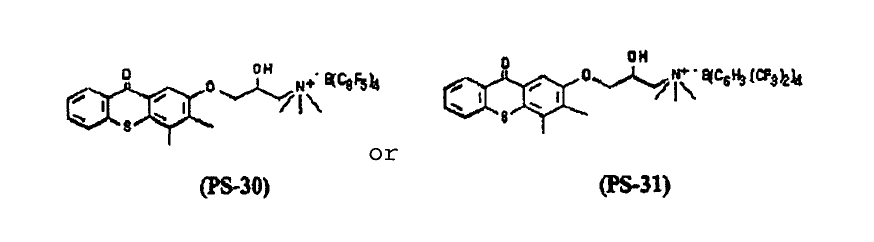

23. Dental composition according to any one of the preceding claims, wherein the photosensitizer (E) is selected from the group consisting of the following compounds:
- **(PS-30)**: 3-(3,4-dimethyl-9-oxo-9-thioxanthenen-2-yloxy)-2-hydroxypropyl)trimethylammonium chloride;
- (**PS-31**): 3-(3,4-dimethyl-9-oxo-9-thioxanthenen-2-yloxy)-2-hydroxypropyl)trimethylammonium tetrakis(pentafluorophenyl)borate;
- (**PS-32**): 3-(3,4-dimethyl-9-oxo-9-thioxanthenen-2-yloxy)-2-hydroxypropyl)trimethylammonium tetrakis(bis(trifluoromethyl)phenyl)borate;
- (**PS-33**): phenanthrenequinone;
- (**PS-34**): camphorquinone;
- (**PS-35**): acenaphthenequinone;
- (**PS-36**): dibenzoyl peroxide;
- (**PS-37**): 2-ethyl-9,10-dimethoxyanthracene;
- (**PS-38**): 9,10-diethoxyanthracene;
- (**PS-39**): 9,10-dibutoxyanthracene;
- (**PS-40**): 9-hydroxymethylanthracene;
- (**PS-41**): 2-dimethylaminothioxanthone;
- (**PS-42**): 3-ethylcarboxy-7-methoxythioxanthone;
- (**PS-43**): 1-phenyl-thio-4-propoxythioxanthone;
- (**PS-44**): 2-methoxythioxanthone;
- (**PS-45**): 2-(N,N-diethylaminopropoxy)thioxanthone;
- (**PS-46**): 2-isopropylthioxanthone;
- (**PS-47**): 1-chloro-4-propoxythioxanthone;
- (**PS-48**): 4-isopropylthioxanthone; 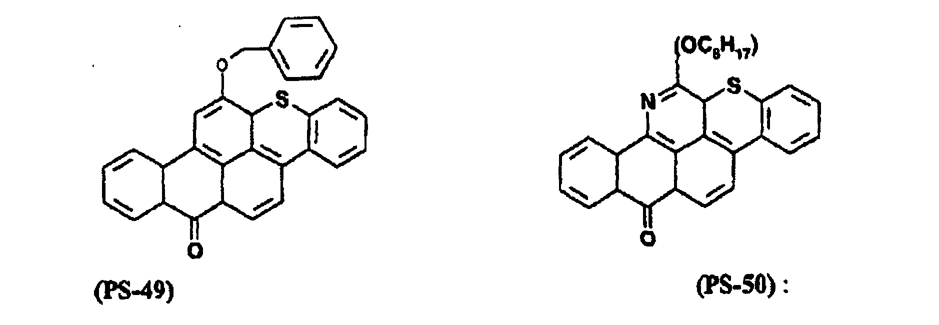
- and mixtures thereof.

24. Dental composition according to any one of the preceding claims, wherein the photosensitizer (**E**) is selected from the group consisting of the compounds from the class of the coumarins, diketones, fluorones, aminoketones, para-aminostyryl ketones and mixtures thereof.

25. Dental composition according to any one of the preceding claims, wherein the silicone polymer (**G-2**) or the silicone oligomer (**G-1**) is combined with an organic epoxy resin or oxetane of formula (**S-103**) or (**S-104**): 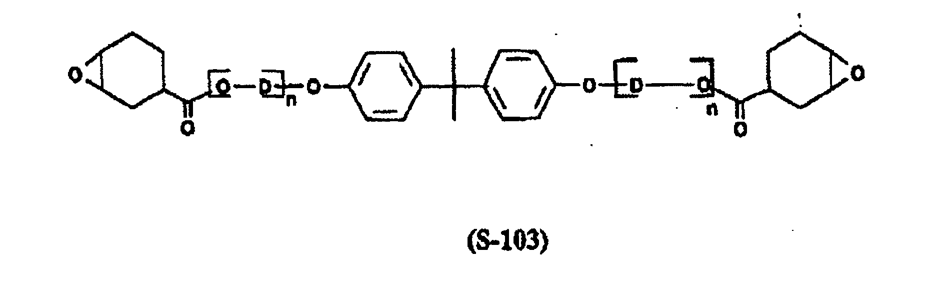 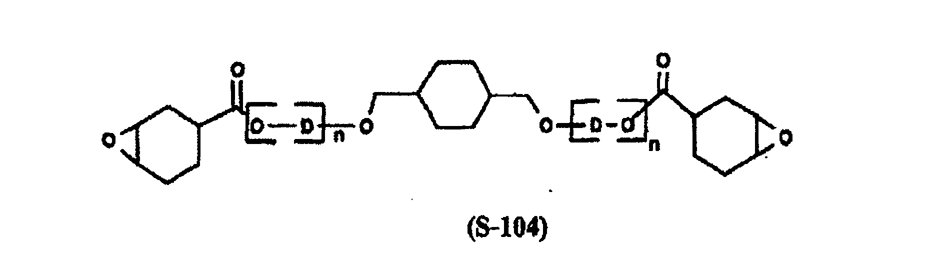 where:
- 0 ≤ n ≤ 100 and
- D = radical selected from linear or branched C1-C12 alkyls.

26. Dental composition according to one of the preceding claims, wherein the dental filler (**B**) is an inorganic glass or a fumed silica.

27. Dental composition according to any one of the preceding claims, wherein the compound (**G**) is a silane (**G-3**) of formula: 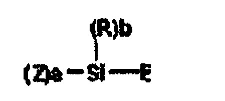 in which formula: 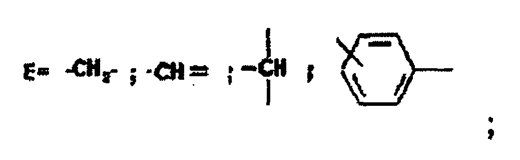
- R, identical or different at each occurrence, represents an alkyl, cycloalkyl, aryl, vinyl, hydrogeno or alkoxy radical, preferably a C1-C6 lower alkyl,
- Z, identical or different at each occurrence, is an organic substituent comprising at least one oxirane, alkenyl ether, oxetane and/or carbonate function, and
- a+b=3.

28. Dental composition according to Claim 27, wherein the silane (**G-3**) is selected from the group consisting of the following molecules:

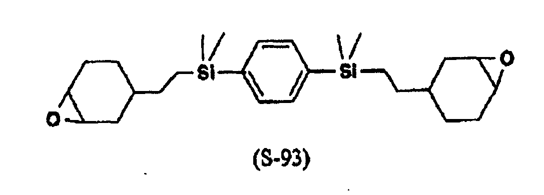

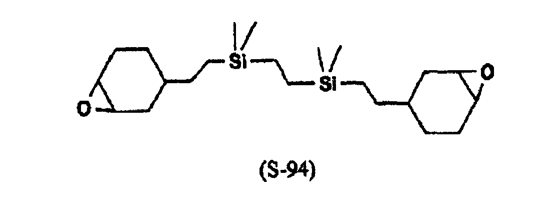

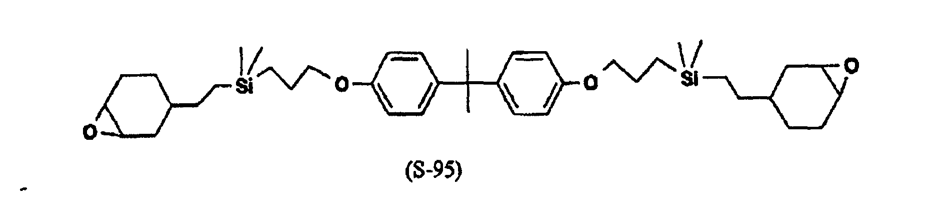

29. Dental composition according to one of the preceding claims, wherein the compound (G) is an organic compound (**G-4**) selected from the group consisting of the molecules (**S-96**) to (**S-104**): 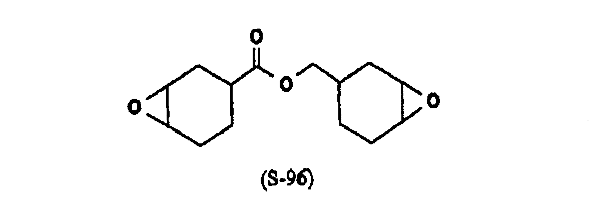 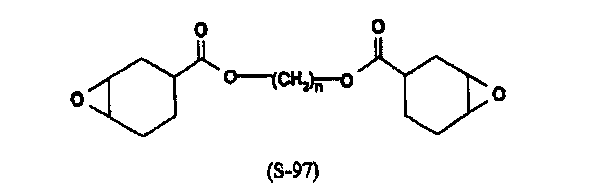 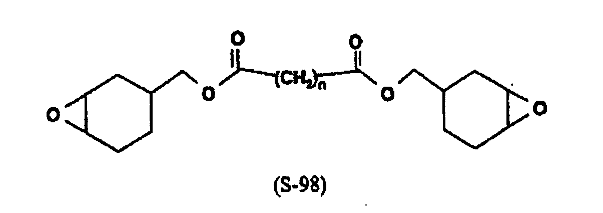 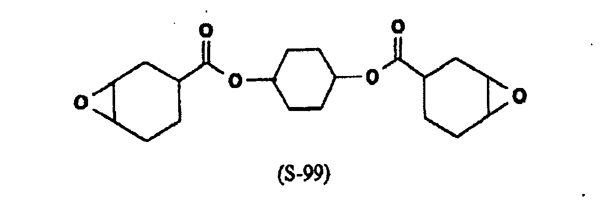 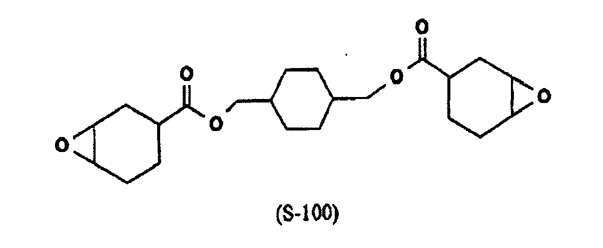 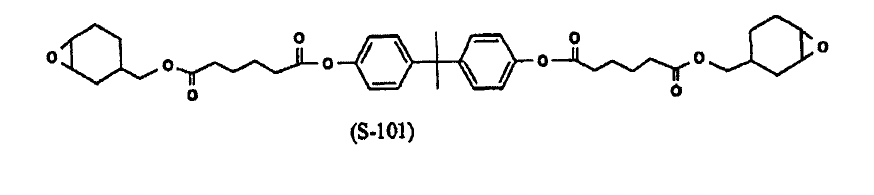 in which formulae: n is an integer between 1 and 10 (inclusive); 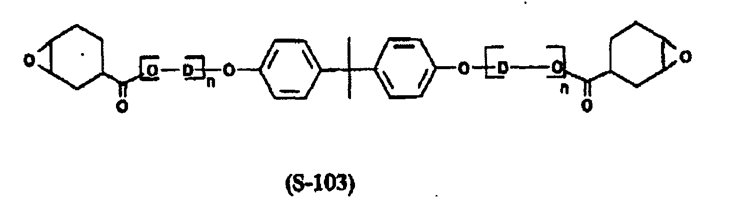 with n < 100 and D = linear or branched C1-C12 alkyls, 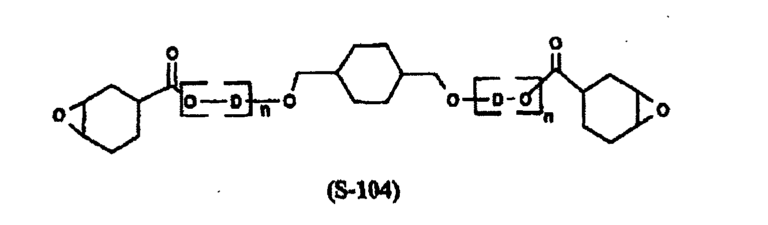 with n < 100 and the group D = linear or branched C1-C12 alkyls.

30. Process for treating a dental reinforcing filler, comprising the following steps:
a) in a solvent medium the dental reinforcing filler (**B**) is mixed with at least one organosilicon coupling agent (**F**) of formula 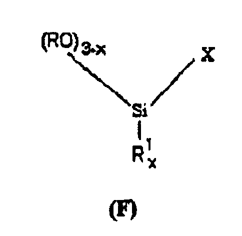 in which formula:
- R is a hydrogen or a linear or branched C1 - C4 alkyl radical,
- R¹ is a linear or branched alkyl radical or a phenyl radical,
- x is 0, 1 or 2, and
- X is defined by the following formulae: 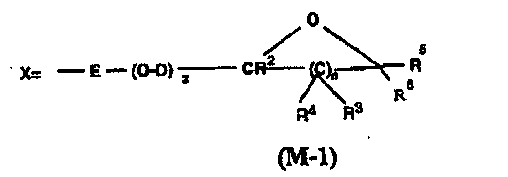 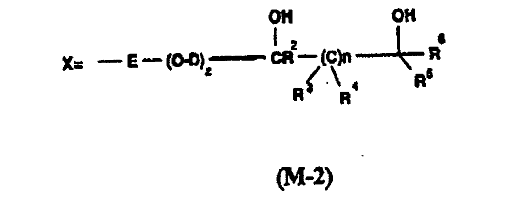 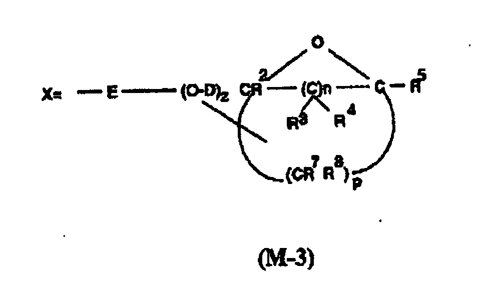 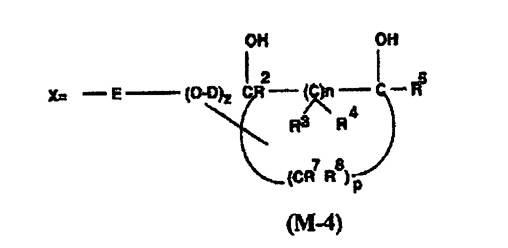 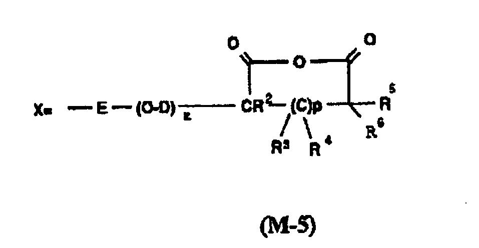 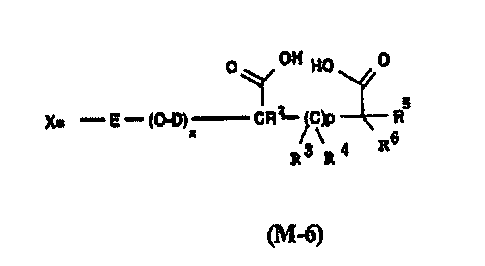
where:
- E and D are identical or different radicals selected from linear or branched C1-C12 alkyls,
- z is 0 or 1,
- n is 0 or 1,
- p is 0, 1, 2, 3, 4, 5 or 6, and
- R², R³, R⁴, R⁵, R⁶, R⁷ and R⁸, which are identical or different radicals, represent hydrogen or a linear or branched C1-C12 alkyl,
b) the solvent is evaporated to give an intermediate dental filler (**B-1**),
c) the intermediate dental filler (**B-1**) undergoes a heat treatment to allow the coupling reaction between the intermediate dental filler (**B-1**) and the coupling agent (**F**) and so to obtain an intermediate dental filler (**B-2**),
d) the intermediate dental filler (**B-2**) is then mixed in a solvent medium with at least one compound (**G**) which is an organic monomer, oligomer or polymer or an organosiloxane comprising at least one oxirane, oxetane, alkenyl ether and/or carbonate function,
e) the solvent is evaporated to give an intermediate dental filler (**B-3**), and
f) the intermediate dental filler (**B-3**) undergoes a heat treatment to allow the reaction between the intermediate dental filler (**B-3**) and the compound (**G**) and so to obtain a treated dental filler (**B-4**).

31. Process for treating a dental reinforcing filler according to the preceding claim, wherein the heat treatment of steps c) and f) of process (I) takes place by heating to a temperature less than or equal to 200°C, preferably less than or equal to 160°C and more preferably between 100 and 165°C.

32. Process for treating a dental reinforcing filler according to either of Claims 30 and 31, wherein the organosilicon coupling agent (**F**) is defined according to Claim 5.

33. Process for treating a dental reinforcing filler according to either of Claims 30 and 31, wherein the compound (**G**) is defined according to any one of Claims 6 to 8.

34. Process for treating a dental reinforcing filler according to either of Claims 30 and 31, wherein the reinforcing filler is an inorganic glass or a fumed silica.

35. Reinforcing filler obtained by the process according to any one of Claims 30 to 34.

36. Dental prosthesis obtainable by crosslinking a composition according to any one of Claims 1 to 29.

37. Dental restoration material obtainable by crosslinking a composition according to any one of Claims 1 to 29.

## Patentansprüche

1. Dentalzusammensetzung, umfassend:
(1) mindestens eine kationisch reaktive Verbindung (A);
(2) mindestens einen Dentalfüllstoff (B);
(3) gegebenenfalls mindestens ein Dispersionsmittel (C), das mindestens ein organisches Polymer oder Copolymer umfasst;
(4) mindestens einen kationischen Photostarter (D);
(5) und gegebenenfalls mindestens einen Photosensibilisator (E),
wobei die Zusammensetzung **dadurch gekennzeichnet ist, dass** mindestens ein Dentalfüllstoff (B) mit einem Verfahren (I) behandelt wird, das die folgenden Schritte umfasst:
a) Mischen in Lösemittelmedium des Dentalfüllstoffs (B) und mindestens eines siliciumorganischen Kopplungsmittels (F)
mit der Formel 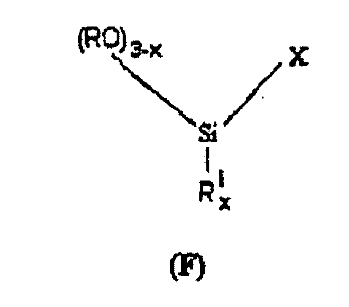 wobei in der Formel:
- R ein Wasserstoff oder ein linearer oder verzweigter C1-C4-Alkylrest ist,
- R¹ ein linearer oder verzweigter Alkylrest oder ein Phenylrest ist,
- x gleich 0, 1 oder 2 ist, und
- X durch die folgenden Formeln definiert ist: 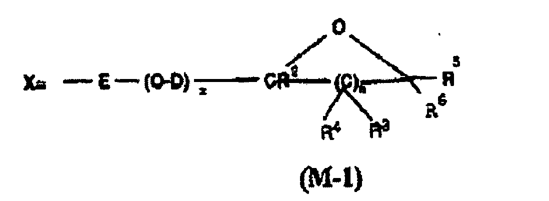 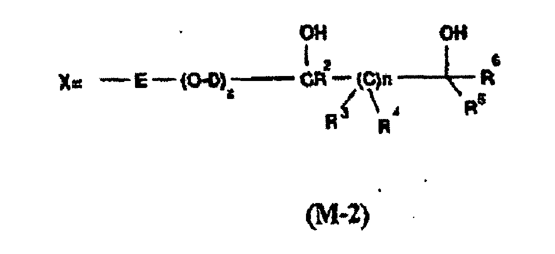 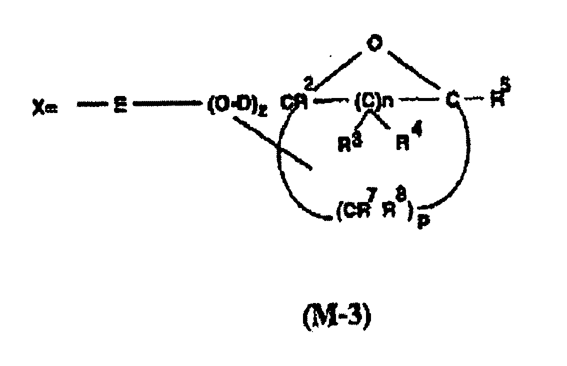 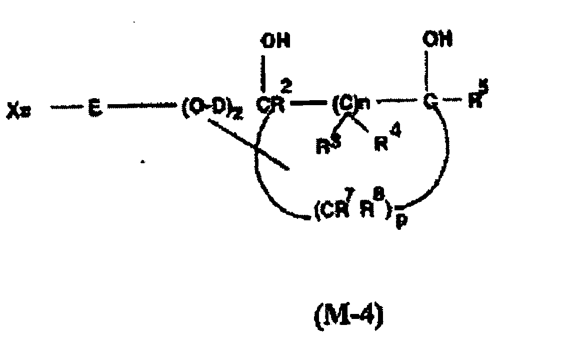 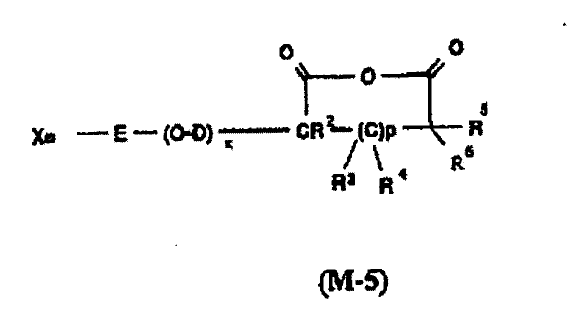 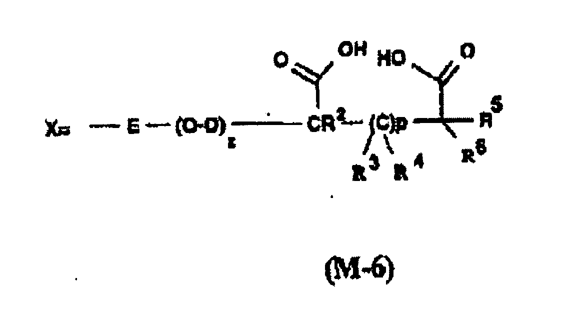
worin:
- E und D gleiche oder verschiedene Reste, ausgewählt aus den linearen oder verzweigten C1-C12-Alkylen, sind
- z gleich 0 oder 1 ist,
- n gleich 0 oder 1 ist,
- p gleich 0, 1, 2, 3, 4, 5 oder 6 ist.
- R², R³, R⁴, R⁵, R⁶, R⁷ und R⁸ gleiche oder verschiedene Reste sind, die für Wasserstoff oder ein lineares oder verzweigtes C1-C12-Alkyl stehen.
b) Verdampfen des Lösemittels, um einen intermediären Dentalfüllstoff (B-1) zu erhalten,
c) der intermediäre Dentalfüllstoff (B-1) wird einer Wärmebehandlung unterzogen, um die Kopplungsreaktion zwischen dem intermediären Dentalfüllstoff (B-1) und dem Kopplungsmittel (F) zu ermöglichen und so einen intermediären Dentalfüllstoff (B-2) zu erhalten,
d) der intermediäre Dentalfüllstoff (B-2) wird anschließend in Lösemittelmedium mit mindestens einer Verbindung (G) gemischt, die ein organisches Monomer, Oligomer oder Polymer oder ein Organosiloxan ist, das mindestens eine Oxiran-, Oxetan-, Alkenylether- und/oder Carbonatfunktion umfasst,
e) Verdampfen des Lösemittels, um einen intermediären Dentalfüllstoff (B-3) zu erhalten, und
f) der intermediäre Dentalfüllstoff (B-3) wird einer Wärmebehandlung unterzogen, um die Reaktion zwischen dem intermediären Dentalfüllstoff (B-3) und der Verbindung (G) zu ermöglichen, und so einen behandelten Dentalfüllstoff (B-4) zu erhalten.

2. Dentalzusammensetzung nach Anspruch 1, wobei die Wärmebehandlung der Schritte c) und f) des Verfahrens (I) durch eine Erwärmung auf eine Temperatur kleiner oder gleich 200 °C, bevorzugt kleiner oder gleich 165 °C und noch stärker bevorzugt im Bereich zwischen 100 und 165 °C durchgeführt wird.

3. Dentalzusammensetzung nach irgendeinem der vorhergehenden Ansprüche, wobei der Gesamtanteil an Dentalfüllstoffen (B) bis zu 85 Gew.-% und bevorzugt zwischen 60 und 80 Gew.-%, bezogen auf das Gesamtgewicht der Dentalzusammensetzung darstellt.

4. Dentalzusammensetzung nach irgendeinem der vorhergehenden Ansprüche, wobei die Behandlung des Dentalfüllstoffs (B) mit bis zu 20 Gew.-% und bevorzugt zwischen 1 und 15 Gew.-% der Verbindung (G) und/oder bis zu 20 Gew.-%, bevorzugt zwischen 1 und 15 Gew.-% der Verbindung (F), bezogen auf das Gesamtgewicht der Dentalzusammensetzung, ausgeführt wird.

5. Dentalzusammensetzung nach irgendeinem der vorhergehenden Ansprüche, wobei das siliciumorganische Kopplungsmittel (F) ausgewählt ist aus der Gruppe, bestehend aus den folgenden Verbindungen: Glycidyloxypropyltrimethoxysilan, dem Hydrolyseprodukt von Glycidyloxypropyltrimethoxysilan; Glycidyloxypropyltriethoxysilan, dem Hydrolyseprodukt von Glycidyloxypropyltriethoxysilan in saurem Medium; Glycidyloxypropyldimethoxymethylsilan oder dem Hydrolyseprodukt, Beta-(3,4-epoxycyclohexyl)ethyltriethoxysilan oder dem Hydrolyseprodukt, Beta-(3,4-epoxycyclohexyl)ethyltrimethoxysilan oder dem Hydrolyseprodukt.

6. Dentalzusammensetzung nach irgendeinem der vorhergehenden Ansprüche, wobei die Verbindung (G) mindestens eine Funktion umfasst, ausgewählt aus der Gruppe bestehend aus den folgenden Strukturen (M-7) bis (M-12): 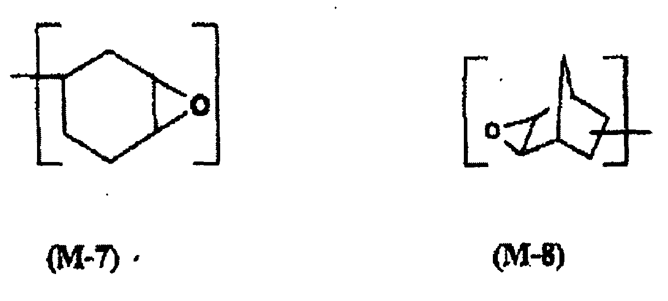 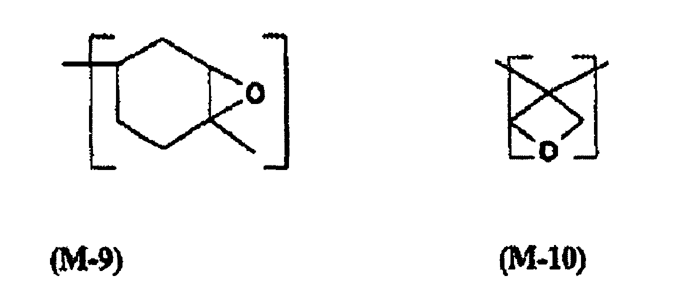
**(M-11)** -(CH₂)₃-O-CH=CH₂;
**(M-12)** -(CH₂)₃-O-CH=CH-R"
worin R" für einen linearen oder verzweigten C₁-C₆-Alkylrest steht,

7. Dentalzusammensetzung nach irgendeinem der vorhergehenden Ansprüche, wobei die Verbindung (G) ein Silikonoligomer (G-1) oder ein Silikonpolymer (G-2) ist.

8. Dentalzusammensetzung nach Anspruch 7, wobei das Silikonoligomer (G-1) und das Silikonpolymer (G-2) Folgendes umfassen:
a) mindestens eine Struktur der Formel: 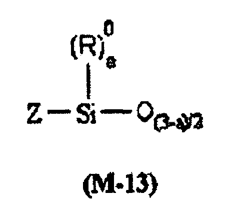 wobei in der Formel:
- a = 0, 1 oder 2,
- - R⁰, gleich oder verschieden, für einen Alkyl-, Cycloalkyl-, Aryl-, Vinyl-, Wasserstoff-, Alkoxyrest, bevorzugt für einen niedrigen C1-C6-Alkylrest steht,
- Z, gleich oder verschieden, ein organischer Substituent ist, der mindestens eine Oxiran-, Alkenylether-, Oxetan- und/oder Carbonatfunktion umfasst, und
b) mindestens zwei Siliciumatome.

9. Dentalzusammensetzung nach Anspruch 8, wobei das Silikonoligomer (G-1) und das Silikonpolymer (G-2) ausgewählt sind aus der Gruppe, bestehend aus den Verbindungen mit den folgenden Formeln: 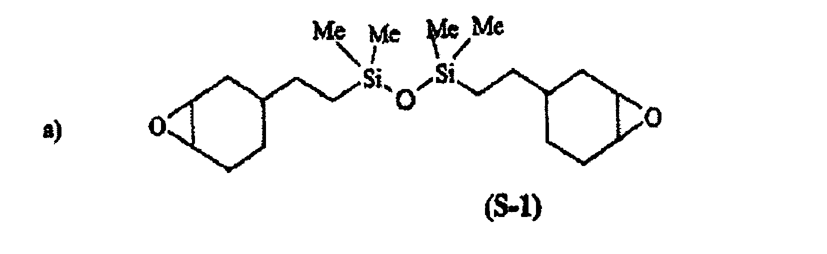 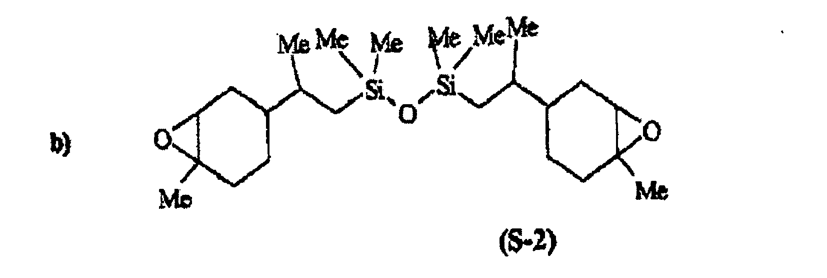 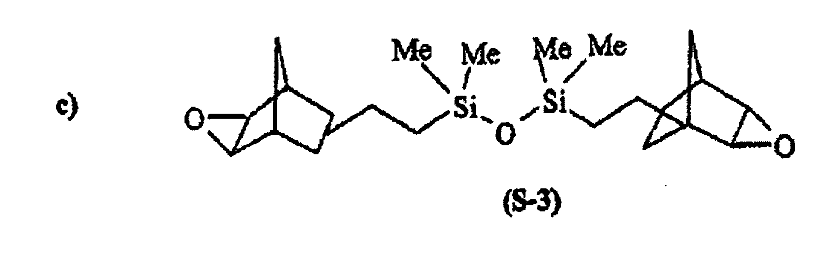 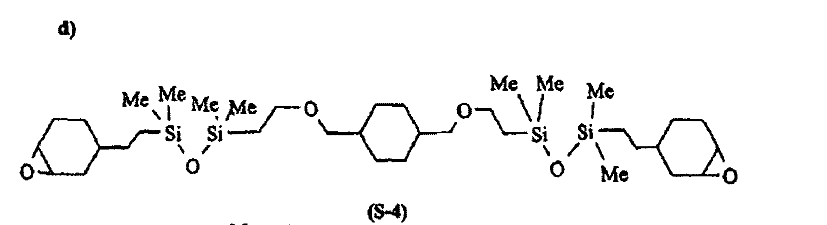 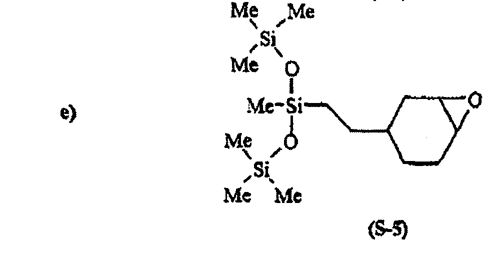 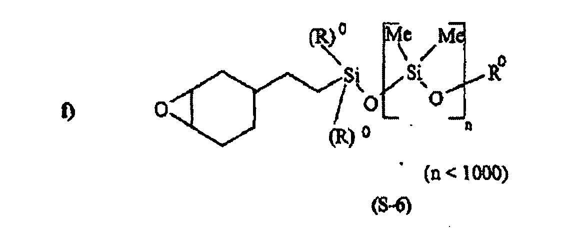 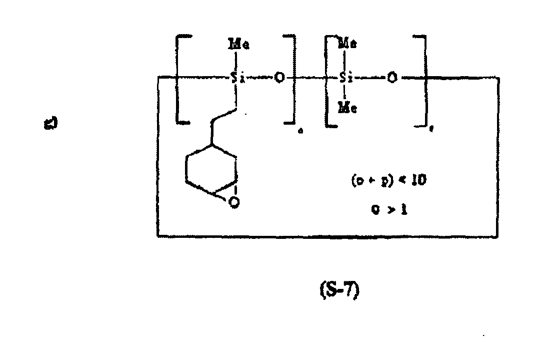 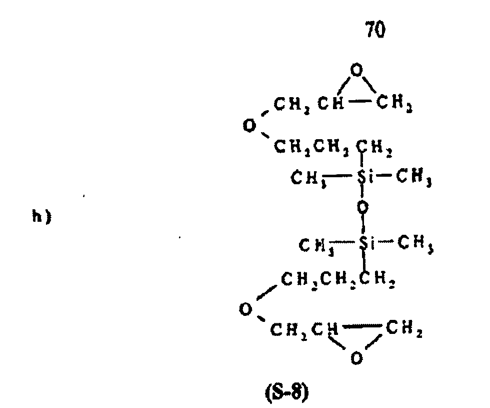 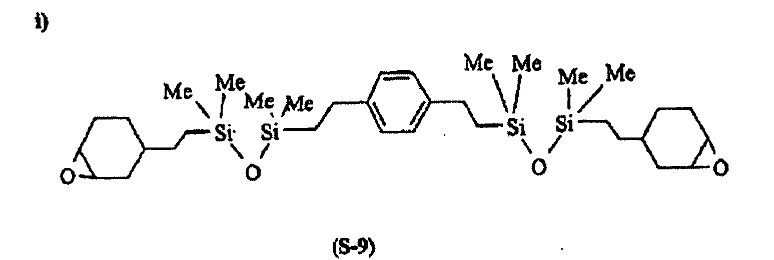 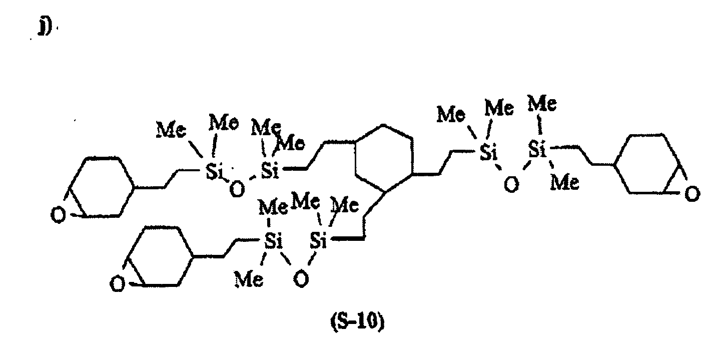 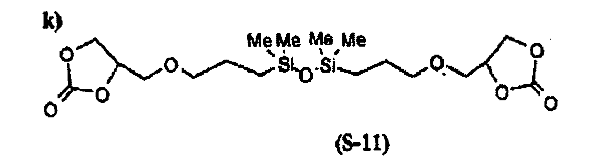 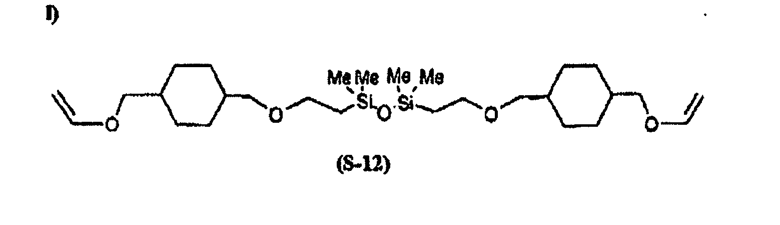 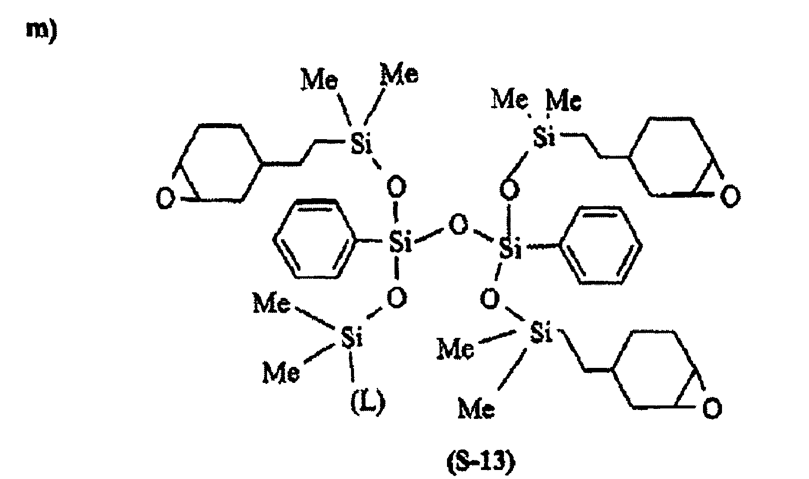 L = H; OH; Me; Phenyl; C₁-C₁₂-Alkyl; C₁-C₆-Cycloalkyl oder den folgenden Gruppen: 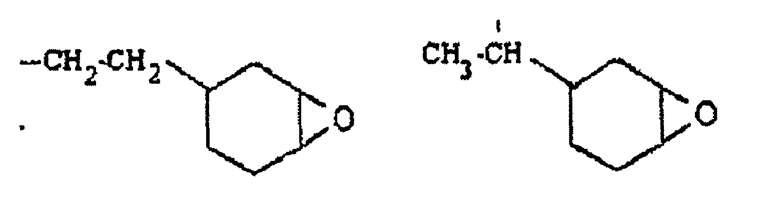 oder 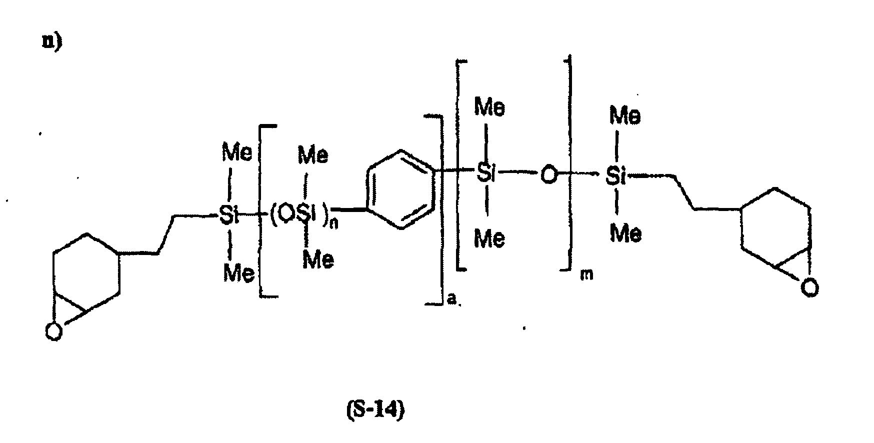 worin n<100; a<100 und m<100 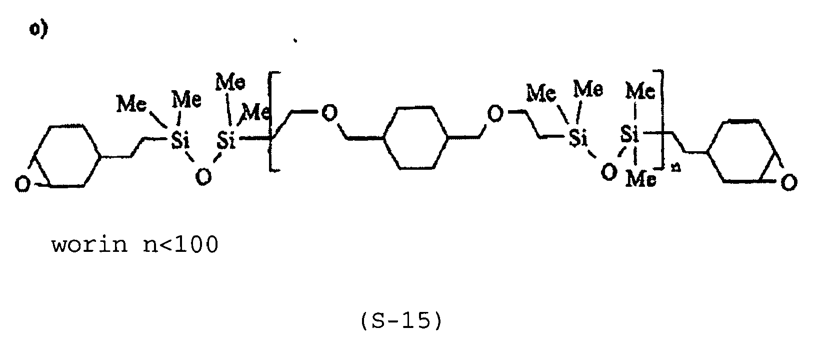 wobei in den Formeln R⁰ oder R₀, gleich oder verschieden, für einen Alkyl-, Cycloalkyl-, Arylrest, bevorzugt einen niedrigen C1-C6-Alkylrest steht.

10. Dentalzusammensetzung nach irgendeinem der vorhergehenden Ansprüche, **dadurch gekennzeichnet, dass** die kationisch reaktive Verbindung (A) ausgewählt ist aus der Gruppe der Monomere und/oder (Co)polymere, umfassend:
Epoxide, Vinylether, Oxetane, Spiroorthocarbonate, Spiroorthoester und deren Assoziationen.

11. Dentalzusammensetzung nach Anspruch 10, wobei die kationisch reaktive Verbindung (A) aus mindestens einem Silikonoligomer (G-1) oder einem Silikonpolymer (G-2) besteht, das vernetzbar und/oder polymerisierbar, bei Umgebungstemperatur flüssig oder bei einer Temperatur von weniger als 100 °C thermofusionierbar ist, und Folgendes umfasst:
a) mindestens eine Struktur mit der folgenden Formel: 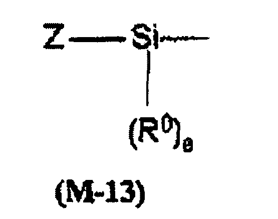 wobei in der Formel:
- a = 0, 1 oder 2,
- - R⁰, gleich oder verschieden, für einen Alkyl-, Cycloalkyl-, Aryl-, Vinyl-, Wasserstoff-, Alkoxyrest, bevorzugt für einen niedrigen C1-C6-Alkylrest steht,
- Z, gleich oder verschieden, ein organischer Substituent ist, der mindestens eine reaktive Oxiran-, Alkenylether-, Oxetan-, Dioxolan- und/oder Carbonatfunktion umfasst, und
b) mindestens zwei Siliciumatome.

12. Dentalzusammensetzung nach Anspruch 11, **dadurch gekennzeichnet, dass** die Struktur (M-13) Gruppen Z umfasst, ausgewählt aus der Gruppe bestehend aus den folgenden Resten (R-1) bis (R-9): 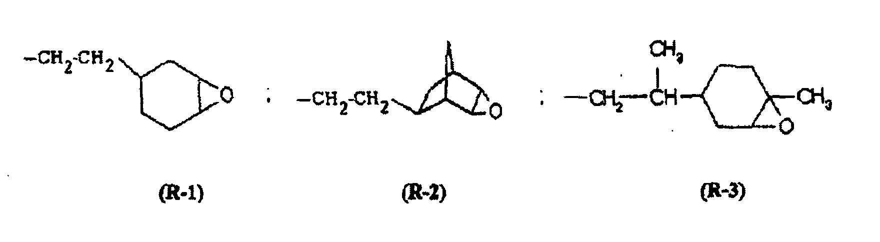 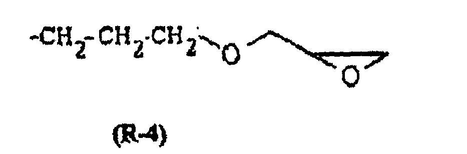 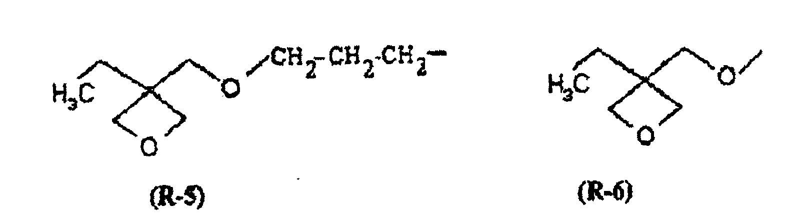 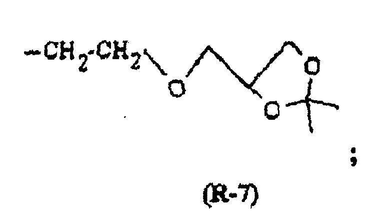
**(R-8)** -(CH₂)₃-O-CH=CH₂;
**(R-9)** -(CH₂)₃-O-CH=CH-R"
worin R" für einen linearen oder verzweigten C₁-C₆-Alkylrest steht.

13. Dentalzusammensetzung nach irgendeinem der vorhergehenden Ansprüche, **dadurch gekennzeichnet, dass** die kationisch reaktive Verbindung (A) ausgewählt ist aus der Gruppe bestehend aus den Verbindungen (S-1) bis (S-15), die in Anspruch 9 beschrieben sind.

14. Dentalzusammensetzung nach irgendeinem der vorhergehenden Ansprüche, **dadurch gekennzeichnet, dass** der kationische Photostarter (D) vom Typ Borat ist, und ausgewählt ist aus jenen mit der folgenden Formel, wovon:
a) die kationische Borateinheit ausgewählt ist aus:
(1) den Oniumsalzen mit der Formel:
**[(R⁹)ₙ-A- (R¹⁰)ₘ]⁺** **(I)**
wobei in der Formel:
- A für ein Element der Gruppen 15 bis 17 steht, wie zum Beispiel: I, S, Se, P oder N,
- R⁹ für einen carbocyclischen oder heterocyclischen C6-C20-Arylrest steht, wobei der heterocyclische Rest als Heteroelemente Stickstoff oder Schwefel enthalten kann,
- R¹⁰ für R⁹ oder einen linearen oder verzweigten C1-C30-Alkyl- oder Alkenylrest steht;
- wobei die Reste R⁹ und R¹⁰ gegebenenfalls durch eine C1-C25-Alkoxy-, Cl-C25-Alkyl-, Nitro-, Chlor-, Brom-, Cyano-, Carboxy-, Ester- oder Mercaptogruppe substituiert sind,
- m und n ganze Zahlen sind, worin n + m = v + 1, wobei v die Valenz des Elements A ist,
(2) den Oxoisothiochromaniumsalzen; und
(3) den metallorganischen Salzen mit der folgenden Formel:
(L₁L₂L₃M^{)+q} **(II)**
wobei in der Formel:
- M für ein Metall der Gruppe 4 bis 10 steht, insbesondere Eisen, Mangan, Chrom, Cobalt,
- L1 für 1 Liganden steht, der an das Metall M durch die Elektronen n gebunden ist, wobei der Ligand ausgewählt ist aus den η³-Alkyl-, η⁵-Cyclopendadienyl- und η⁷-Cycloheptatrienyl-Liganden und den aromatischen η⁶-Verbindungen, ausgewählt aus den gegebenenfalls substituierten η⁶-Benzen-Liganden und den Verbindungen, die 2 bis 4 kondensierte Ringe aufweisen, wobei jeder Ring in der Lage ist, mit 3 bis 8 Elektronen n zur Valenzschicht des Metalls M beizutragen,
- L2 für einen Liganden steht, der an das Metall M durch die Elektronen n gebunden ist,
wobei der Ligand ausgewählt ist aus den η⁷-Cycloheptatrienyl-Liganden und den aromatischen η⁶-Verbindungen, ausgewählt aus den gegebenenfalls substituierten η⁶-Benzen-Liganden und den Verbindungen, die 2 bis 4 kondensierte Ringe aufweisen, wobei jeder Ring in der Lage ist, mit 6 oder 7 Elektronen n zur Valenzschicht des Metalls M beizutragen,
- L3 für 0 bis 3 Liganden, gleich oder verschieden, steht, die an das Metall M durch die Elektronen σ gebunden sind, wobei der (die) Ligand(en) ausgewählt ist (sind) aus CO und NO₂⁺; wobei die gesamte Elektronenladung q des Komplexes, zu der L1, L2 und L3 beitragen und die Ionenladung des Metalls M positiv und gleich 1 oder 2 ist; und
b) die anionische Borateinheit folgende Formel aufweist:
**[BXₐR_{b}]⁻** **(III)**
wobei in der Formel:
- a und b ganze Zahlen sind, die für a von 0 bis 3 und für b von 1 bis 4 reichen, worin a + b = 4,
- wobei die Symbole X für Folgendes stehen:
* ein Halogenatom (Chlor, Fluor) worin a = 0 bis 3, oder
* eine OH-Funktion, worin a = 0 bis 2,
- wobei die Symbole R gleich oder verschieden sind und für Folgendes stehen:
- einen Phenylrest, der mit mindestens einer elektronenziehenden Gruppe substituiert ist, wie zum Beispiel OCF₃, CF₃, NO₂, CN, und/oder mit mindestens 2 Halogenatomen (ganz besonders Fluor), und zwar wenn die kationische Einheit ein Onium eines Elements der Gruppen 15 bis 17 ist,
- einen Phenylrest, der mit mindestens einem Element oder einer elektronenziehenden Gruppe substituiert ist, insbesondere einem Halogenatom (ganz besonders Fluor), CF₃, OCF₃, NO₂, CN, und zwar wenn die kationische Einheit ein metallorganischer Komplex eines Elements der Gruppen 4 bis 10 ist, oder
- einen Arylrest, der mindestens zwei aromatische Zentren enthält, wie zum Beispiel Biphenyl, Naphtyl, der gegebenenfalls mit mindestens einem Element oder einer elektronenziehenden Gruppe substituiert ist, insbesondere einem Halogenatom, darunter insbesondere Fluor, OCF₃, CF₃, NO₂ CN, unabhängig von der kationischen Einheit.

15. Zusammensetzung nach Anspruch 14, **dadurch gekennzeichnet, dass** der kationische Photostarter (D) ausgewählt ist aus der Gruppe, bestehend aus den folgenden Verbindungen:
(P-16): [(C₈H₁₇)-O-C₆H₄-I-C₆H₅)]⁺, [B(C⁶F⁵)₄]⁻;
(P-17) : [C₁₂H₂₅-C6H₄-I-C₆H₅]⁺, [B(C₆F₅)₄]⁻;
(P-18): [(C₈H₁₇-O-C₆H₄)₂I⁺, [B(C₆F₅)₄]⁻;
(P-19): [(C₈H₁₇)-O-C₆H₄-I-C₆H₅)⁺, [B(C₆F₅)₄]⁻;
(P-20): [(C₆H₅)₂S-C₆H₄-O-C₈H₁₇]⁺, [B(C₆H₄CF₃)₄]⁻;
(P-21): [(C₁₂H₂₅-C₆H₄)₂I]⁺, [B(C₆F₅)₄]⁻;
(P-22): [CH₃-C₆H₄-I-C₆H₄-CH(CH₃)₂]⁺, [B(C₆F₅)₄]⁻;
(P-23): (η5-Cyclopentadienyl) (η6-Toluol)Fe⁺, [B(C₆F₅)₄]⁻;
(P-24): (η5-Cyclopentadienyl) (η6-Methyl-1-naphtalin) Fe⁺, [B(C₆F₅)₄]-;
(P-25): (η5-Cyclopentadienyl) (η6-Cumen)Fe⁺, [B(C₆F₅)₄]-;
(P-26): [(C₁₂H₂₅-C₆H₄)₂I]⁺, [B(C₆H₃(CF₃)₂]⁻;
(P-27): [CH₃-C₆H₄-I-C₆H₄-CH₂CH(CH₃)₂]⁺, [B(C₆F₅)₄]⁻;
(P-28) : [CH₃-C₆H₄-I-C₆H₄-CH₂CH(CH₃)₂]+, [B(C₆H₃(CF₃)₂)₄]-; und
(P-29): [CH₃ -C₆H₄-I-C₆H₄-CH(CH₃)₂]⁺, [B(C₆H₃(CF₃)₂)₄]⁻.

16. Dentalzusammensetzung nach Anspruch 1, **dadurch gekennzeichnet, dass** der kationische Photostarter (D) ein Iodoniumsalz ist.

17. Dentalzusammensetzung nach irgendeinem der vorhergehenden Ansprüche, wobei der Photosensibilisator (E) in seiner Struktur einen oder mehrere aromatische Zentren, substituiert oder nicht, umfasst, die eine Restabsorption des Lichts im Bereich zwischen 200 und 500 nm aufweisen.

18. Dentalzusammensetzung nach irgendeinem der vorhergehenden Ansprüche, wobei der Photosensibilisator (E) ausgewählt ist aus der Gruppe, bestehend aus den Verbindungen der Klasse der Anthracene, Thioxanthone, Campherchinone und Phenanthrenchinon, sowie deren Gemischen.

19. Dentalzusammensetzung nach Anspruch 18, wobei der Photosensibilisator (E) ein Salz eines Thioxanthons ist, das mit mindestens einer Gruppe substituiert ist, die eine Ammoniumfunktion umfasst.

20. Dentalzusammensetzung nach Anspruch 19, **dadurch gekennzeichnet, dass** das Anion, das mit dem Salz des Thioxanthons assoziiert ist, das mit mindestens einer Gruppe substituiert ist, die eine Ammoniumfunktion umfasst, aus den folgenden Anionen ausgewählt ist: einem Halogenid, BF₄⁻, PF₆⁻; SbF₆⁻; dem Anion (III) mit der Formel [BXₐR_{b}]⁻, definiert nach Anspruch 18, R_{f}SO₃⁻; (R_{f}SO₂)₃C⁻ oder (R_{f}SO₂)₂N⁻, wobei R_{f} ein linearer oder verzweigter Alkylrest ist, der mit mindestens einem Halogenatom, bevorzugt einem Fluoratom, substituiert ist.

21. Dentalzusammensetzung nach irgendeinem der vorhergehenden Ansprüche, wobei der Photosensibilisator (E), gegebenenfalls in Assoziation mit mindestens einem Campherchinon, einem Phenanthrenchinon und/oder einem substituierten Anthracen, folgende Formel aufweist: 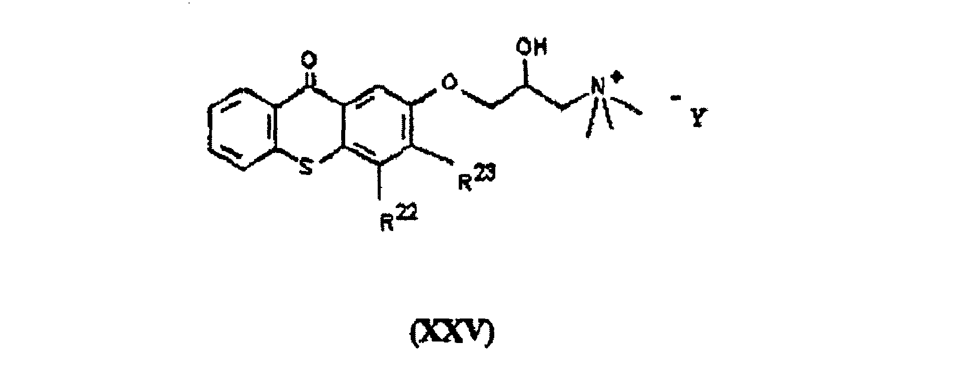 wobei in der Formel:
- R22 und R23 gleich oder verschieden sind und für einen Wasserstoff oder einen gegebenenfalls substituierten C1-C10-Alkylrest stehen, bevorzugt R22=R23= Methyl,
- wobei (X') eine anionische Einheit ist, bevorzugt ein Halogenid, BF₄⁻, PF₆⁻; SbF₆⁻; das Anion (III) mit der Formel [BXₐR_{b}]⁻, definiert nach Anspruch 16, R_{f}SO₃⁻; (R_{f}SO₂)₃C⁻ oder (R_{f}SO₂)₂N⁻, wobei R_{f} ein linearer oder verzweigter Alkylrest ist, der mit mindestens einem Halogenatom, bevorzugt einem Fluoratom, substituiert ist.
und noch stärker bevorzugt (X') ausgewählt ist aus den Boraten mit den folgenden Formeln: [B(C₆H₃(CF₃)₂)₄]⁻ und [B(C₆F₅)₄]⁻.

22. Dentalzusammensetzung nach irgendeinem der vorhergehenden Ansprüche, wobei der Photosensibilisator (E), gegebenenfalls in Assoziation mit mindestens einem Campherchinon, einem Phenanthrenchinon und/oder einem substituierten Anthracen, folgende Formel aufweist:

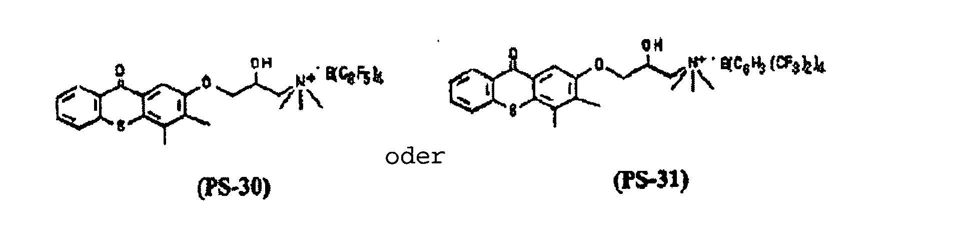

23. Dentalzusammensetzung nach irgendeinem der vorhergehenden Ansprüche, wobei der Photosensibilisator (E) ausgewählt ist aus der Gruppe, bestehend aus den folgenden Verbindungen:
- (PS-30): 3-(3,4-Dimethyl-9-oxo-9-thioxanthen-2-yloxy)-2-hydroxypropyl)-trimethylammoniumchlorid;
- (PS-31): 3-(3,4-Dimethyl-9-oxo-9-thioxanthen-2-yloxy)-2-hydroxypropyl)-trimethylammonium-tetrakis(pentafluorphenyl)borat;
- (PS-32): 3-(3,4-Dimethyl-9-oxo-9-thioxanthen-2-yloxy) 2-hydroxypropyl)-trimethylammonium-tetrakis(bis(trifluormethyl)phenyl)borat;
- (PS-33): Phenanthrenchinon;
- (PS-34): Campherchinon;
- (PS-35): Acenaphtenchinon;
- (PS-36): Dibenzoylperoxid;
- (PS-37): 2-Ethyl-9,10-dimethoxyanthracen;
- (PS-38): 9,10-Diethoxyanthracen;
- (PS-39): 9,10-Dibutoxyanthracen;
- (PS-40): 9-Hydroxymethylanthracen;
- (PS-41): 2-Dimethylaminothioxanthon;
- (PS-42): 3-Ethylcarboxy-7-methoxythioxanthon;
- (PS-43): 1-Phenyl-thio-4-propoxythioxanthon;
- (PS-44): 2-Methoxythioxanthon;
- (PS-45): 2-(N,N-Diethylaminopropoxy)thioxanthon;
- (PS-46): 2-Isopropylthioxanthon;
- (PS-47): 1-Chlor-4-propoxythioxanthon;
- (PS-48): 4-Isopropylthioxanthon; 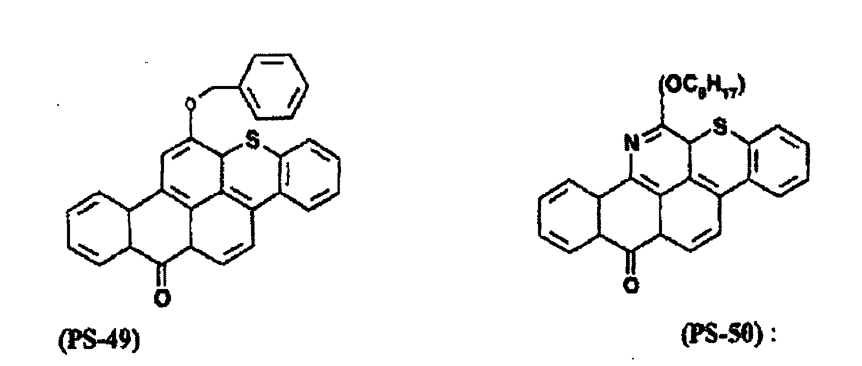
- und deren Gemische.

24. Dentalzusammensetzung nach irgendeinem der vorhergehenden Ansprüche, wobei der Photosensibilisator (E) ausgewählt ist aus der Gruppe, bestehend aus den Verbindungen der Familie der Cumarine, Diketone, Fluorone, Aminoketone, para-Aminostyrylketone sowie deren Gemischen.

25. Dentalzusammensetzung nach irgendeinem der vorhergehenden Ansprüche, wobei das Silikonpolymer (G-2) oder Silikonoligomer (G-1) mit einem organischen Epoxidharz oder einem Oxetan mit der Formel (S-103) oder (S-104) assoziiert ist: 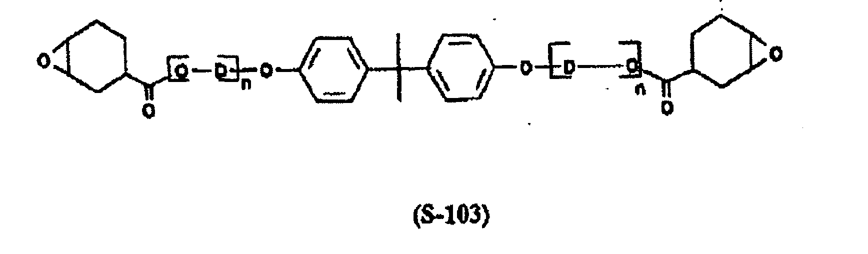 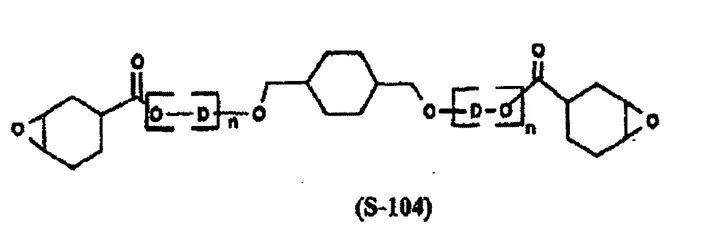 worin:
- 0 ≤ n ≤ 100 und
- D = Rest, ausgewählt aus den linearen oder verzweigten C1-C12-Alkylen.

26. Dentalzusammensetzung nach einem der vorhergehenden Ansprüche, wobei der Dentalfüllstoff (B) ein mineralisches Glas oder eine pyrogene Kieselsäure ist.

27. Dentalzusammensetzung nach irgendeinem der vorhergehenden Ansprüche, wobei die Verbindung (G) ein Silan (G-3) ist, mit der Formel: 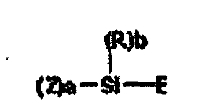 wobei in der Formel: 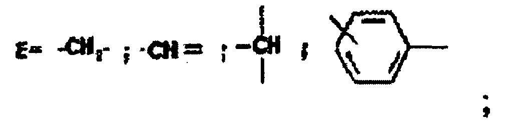
- R, gleich oder verschieden, für einen Alkyl-, Cycloalkyl-, Aryl-, Vinyl-, Wasserstoff-, Alkoxyrest, bevorzugt für einen niedrigen C1-C6-Alkylrest steht,
- Z, gleich oder verschieden, ein organischer Substituent ist, der mindestens eine Oxiran-, Alkenylether-, Oxetan- und/oder Carbonatfunktion umfasst, und
- a + b = 3.

28. Dentalzusammensetzung nach Anspruch 27, wobei das Silan (G-3) ausgewählt ist aus der Gruppe, bestehend aus den folgenden Molekülen:

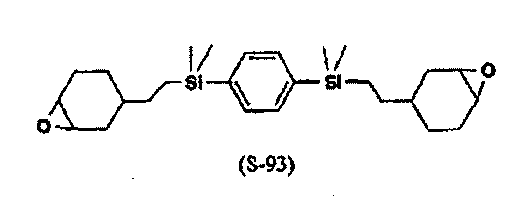

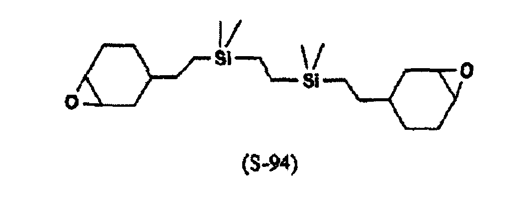

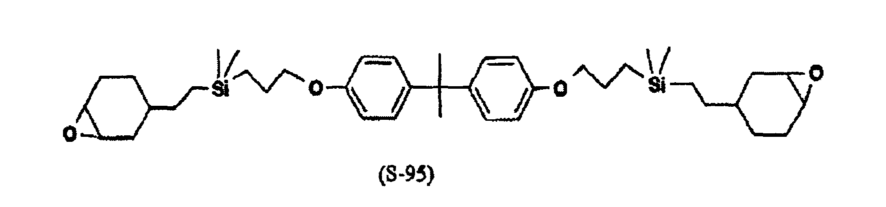

29. Dentalzusammensetzung nach einem der vorhergehenden Ansprüche, wobei die Verbindung (G) eine organische Verbindung (G-4) ist, ausgewählt aus der Gruppe bestehend aus Molekülen (S-96) bis (S-104): 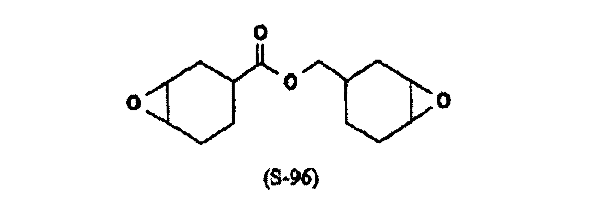 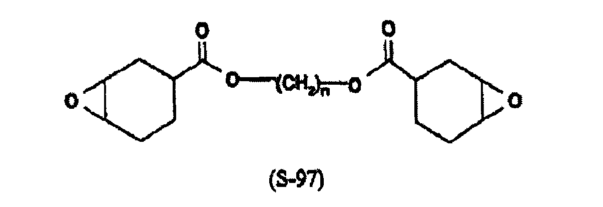 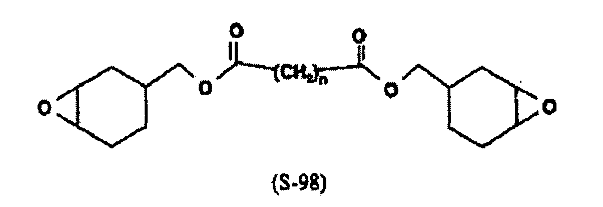 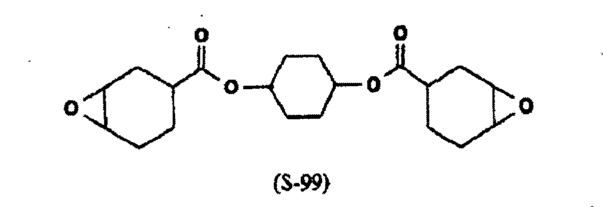 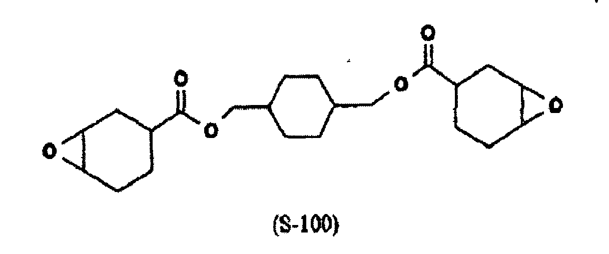 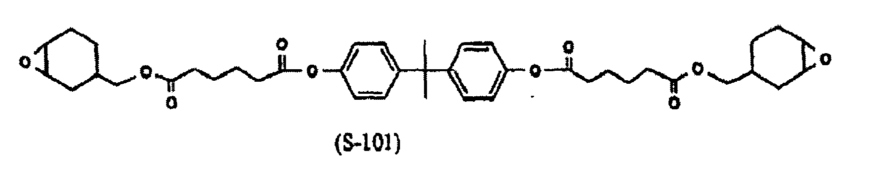 wobei in den Formeln: n eine ganze Zahl im Bereich zwischen 1 und 10 ist (einschließlich der Grenzwerte); 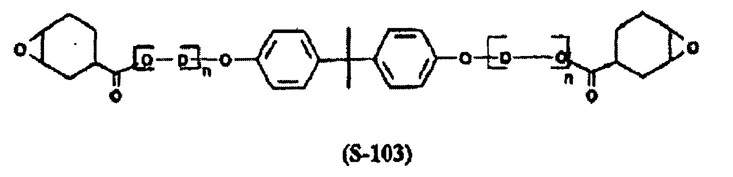 worin n<100 ist und D = lineares oder verzweigtes C1-C12-Alkyl. 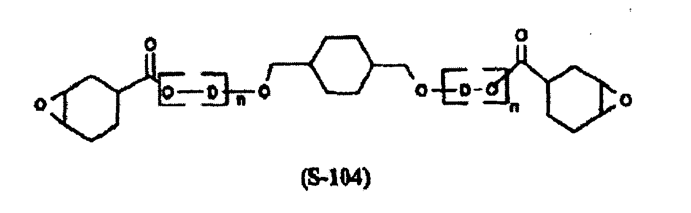 worin n<100 ist und die Gruppe D = lineares oder verzweigtes C1-C12-Alkyl.

30. Verfahren zum Behandeln eines Dentalverstärkungsfüllstoffs, das die folgenden Schritte umfasst:
a) Mischen in Lösemittelmedium des Dentalverstärkungsfüllstoffs (B) und mindestens eines siliciumorganischen Kopplungsmittels (F) mit der Formel 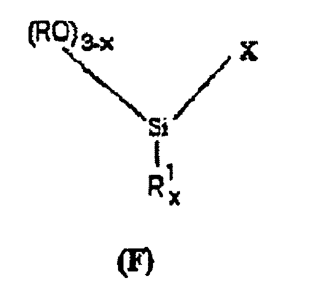 wobei in der Formel:
- R ein Wasserstoff oder ein linearer oder verzweigter C1-C4-Alkylrest ist,
- R¹ ein linearer oder verzweigter Alkylrest oder ein Phenylrest ist,
- x gleich 0, 1 oder 2 ist, und
- X durch die folgende Formel definiert ist: 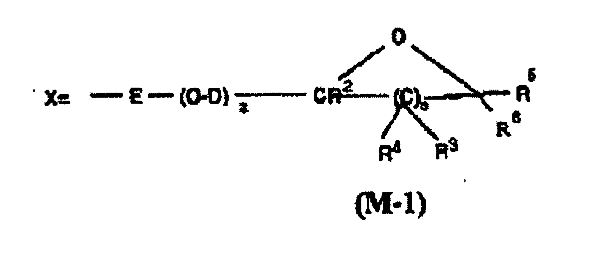 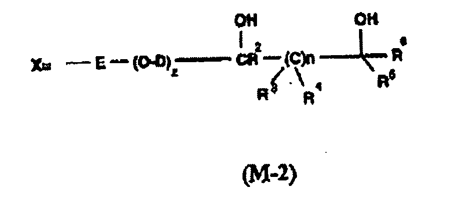 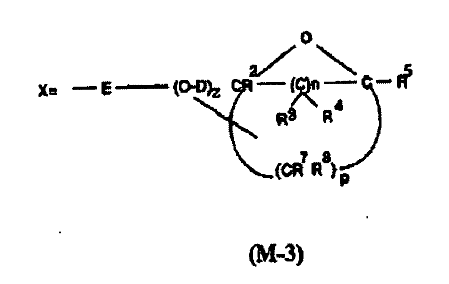 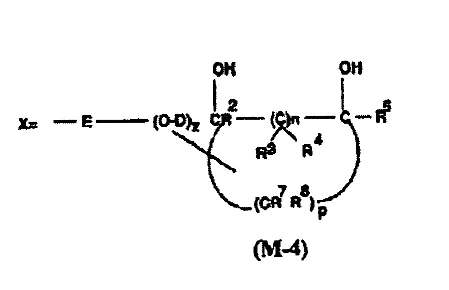 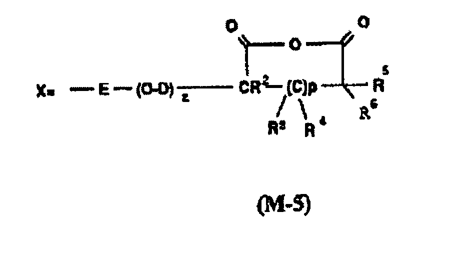 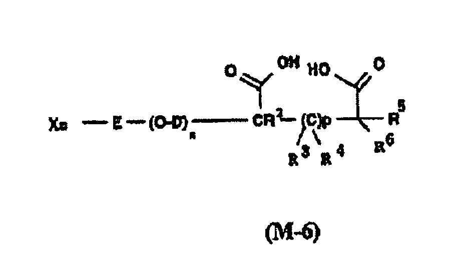
worin:
- E und D gleiche oder verschiedene Reste sind, ausgewählt aus den linearen oder verzweigten C1-C12-Alkylen,
- z gleich 0 oder 1 ist,
- n gleich 0 oder 1 ist,
- p gleich 0, 1, 2, 3, 4, 5 oder 6 ist.
- R², R³, R⁴, R⁵, R⁶, R⁷ und R⁸ gleiche oder verschiedene Reste sind, die für Wasserstoff oder ein lineares oder verzweigtes C1-C12-Alkyl stehen.
b) Verdampfen des Lösemittels, um einen intermediären Dentalfüllstoff (B-1) zu erhalten,
c) der intermediäre Dentalfüllstoff (B-1) wird einer Wärmebehandlung unterzogen, um die Kopplungsreaktion zwischen dem intermediären Dentalfüllstoff (B-1) und dem Kopplungsmittel (F) zu ermöglichen und so einen intermediären Dentalfüllstoff (B-2) zu erhalten,
d) der intermediäre Dentalfüllstoff (B-2) wird anschließend in Lösemittelmedium mit mindestens einer Verbindung (G) gemischt, die ein organisches Monomer, Oligomer oder Polymer oder ein Organosiloxan ist, das mindestens eine Oxiran-, Oxetan-, Alkenylether- und/oder Carbonatfunktion umfasst,
e) Verdampfen des Lösemittels, um einen intermediären Dentalfüllstoff (B-3) zu erhalten, und
f) der intermediäre Dentalfüllstoff (B-3) wird einer Wärmebehandlung unterzogen, um die Reaktion zwischen dem intermediären Dentalfüllstoff (B-3) und der Verbindung (G) zu ermöglichen, und so einen behandelten Dentalfüllstoff (B-4) zu erhalten.

31. Verfahren zum Behandeln eines Dentalverstärkungsfüllstoffs nach dem vorhergehenden Anspruch, wobei die Wärmebehandlung der Schritte c) und f) des Verfahrens (I) durch eine Erwärmung auf eine Temperatur kleiner oder gleich 200 °C, bevorzugt kleiner oder gleich 160 °C und noch stärker bevorzugt im Bereich zwischen 100 und 165 °C durchgeführt wird.

32. Verfahren zum Behandeln eines Dentalverstärkungsfüllstoffs nach irgendeinem der Ansprüche 30 bis 31, wobei das siliciumorganische Kopplungsmittel (F) nach Anspruch 5 definiert ist.

33. Verfahren zum Behandeln eines Dentalverstärkungsfüllstoffs nach irgendeinem der Ansprüche 30 bis 31, wobei die Verbindung (G) nach irgendeinem der Ansprüche 6 bis 8 definiert ist.

34. Verfahren zum Behandeln eines Dentalverstärkungsfüllstoffs nach irgendeinem der Ansprüche 30 bis 31, wobei der Verstärkungsfüllstoff ein mineralisches Glas oder eine pyrogene Kieselsäure ist.

35. Verstärkungsfüllstoff, der durch ein Verfahren nach irgendeinem der Ansprüche 30 bis 34 erhalten wurde.

36. Dentalprothese, die durch Vernetzung einer Zusammensetzung nach einem der Ansprüche 1 bis 29 erhalten werden kann.

37. Material zur Dentalrestauration, die durch Vernetzung einer Zusammensetzung nach einem der Ansprüche 1 bis 29 erhalten werden kann.
